# EUROPEAN PATENT APPLICATION

(11) **EP 4 516 341 A1**
(43) Date of publication of application: **05.03.2025**
(21) Application number: 23796410.1
(22) Date of filing: 26.04.2023
(51) Int. Cl.: A61M 5/31, A61K 9/08, A61K 39/395, A61M 5/28, A61P 7/04, A61P 25/00, A61P 27/02, A61P 29/00, A61P 37/02

(54) **PHARMACEUTICAL-PREPARATION-CONTAINING SYRINGE EQUIPPED WITH FILTER**

(30) Priority: 26.04.2022 JP 2022072513
(71) Applicant: CHUGAI SEIYAKU KABUSHIKI KAISHA, Tokyo 115-8543 (JP)
(72) Inventor: YAMANAKA, Yuji, Tokyo 115-8543 (JP); EGAMI, Kiichi, Tokyo 115-8543 (JP); HORIKAWA, Ayano, Tokyo 115-8543 (JP); HORITA, Taiji, Ibaraki-shi, Osaka 567-0054 (JP); OGAWA, Yukihiro, Ibaraki-shi, Osaka 567-0054 (JP); TANIGUCHI, Kensuke, Ibaraki-shi, Osaka 567-0054 (JP); MIYAGAWA, Junki, Ibaraki-shi, Osaka 567-0054 (JP)
(74) Representative: Vossius & Partner Patentanwälte Rechtsanwälte mbB
(86) International application number: PCT/JP2023/016386
(87) International publication number: WO 2023/210670

(57) **Abstract**

An object of the present invention is to remove particles from a liquid formulation filled in a syringe immediately before administration. The present invention provides a formulation for injection in which a solution is filled in a syringe, wherein the syringe comprises: a barrel formed in a cylindrical shape having a leading end portion and a base end portion, wherein the leading end portion is provided with a nozzle hole for externally ejecting a solution held therein; and a filter disposed within the barrel in a position on a base end side of the nozzle hole, the filter is provided in such a manner as to allow the solution to flow from the base end side to a leading end side through the filter, the filter is a membrane, and a material of the membrane includes one or more selected from polyether sulfone (PES), polyvinylidene fluoride (PVDF), polysulfone (PS), and an acrylic copolymer.

## Description

### TECHNICAL FIELD

The present invention relates to a pharmaceutical formulation in which a solution containing an active ingredient is filled in a container.

### BACKGROUND ART

In recent years, various antibody-containing formulations have been developed and put into practical use, but many antibody-containing formulations are used as formulations for intravenous injection. Meanwhile, due to needs in actual medical practice, there are increasing demands for developing antibody-containing formulations as self-injectable formulations for subcutaneous injection. Especially, there is a high demand for developing a pre-filled syringe formulation in which a solution is enclosed in a syringe due to its convenience.

In design of an antibody-containing formulation for subcutaneous injection, it is essential that the concentration of an antibody in a liquid to be administered is high because while the amount of the antibody to be administered per dose is large (about 80 to 200 mg), the amount of an injection liquid is generally restricted in subcutaneous injection.

In recent years, in a formulation used in actual medical practice as a pre-filled syringe formulation for self-injection, a syringe comprising a cylindrical injection syringe body filled with a drug, a needle attached to the leading end of the injection syringe body, a detachably-attached syringe cap covering the needle, and a plunger that is inserted into the injection syringe body and is slidable in the axial direction of the injection syringe body is used.

In using the pre-filled syringe formulation, the syringe cap is removed, the needle is inserted into an administration site, and then the plunger is moved forward with a plunger rod to excrete and administer a drug solution. Generally, in order to ensure slidability of the plunger, a lubricant of silicone oil or the like is applied to the inside wall of the pre-filled syringe and the plunger.

Formation of particles in an aqueous solution is a problem in an antibody-containing formulation. The particles to be formed are aggregates that are larger than multimers such as dimers and trimers, and known particles include sub-visible particles (SVPs), that is, microparticles with a particle size of 1.5 µm to less than 50 µm that are generally difficult to see with eyes, and visible particles (VPs, larger than 100 µm) that are visually detectable under standard illuminance (about 2,000 to 3,000 lx). A visual detection rate of visible particles in a pharmaceutical formulation varies greatly depending on an examiner, and under standard illuminance prescribed in the Japanese pharmacopoeia (about 2,000 to 3,000 lx), it is reported that detection sensitivity of particles with a particle size of 100 µm is about 40%, detection sensitivity of particles with a particle size of 150 µm is about 70%, and detection sensitivity of particles with a particle size of 200 µm is almost 100% (Non Patent Literature 1). Actually, particles with a smaller particle size of a minimum of about 40 µm can be visually detected by increasing illuminance for observing a pharmaceutical formulation or by increasing an observation time period. Herein, such particles of 40 µm or more to 100 µm are particularly referred to as particles visually detectable only under high illuminance. Besides, particles with a particle size of 40 µm or more are particles visually detectable under high illuminance, and are referred to as visually detectable particles.

Generally, antibodies have a property of aggregating on interfaces such as air-liquid interfaces and solid-liquid interfaces. The presence of such interfaces may be involved in the formation of the visually detectable particles described above. It is reported that application of mechanical stress to a syringe filled with an antibody solution results in marked increase of microparticles ascribable to the presence of an interface (NPL 2). An air-liquid interface is formed in an antibody solution filled in a syringe due to presence of air bubbles, and a solid-liquid interface is formed when the solution contacts a plunger and a syringe barrel. Further, when silicone is applied to the plunger and the barrel of the pre-filled syringe, the antibody solution contacts the silicone on the solid phase surface and forms a new solid-liquid interface. Also, it is reported that proteins that adsorb to and aggregate on a solid-liquid interface are detached into the solution by the movement of air in the solution of the pre-filled syringe and appear as visible particles (NPL 3).

Since it cannot be denied that the proteinaceous particles thus formed in the aqueous solution has a risk of immunogenicity, it is preferable that such particles are not administered into the body.

Besides, not only in an antibody-containing formulation but also in a pre-filled syringe formulation comprising a low molecular weight compound or a middle molecular weight compound as an active ingredient, particles of internal foreign matters and external foreign matters can cause a problem.

As a method for removing particles from a formulation filled in a vial immediately before administration, a filter needle in which a filter of a pore size of 5 µm is provided in a transfusion needle, or an in-line filter included in an infusion set may be used. The filter needle is commercially available under a trade name of BD Blunt Filter Needle (Becton, Dickinson and Company Japan), or Sterifix(R) (B. Braun). Besides, there are known syringes, such as a syringe having a sealing mechanism and a filter for partitioning the chamber space into a wet part and a dry part when a mixture of a plurality of contents is administered (PTLs 1 to 3), and a syringe including a filter for filtering foreign matters within the syringe when a drug solution of an ampoule is sucked through a needle for injecting it through the needle (PTL 4). On the other hand, a method for removing particles from a pre-filled syringe formulation immediately before administration has not been established yet.

### CITATION LIST

### PATENT LITERATURE

PTL 1: Japanese Patent Laid-Open No. 2007-508898
PTL 2: Japanese Patent Laid-Open No. 2019-051338
PTL 3: Japanese Translation of PCT International Application Publication No. 2005-537106
PTL 4: Japanese Translation of PCT International Application Publication No. 2015-536213

### NON PATENT LITERATURE

NPL 1: James A. Melchore, AAPS Pharm Sci Tech; 2011; 12(1): 215-221
NPL 2: Torisu et al., J. Pharm. Sci. 106 (2017) 2966-2978
NPL 3: Gerhardt et al., J. Pharm. Sci. 103 (2014) 1601-1612

### SUMMARY OF INVENTION

### TECHNICAL PROBLEM

Although a method for removing particles from a formulation filled in a vial immediately before administration is known, a method for removing particles from a liquid formulation filled in a syringe immediately before administration has not been established yet, and is technically difficult.

### SOLUTION TO PROBLEM

Therefore, the present inventors made earnest studies on a novel syringe including a filter, resulting in accomplishing the present invention. The present specification includes the following disclosures of the invention:
[1-1] A formulation for injection in which a solution is filled in a syringe, wherein
   the syringe comprises:
   a barrel formed in a cylindrical shape having a leading end portion and a base end portion, wherein the leading end portion is provided with a nozzle hole for externally ejecting a solution held therein; and
   a filter disposed within the barrel in a position on a base end side of the nozzle hole, wherein
   the filter is provided in such a manner as to allow the solution to flow from the base end side to a leading end side through the filter, and
   the filter is a membrane, and a material of the membrane includes one or more selected from polyether sulfone (PES), polyvinylidene fluoride (PVDF), polysulfone (PS), and an acrylic copolymer.
[1-2] The formulation for injection according to [1-1], wherein the material of the membrane is an acrylic copolymer.
[1-3] The formulation for injection according to [1-2], wherein the acrylic copolymer is hydrophobic.
[1-4] The formulation for injection according to any one of [1-1] to [1-3], wherein the filter has a pore size of 5 µm or less, and preferably 5 µm.
[1-5] The formulation for injection according to any one of [1-1] to [1-4], wherein the leading end side of the filter is provided with a leading end side subspace, the base end side of the filter is provided with a base end side subspace, and when seen from the axial direction of the barrel, the nozzle hole has an area smaller than an area of the base end side subspace, and smaller than an area of the leading end side subspace.
[1-6] The formulation for injection according to any one of [1-1] to [1-5], wherein
   the syringe comprises partitioning means for partitioning an inside space of the barrel into a base end side space and a leading end side space,
   the partitioning means comprises: a cylindrical housing disposed to liquid-tightly contact an inner surface of the barrel; and an inner plug portion disposed in such a manner as to be openable/closable to the inside of the housing for communicating or non-communicating the base end side space and the leading end side space with each other through the inside of the housing,
   the housing has a close-time engaging portion,
   the inner plug portion has a close-time engaged portion to be engaged with the close-time engaging portion from the base end side,
   the partitioning means is constituted in such a manner that one of the close-time engaging portion and the close-time engaged portion moves beyond the other to release engagement between the close-time engaging portion and the close-time engaged portion when a pressing force is applied from the base end side by a piston inserted into the barrel from the base end side, and that the inner plug portion is opened to the inside of the housing to communicate the base end side space and the leading end side space with each other, and
   the filter is held in a leading end side portion of the housing.
[1-7] The formulation for injection according to [1-6], wherein one of the close-time engaging portion and the close-time engaged portion is disposed on the leading end side and the base end side of the other of the close-time engaging portion and the close-time engaged portion.
[1-8] The formulation for injection according to [1-6] or [1-7], wherein a material of the inner plug portion is linear low density polyethylene (LLDPE), or low density polyethylene (LDPE), and is preferably low density polyethylene (LDPE).
[1-9] The formulation for injection according to any one of [1-1] to [1-5], wherein
   the syringe comprises partitioning means for partitioning an inside space of the barrel into a base end side space and a leading end side space,
   the partitioning means comprises a cylindrical housing disposed to liquid-tightly contact an inner surface of the barrel, and
   the filter is held in a leading end side portion of the housing.
[1-10] The formulation for injection according to any one of [1-6] to [1-9], wherein the partitioning means is disposed in such a manner that a volume of the leading end side space of the barrel is 150 mm³ or less.
[1-11] The formulation for injection according to any one of [1-6] to [1-10], wherein a material of the housing is linear low density polyethylene (LLDPE), or low density polyethylene (LDPE), and is preferably low density polyethylene (LDPE).
[1-12] The formulation for injection according to any one of [1-1] to [1-11], wherein a compound contained in the solution as an active ingredient is a low molecular weight compound, a middle molecular weight compound, or a protein.
[1-13] The formulation for injection according to [1-12], wherein the compound comprises an antibody or a bound fragment thereof, or a cyclic peptide.
[1-14] The formulation for injection according to [1-12], wherein the low molecular weight compound has a molecular weight lower than 500.
[1-15] The formulation for injection according to [1-12], wherein the middle molecular weight compound has a molecular weight of 500 to 15000.
[1-16] The formulation for injection according to [1-12], wherein the compound is a polynucleotide selected from the group consisting of a ribozyme, an antisense molecule, an inhibitor oligonucleotide, an aptamer, a micro-RNA, and a small interfering RNA (siRNA).
[1-17] The formulation for injection according to any one of [1-1] to [1-16], wherein the solution is kept in a state not in contact with the filter before use.
[1-18] The formulation for injection according to any one of [1-1] to [1-16], wherein the solution is in a state in contact with the filter before use.
[1-19] The formulation for injection according to [1-17], wherein at the time of, or after production of the formulation for injection in which the solution is filled in the syringe, the solution is kept in a state not in contact with the filter before use.
[1-20] The formulation for injection according to any one of [1-1] to [1-19], wherein the filter is not damaged, and an adherend between the filter and the housing is not peeled off even after discharging, through the filter, a solution having a viscosity of 95 cP or less at a discharge rate of 300 mm/min.
[1-21] The formulation for injection according to any one of [1-1] to [1-20], wherein when a solution having a viscosity of 95 cP or less is discharged at a discharge rate of 100 mm/min through the filter, an average sliding resistance value for 10 mm is within 1.3-fold, 1.4-fold, or 1.5-fold as compared with that of a syringe not including a filter.
[1-22] The formulation for injection according to any one of [1-1] to [1-21], wherein the solution comprises one or more pharmaceutically acceptable excipients selected from a sugar, a sugar alcohol, a buffer, a preservative, a carrier, an antioxidant, a chelating agent, a natural polymer, a synthetic polymer, a cryoprotective agent, an extending agent, and a stabilizing agent.
[1-23] The formulation for injection according to any one of [1-1] to [1-22] comprising a needle to be connected to the syringe.
[1-24] The formulation for injection according to any one of [1-1] to [1-23], wherein the solution is an aqueous solution.
[1-25] The formulation for injection according to any one of [1-1] to [1-24], wherein the solution comprises emicizumab as an active ingredient.
[2-1] A formulation for injection in which a solution is filled in a syringe, wherein
   the syringe comprises:
      a barrel formed in a cylindrical shape having a leading end portion and a base end portion, wherein the leading end portion is provided with a nozzle hole for externally ejecting a solution held therein; and
      a filter disposed within the barrel in a position on a base end side of the nozzle hole, wherein
      the filter is provided in such a manner as to allow the solution to flow from the base end side to a leading end side through the filter,
      the filter is a membrane, and a material of the membrane includes one or more selected from polyether sulfone (PES), polyvinylidene fluoride (PVDF), polysulfone (PS), and an acrylic copolymer, and
   in discharging the solution, visually detectable particles are removable from the discharged solution.
[2-2] The formulation for injection according to [2-1], wherein the material of the membrane is an acrylic copolymer.
[2-3] The formulation for injection according to [2-2], wherein the acrylic copolymer is hydrophobic.
[2-4] The formulation for injection according to any one of [2-1] to [2-3], wherein the filter has a pore size of 5 µm or less, and preferably 5 µm.
[2-5] The formulation for injection according to any one of [2-1] to [2-4], wherein a leading end side subspace is provided on the leading end side of the filter, a base end side subspace is provided on the base end side of the filter, and when seen from the axial direction of the barrel, the nozzle hole has an area smaller than an area of the base end side subspace, and smaller than an area of the leading end side subspace.
[2-6] The formulation for injection according to any one of [2-1] to [2-5], wherein the syringe comprises partitioning means for partitioning an inside space of the barrel into a base end side space and a leading end side space,
   the partitioning means comprises: a cylindrical housing disposed to liquid-tightly contact an inner surface of the barrel; and an inner plug portion disposed in such a manner as to be openable/closable to the inside of the housing for communicating or non-communicating the base end side space and the leading end side space with each other through the inside of the housing,
   the housing has a close-time engaging portion,
   the inner plug portion has a close-time engaged portion to be engaged with the close-time engaging portion from the base end side,
   the partitioning means is constituted in such a manner that one of the close-time engaging portion and the close-time engaged portion moves beyond the other to release engagement between the close-time engaging portion and the close-time engaged portion when a pressing force is applied from the base end side by a piston inserted into the barrel from the base end side, and that the inner plug portion is opened to the inside of the housing to communicate the base end side space and the leading end side space with each other, and
   the filter is held in a leading end side portion of the housing.
[2-7] The formulation for injection according to [2-6], wherein one of the close-time engaging portion and the close-time engaged portion is disposed on the leading end side and the base end side of the other of the close-time engaging portion and the close-time engaged portion.
[2-8] The formulation for injection according to any one of [2-1] to [2-5], wherein
   the syringe comprises partitioning means for partitioning an inside space of the barrel into a base end side space and a leading end side space,
   the partitioning means comprises a cylindrical housing disposed to liquid-tightly contact an inner surface of the barrel, and
   the filter is held in a leading end side portion of the housing.
[2-9] The formulation for injection according to any one of [2-6] to [2-8], wherein the partitioning means is disposed in such a manner that a volume of the leading end side space of the barrel is 150 mm³ or less.
[2-10] The formulation for injection according to any one of [2-1] to [2-9], wherein the particles have a particle size larger than 40 µm.
[2-11] The formulation for injection according to any one of [2-1] to [2-10], wherein the particles include a particle derived from a component of the solution, or a particle derived from a substance except for the component of the solution.
[2-12] The formulation for injection according to [2-11], wherein the particles are derived from a component of the solution.
[2-13] The formulation for injection according to [2-11], wherein the particles are derived from a substance except for the component of the solution.
[2-14] The formulation for injection according to any one of [2-1] to [2-13], wherein a compound contained in the solution as an active ingredient is a low molecular weight compound, a middle molecular weight compound, or a protein.
[2-15] The formulation for injection according to [2-14], wherein the compound comprises an antibody or a bound fragment thereof, or a cyclic peptide.
[2-16] The formulation for injection according to [2-14], wherein the low molecular weight compound has a molecular weight lower than 500.
[2-17] The formulation for injection according to [2-14], wherein the middle molecular weight compound has a molecular weight of 500 to 15000.
[2-18] The formulation for injection according to [2-14], wherein the compound is a polynucleotide selected from the group consisting of a ribozyme, an antisense molecule, an inhibitor oligonucleotide, an aptamer, a micro-RNA, and a small interfering RNA (siRNA).
[2-19] The formulation for injection according to any one of [2-1] to [2-18], wherein the solution is kept in a state not in contact with the filter before use.
[2-20] The formulation for injection according to any one of [2-1] to [2-18], wherein the solution is in a state in contact with the filter before use.
[2-21] The formulation for injection according to [2-19], wherein at the time of, or after production of the formulation for injection in which the solution is filled in the syringe, the solution is kept in a state not in contact with the filter before use.
[2-22] The formulation for injection according to any one of [2-1] to [2-21], wherein the filter is not damaged, and an adherend between the filter and the housing is not peeled off even after discharging, through the filter, a solution having a viscosity of 95 cP or less at a discharge rate of 300 mm/min.
[2-23] The formulation for injection according to any one of [2-1] to [2-22], wherein when a solution having a viscosity of 95 cP or less is discharged at a discharge rate of 100 mm/min through the filter, an average sliding resistance value for 10 mm is within 1.3-fold, 1.4-fold, or 1.5-fold as compared with that of a syringe not including a filter.
[2-24] The formulation for injection according to any one of [2-1] to [2-23], wherein the solution comprises one or more pharmaceutically acceptable excipients selected from a sugar, a sugar alcohol, a buffer, a preservative, a carrier, an antioxidant, a chelating agent, a natural polymer, a synthetic polymer, a cryoprotective agent, an extending agent, and a stabilizing agent.
[2-25] The formulation for injection according to any one of [2-1] to [2-24], wherein the solution is an aqueous solution.
[2-26] The formulation for injection according to any one of [2-1] to [2-25], wherein the solution comprises emicizumab as an active ingredient.
[3-1] A method for removing visually detectable particles contained in a formulation for injection, wherein
   the formulation for injection is a solution, and is filled in a syringe,
   the syringe comprises: a barrel formed in a cylindrical shape having a leading end portion and a base end portion, wherein the leading end portion is provided with a nozzle hole for externally ejecting the formulation for injection held therein; and a filter disposed within the barrel in a position on a base end side of the nozzle hole, the filter being provided in such a manner as to allow the solution to flow from the base end side to a leading end side through the filter,
   the method comprises discharging the solution filled in the syringe through the filter, and the filter is a membrane, and a material of the membrane includes one or more selected from polyether sulfone (PES), polyvinylidene fluoride (PVDF), polysulfone (PS), and an acrylic copolymer.
[3-2] The method according to [3-1], wherein the material of the membrane is an acrylic copolymer.
[3-3] The method according to [3-2], wherein the acrylic copolymer is hydrophobic.
[3-4] The method according to any one of [3-1] to [3-3], wherein the filter has a pore size of 5 µm or less, and preferably 5 µm.
[3-5] The method according to any one of [3-1] to [3-4], wherein the leading end side of the filter is provided with a leading end side subspace, the base end side of the filter is provided with a base end side subspace, and when seen from an axial direction of the barrel, the nozzle hole has an area smaller than an area of the base end side subspace, and smaller than an area of the leading end side subspace.
[3-6] The method according to any one of [3-1] to [3-5], wherein
   the syringe comprises partitioning means for partitioning an inside space of the barrel into a base end side space and a leading end side space,
   the partitioning means comprises: a cylindrical housing disposed to liquid-tightly contact an inner surface of the barrel; and an inner plug portion disposed in such a manner as to be openable/closable to the inside of the housing for communicating or non-communicating the base end side space and the leading end side space with each other through the inside of the housing,
   the housing has a close-time engaging portion,
   the inner plug portion has a close-time engaged portion to be engaged with the close-time engaging portion from the base end side,
   the partitioning means is constituted in such a manner that one of the close-time engaging portion and the close-time engaged portion moves beyond the other to release engagement between the close-time engaging portion and the close-time engaged portion when a pressing force is applied from the base end side by a piston inserted into the barrel from the base end side, and that the inner plug portion is opened to the inside of the housing to communicate the base end side space and the leading end side space with each other, and
   the filter is held in a leading end side portion of the housing.
[3-7] The method according to [3-6], wherein one of the close-time engaging portion and the close-time engaged portion is disposed on the leading end side and the base end side of the other of the close-time engaging portion and the close-time engaged portion.
[3-8] The method according to any one of [3-1] to [3-5], wherein
   the syringe comprises partitioning means for partitioning an inside space of the barrel into a base end side space and a leading end side space,
   the partitioning means comprises a cylindrical housing disposed to liquid-tightly contact an inner surface of the barrel, and
   the filter is held in a leading end side portion of the housing.
[3-9] The method according to any one of [3-6] to [3-8], wherein the partitioning means is disposed in such a manner that a volume of the leading end side space of the barrel is 150 mm³ or less.
[3-10] The method according to any one of [3-1] to [3-9], wherein the particles have a particle size larger than 40 µm.
[3-11] The method according to any one of [3-1] to [3-10], wherein the particles include a particle derived from a component of the solution, or a particle derived from a substance except for the component of the solution.
[3-12] The method according to [3-11], wherein the particles are derived from a component of the solution.
[3-13] The method according to [3-11], wherein the particles are derived from a substance except for the component of the solution.
[3-14] The method according to any one of [3-1] to [3-13], wherein a compound contained in the solution as an active ingredient is a low molecular weight compound, a middle molecular weight compound, or a protein.
[3-15] The method according to [3-14], wherein the compound comprises an antibody or a bound fragment thereof, or a cyclic peptide.
[3-16] The method according to [3-14], wherein the low molecular weight compound has a molecular weight lower than 500.
[3-17] The method according to [3-14], wherein the middle molecular weight compound has a molecular weight of 500 to 15000.
[3-18] The method according to [3-14], wherein the compound is a polynucleotide selected from the group consisting of a ribozyme, an antisense molecule, an inhibitor oligonucleotide, an aptamer, a micro-RNA, and a small interfering RNA (siRNA).
[3-19] The method according to any one of [3-1] to [3-18], wherein the solution is kept in a state not in contact with the filter before use.
[3-20] The method according to any one of [3-1] to [3-18], wherein the solution is in a state in contact with the filter before use.
[3-21] The method according to [3-19], wherein at the time of, or after production of the formulation for injection in which the solution is filled in the syringe, the solution is kept in a state not in contact with the filter before use.
[3-22] The method according to any one of [3-1] to [3-21], wherein the filter is not damaged, and an adherend between the filter and the housing is not peeled off even after discharging, through the filter, a solution having a viscosity of 95 cP or less at a discharge rate of 300 mm/min.
[3-23] The method according to any one of [3-1] to [3-22], wherein when a solution having a viscosity of 95 cP or less is discharged at a discharge rate of 100 mm/min through the filter, an average sliding resistance value for 10 mm is within 1.3-fold, 1.4-fold, or 1.5-fold as compared with that of a syringe not including a filter.
[3-24] The method according to any one of [3-1] to [3-23], wherein the solution comprises one or more pharmaceutically acceptable excipients selected from a sugar, a sugar alcohol, a buffer, a preservative, a carrier, an antioxidant, a chelating agent, a natural polymer, a synthetic polymer, a cryoprotective agent, an extending agent, and a stabilizing agent.
[3-25] The method according to any one of [3-1] to [3-24], wherein the solution is an aqueous solution.
[3-26] The method according to any one of [3-1] to [3-25], wherein the solution comprises emicizumab as an active ingredient.
[4-1] A syringe filled with a solution, comprising:
   a barrel formed in a cylindrical shape having a leading end portion and a base end portion, wherein the leading end portion is provided with a nozzle hole for externally ejecting a solution held therein; and a filter disposed within the barrel in a position on a base end side of the nozzle hole, the filter being provided in such a manner as to allow the solution to flow from the base end side to a leading end side through the filter,
   wherein the filter is a membrane, and a material of the membrane includes one or more selected from polyether sulfone (PES), polyvinylidene fluoride (PVDF), polysulfone (PS), and an acrylic copolymer, and the filter is not damaged, and an adherend between the filter and the housing is not peeled off even after discharging, through the filter, a solution having a viscosity of 95 cP or less at a discharge rate of 300 mm/min.
[4-2] A syringe filled with a solution, comprising:
   a barrel formed in a cylindrical shape having a leading end portion and a base end portion, wherein the leading end portion is provided with a nozzle hole for externally ejecting a solution held therein; and a filter disposed within the barrel in a position on a base end side of the nozzle hole, the filter being provided in such a manner as to allow the solution to flow from the base end side to a leading end side through the filter,
   wherein the filter is a membrane, and a material of the membrane includes one or more selected from polyether sulfone (PES), polyvinylidene fluoride (PVDF), polysulfone (PS), and an acrylic copolymer, and when a solution having a viscosity of 95 cP or less is discharged at a discharge rate of 100 mm/min through the filter, an average sliding resistance value for 10 mm is within 1.3-fold, 1.4-fold, or 1.5-fold as compared with that of a syringe not including a filter.
[4-3] The syringe according to [4-1] or [4-2], wherein the material of the membrane is an acrylic copolymer.
[4-4] The syringe according to [4-3], wherein the acrylic copolymer is hydrophobic.
[4-5] The syringe according to any one of [4-1] to [4-4], wherein the filter has a pore size of 5 µm or less, and preferably 5 µm.
[4-6] The syringe according to any one of [4-1] to [4-5], wherein a leading end side subspace is provided on the leading end side of the filter, a base end side subspace is provided on the base end side of the filter, and when seen from an axial direction of the barrel, the nozzle hole has an area smaller than an area of the base end side subspace, and smaller than an area of the leading end side subspace.
[4-7] The syringe according to any one of [4-1] to [4-6], wherein
   the syringe comprises partitioning means for partitioning an inside space of the barrel into a base end side space and a leading end side space,
   the partitioning means comprises: a cylindrical housing disposed to liquid-tightly contact an inner surface of the barrel: and an inner plug portion disposed in such a manner as to be openable/closable to the inside of the housing for communicating or non-communicating the base end side space and the leading end side space with each other through the inside of the housing,
   the housing has a close-time engaging portion,
   the inner plug portion has a close-time engaged portion to be engaged with the close-time engaging portion from the base end side,
   the partitioning means is constituted in such a manner that one of the close-time engaging portion and the close-time engaged portion moves beyond the other to release engagement between the close-time engaging portion and the close-time engaged portion when a pressing force is applied from the base end side by a piston inserted into the barrel from the base end side, and that the inner plug portion is opened to the inside of the housing to communicate the base end side space and the leading end side space with each other, and
   the filter is held in a leading end side portion of the housing.
[4-8] The syringe according to [4-7], wherein one of the close-time engaging portion and the close-time engaged portion is disposed on the leading end side and the base end side of the other of the close-time engaging portion and the close-time engaged portion.
[4-9] The syringe according to [4-7] or [4-8], wherein a material of the inner plug portion is linear low density polyethylene (LLDPE), or low density polyethylene (LDPE), and is preferably low density polyethylene (LDPE).
[4-10] The syringe according to any one of [4-1] to [4-6], wherein
   the syringe comprises partitioning means for partitioning an inside space of the barrel into a base end side space and a leading end side space,
   the partitioning means comprises a cylindrical housing disposed to liquid-tightly contact an inner surface of the barrel, and
   the filter is held in a leading end side portion of the housing.
[4-11] The syringe according to any one of [4-7] to [4-10], wherein the partitioning means is disposed in such a manner that a volume of the leading end side space of the barrel is 150 mm³ or less.
[4-12] The syringe according to any one of [4-7] to [4-11], wherein a material of the housing is linear low density polyethylene (LLDPE), or low density polyethylene (LDPE), and is preferably low density polyethylene (LDPE).
[4-13] The syringe according to any one of [4-1] to [4-12], wherein a compound contained in the solution as an active ingredient is a low molecular weight compound, a middle molecular weight compound, or a protein.
[4-14] The syringe according to [4-13], wherein the compound comprises an antibody or a bound fragment thereof, or a cyclic peptide.
[4-15] The syringe according to [4-13], wherein the low molecular weight compound has a molecular weight lower than 500.
[4-16] The syringe according to [4-13], wherein the middle molecular weight compound has a molecular weight of 500 to 15000.
[4-17] The syringe according to [4-13], wherein the compound is a polynucleotide selected from the group consisting of a ribozyme, an antisense molecule, an inhibitor oligonucleotide, an aptamer, a micro-RNA, and a small interfering RNA (siRNA).
[4-18] The syringe according to any one of [4-1] to [4-17], wherein the solution is kept in a state not in contact with the filter before use.
[4-19] The syringe according to any one of [4-1] to [4-17], wherein the solution is in a state in contact with the filter before use.
[4-20] The syringe according to [4-18], wherein at the time of, or after production of the formulation for injection in which the solution is filled in the syringe, the solution is kept in a state not in contact with the filter before use.
[4-21] The syringe according to any one of [4-1] to [4-20], wherein the solution comprises one or more pharmaceutically acceptable excipients selected from a sugar, a sugar alcohol, a buffer, a preservative, a carrier, an antioxidant, a chelating agent, a natural polymer, a synthetic polymer, a cryoprotective agent, an extending agent, and a stabilizing agent.
[4-22] The syringe according to any one of [4-1] to [4-21], wherein the solution is an aqueous solution.
[4-23] The syringe according to any one of [4-1] to [4-22], wherein the solution comprises emicizumab as an active ingredient.
[5-1] A formulation for injection in which a solution is filled in a syringe, for use as a pharmaceutical storable for 10 days or more, wherein
   the syringe comprises: a barrel formed in a cylindrical shape having a leading end portion and a base end portion, wherein the leading end portion is provided with a nozzle hole for externally ejecting a solution held therein; and a filter disposed within the barrel in a position on a base end side of the nozzle hole, the filter being provided in such a manner as to allow the solution to flow from the base end side to a leading end side through the filter, and
   the filter is a membrane, and a material of the membrane includes one or more selected from polyether sulfone (PES), polyvinylidene fluoride (PVDF), polysulfone (PS), and an acrylic copolymer.
[5-2] The formulation for injection according to [5-1], wherein physical properties of the formulation are less changed even after storage at 5°C, 25°C, or 40°C as compared with a formulation filled in a pre-filled syringe not including a filter.
[5-3] The formulation for injection according to [5-1] or [5-2], wherein the material of the membrane is an acrylic copolymer.
[5-4] The formulation for injection according to [5-3], wherein the acrylic copolymer is hydrophobic.
[5-5] The formulation for injection according to any one of [5-1] to [5-4], wherein the filter has a pore size of 5 µm or less, and preferably 5 µm.
[5-6] The formulation for injection according to any one of [5-1] to [5-5], wherein a leading end side subspace is provided on the leading end side of the filter, a base end side subspace is provided on the base end side of the filter, and when seen from an axial direction of the barrel, the nozzle hole has an area smaller than an area of the base end side subspace, and smaller than an area of the leading end side subspace.
[5-7] The formulation for injection according to any one of [5-1] to [5-6], wherein
   the syringe comprises partitioning means for partitioning an inside space of the barrel into a base end side space and a leading end side space,
   the partitioning means comprises: a cylindrical housing disposed to liquid-tightly contact an inner surface of the barrel; and an inner plug portion disposed in such a manner as to be openable/closable to the inside of the housing for communicating or non-communicating the base end side space and the leading end side space with each other through the inside of the housing,
   the housing has a close-time engaging portion,
   the inner plug portion has a close-time engaged portion to be engaged with the close-time engaging portion from the base end side,
   the partitioning means is constituted in such a manner that one of the close-time engaging portion and the close-time engaged portion moves beyond the other to release engagement between the close-time engaging portion and the close-time engaged portion when a pressing force is applied from the base end side by a piston inserted into the barrel from the base end side, and that the inner plug portion is opened to the inside of the housing to communicate the base end side space and the leading end side space with each other, and
   the filter is held in a leading end side portion of the housing.
[5-8] The formulation for injection according to [5-7], wherein one of the close-time engaging portion and the close-time engaged portion is disposed on the leading end side and the base end side of the other of the close-time engaging portion and the close-time engaged portion.
[5-9] The formulation for injection according to any one of [5-1] to [5-6], wherein
   the syringe comprises partitioning means for partitioning an inside space of the barrel into a base end side space and a leading end side space,
   the partitioning means comprises a cylindrical housing disposed to liquid-tightly contact an inner surface of the barrel, and
   the filter is held in a leading end side portion of the housing.
[5-10] The formulation for injection according to any one of [5-7] to [5-9], wherein the partitioning means is disposed in such a manner that a volume of the leading end side space of the barrel is 150 mm³ or less.
[5-11] The formulation for injection according to any one of [5-1] to [5-10], wherein a compound contained in the solution as an active ingredient is a low molecular weight compound, a middle molecular weight compound, or a protein.
[5-12] The formulation for injection according to [5-11], wherein the compound comprises an antibody or a bound fragment thereof, or a cyclic peptide.
[5-13] The formulation for injection according to [5-11], wherein the low molecular weight compound has a molecular weight lower than 500.
[5-14] The formulation for injection according to [5-11], wherein the middle molecular weight compound has a molecular weight of 500 to 15000.
[5-15] The formulation for injection according to [5-11], wherein the compound is a polynucleotide selected from the group consisting of a ribozyme, an antisense molecule, an inhibitor oligonucleotide, an aptamer, a micro-RNA, and a small interfering RNA (siRNA).
[5-16] The formulation for injection according to any one of [5-1] to [5-15], wherein the formulation for injection is kept in a state not in contact with the filter before use.
[5-17] The formulation for injection according to any one of [5-1] to [5-15], wherein the formulation for injection is in a state in contact with the filter before use.
[5-18] The formulation for injection according to [5-16], wherein at the time of, or after production of the formulation for injection in which the solution is filled in the syringe, the solution is kept in a state not in contact with the filter even under stress application.
[5-19] The formulation for injection according to any one of [5-1] to [5-18], wherein the filter is not damaged, and an adherend between the filter and the housing is not peeled off even after discharging, through the filter, a solution having a viscosity of 95 cP or less at a discharge rate of 300 mm/min.
[5-20] The formulation for injection according to any one of [5-1] to [5-19], wherein when a solution having a viscosity of 95 cP or less is discharged at a discharge rate of 100 mm/min through the filter, an average sliding resistance value for 10 mm is within 1.3-fold, 1.4-fold, or 1.5-fold as compared with that of a syringe not including a filter.
[5-21] The formulation for injection according to any one of [5-1] to [5-20], wherein the solution comprises one or more pharmaceutically acceptable excipients selected from a sugar, a sugar alcohol, a buffer, a preservative, a carrier, an antioxidant, a chelating agent, a natural polymer, a synthetic polymer, a cryoprotective agent, an extending agent, and a stabilizing agent.
[5-22] The formulation for injection according to any one of [5-1] to [5-21], wherein the solution is an aqueous solution.
[5-23] The formulation for injection according to any one of [5-1] to [5-22], wherein the solution comprises emicizumab as an active ingredient.
[6-1] A formulation for injection in which a solution comprising a protein as an active ingredient is filled in a syringe, wherein
   the syringe comprises:
   a barrel formed in a cylindrical shape having a leading end portion and a base end portion, wherein the leading end portion is provided with a nozzle hole for externally ejecting a solution held therein; and
   a filter disposed within the barrel in a position on a base end side of the nozzle hole, wherein
   the filter is provided in such a manner as to allow the solution to flow from the base end side to a leading end side through the filter, and
   the filter is a membrane, and a material of the membrane includes one or more selected from polyether sulfone (PES), polyvinylidene fluoride (PVDF), polysulfone (PS), and an acrylic copolymer.
[6-2] The formulation for injection according to [6-1], wherein the material of the membrane is an acrylic copolymer.
[6-3] The formulation for injection according to [6-2], wherein the acrylic copolymer is hydrophobic.
[6-4] The formulation for injection according to any one of [6-1] to [6-3], wherein the filter has a pore size of 5 µm or less, and preferably 5 µm.
[6-5] The formulation for injection according to any one of [6-1] to [6-4], wherein the leading end side of the filter is provided with a leading end side subspace, the base end side of the filter is provided with a base end side subspace, and when seen from an axial direction of the barrel, the nozzle hole has an area smaller than an area of the base end side subspace, and smaller than an area of the leading end side subspace.
[6-6] The formulation for injection according to any one of [6-1] to [6-5], wherein
   the syringe comprises partitioning means for partitioning an inside space of the barrel into a base end side space and a leading end side space,
   the partitioning means comprises: a cylindrical housing disposed to liquid-tightly contact an inner surface of the barrel; and an inner plug portion disposed in such a manner as to be openable/closable to an inside of the housing for communicating or non-communicating the base end side space and the leading end side space with each other through the inside of the housing,
   the housing has a close-time engaging portion,
   the inner plug portion has a close-time engaged portion to be engaged with the close-time engaging portion from the base end side,
   the partitioning means is constituted in such a manner that one of the close-time engaging portion and the close-time engaged portion moves beyond the other to release engagement between the close-time engaging portion and the close-time engaged portion when a pressing force is applied from the base end side by a piston inserted into the barrel from the base end side, and that the inner plug portion is opened to the inside of the housing to communicate the base end side space and the leading end side space with each other, and
   the filter is held in a leading end side portion of the housing.
[6-7] The formulation for injection according to [6-6], wherein one of the close-time engaging portion and the close-time engaged portion is disposed on the leading end side and the base end side of the other of the close-time engaging portion and the close-time engaged portion.
[6-8] The formulation for injection according to [6-6] or [6-7], wherein a material of the inner plug portion is linear low density polyethylene (LLDPE), or low density polyethylene (LDPE), and is preferably low density polyethylene (LDPE).
[6-9] The formulation for injection according to any one of [6-1] to [6-5], wherein
   the syringe comprises partitioning means for partitioning an inside space of the barrel into a base end side space and a leading end side space,
   the partitioning means comprises a cylindrical housing disposed to liquid-tightly contact an inner surface of the barrel, and
   the filter is held in a leading end side portion of the housing.
[6-10] The formulation for injection according to any one of [6-6] to [6-9], wherein the partitioning means is disposed in such a manner that a volume of the leading end side space of the barrel is 150 mm³ or less.
[6-11] The formulation for injection according to any one of [6-6] to [6-10], wherein a material of the housing is linear low density polyethylene (LLDPE), or low density polyethylene (LDPE), and is preferably low density polyethylene (LDPE).
[6-12] The formulation for injection according to any one of [6-1] to [6-11], wherein the protein is a monoclonal antibody.
[6-13] The formulation for injection according to any one of [6-1] to [6-12], wherein the solution is kept in a state not in contact with the filter before use.
[6-14] The formulation for injection according to any one of [6-1] to [6-12], wherein the solution is in a state in contact with the filter before use.
[6-15] The formulation for injection according to [6-13], wherein at the time of, or after production of the formulation for injection in which the solution is filled in the syringe, the solution is kept in a state not in contact with the filter before use.
[6-16] The formulation for injection according to any one of [6-1] to [6-15], wherein a concentration of the protein in the solution is 0.1 mg/mL or more.
[6-17] The formulation for injection according to any one of [6-1] to [6-16], wherein a concentration of the protein in the solution is in the range of 0.1 to 300 mg/mL.
[6-18] The formulation for injection according to any one of [6-1] to [6-17], wherein a concentration of the protein in the solution is in the range of 1 to 200 mg/mL, 50 to 200 mg/mL, or 80 to 200 mg/mL.
[6-19] The formulation for injection according to any one of [6-1] to [6-18], wherein the filter is not damaged, and an adherend between the filter and the housing is not peeled off even after discharging, through the filter, a solution having a viscosity of 95 cP or less at a discharge rate of 300 mm/min.
[6-20] The formulation for injection according to any one of [6-1] to [6-19], wherein when a solution having a viscosity of 95 cP or less is discharged at a discharge rate of 100 mm/min through the filter, an average sliding resistance value for 10 mm is within 1.3-fold, 1.4-fold, or 1.5-fold as compared with that of a syringe not including a filter.
[6-21] The formulation for injection according to any one of [6-1] to [6-20], wherein the solution comprises one or more pharmaceutically acceptable excipients selected from a sugar, a sugar alcohol, a buffer, a preservative, a carrier, an antioxidant, a chelating agent, a natural polymer, a synthetic polymer, a cryoprotective agent, an extending agent, and a stabilizing agent.
[6-22] The formulation for injection according to any one of [6-1] to [6-21] comprising a needle to be connected to the syringe.
[6-23] The formulation for injection according to any one of [6-1] to [6-22], wherein the solution is an aqueous solution.
[6-24] The formulation for injection according to any one of [6-1] to [6-23], wherein the protein is emicizumab.
[7-1] A formulation for injection in which a solution comprising a protein as an active ingredient is filled in a syringe, wherein
   the syringe comprises:
      a barrel formed in a cylindrical shape having a leading end portion and a base end portion, wherein the leading end portion is provided with a nozzle hole for externally ejecting a solution held therein; and
      a filter disposed within the barrel in a position on a base end side of the nozzle hole, wherein
      the filter is provided in such a manner as to allow the solution to flow from the base end side to a leading end side through the filter,
      the filter is a membrane, and a material of the membrane includes one or more selected from polyether sulfone (PES), polyvinylidene fluoride (PVDF), polysulfone (PS), and an acrylic copolymer, and
   in discharging the solution, visually detectable particles are removable from the discharged solution.
[7-2] The formulation for injection according to [7-1], wherein the material of the membrane is an acrylic copolymer.
[7-3] The formulation for injection according to [7-2], wherein the acrylic copolymer is hydrophobic.
[7-4] The formulation for injection according to any one of [7-1] to [7-3], wherein the filter has a pore size of 5 µm or less, and preferably 5 µm.
[7-5] The formulation for injection according to any one of [7-1] to [7-4], wherein a leading end side subspace is provided on the leading end side of the filter, a base end side subspace is provided on the base end side of the filter, and when seen from an axial direction of the barrel, the nozzle hole has an area smaller than an area of the base end side subspace, and smaller than an area of the leading end side subspace.
[7-6] The formulation for injection according to any one of [7-1] to [7-5], wherein
   the syringe comprises partitioning means for partitioning an inside space of the barrel into a base end side space and a leading end side space,
   the partitioning means comprises: a cylindrical housing disposed to liquid-tightly contact an inner surface of the barrel; and an inner plug portion disposed in such a manner as to be openable/closable to an inside of the housing for communicating or non-communicating the base end side space and the leading end side space with each other through the inside of the housing,
   the housing has a close-time engaging portion,
   the inner plug portion has a close-time engaged portion to be engaged with the close-time engaging portion from the base end side,
   the partitioning means is constituted in such a manner that one of the close-time engaging portion and the close-time engaged portion moves beyond the other to release engagement between the close-time engaging portion and the close-time engaged portion when a pressing force is applied from the base end side by a piston inserted into the barrel from the base end side, and that the inner plug portion is opened to the inside of the housing to communicate the base end side space and the leading end side space with each other, and
   the filter is held in a leading end side portion of the housing.
[7-7] The formulation for injection according to [7-6], wherein one of the close-time engaging portion and the close-time engaged portion is disposed on the leading end side and the base end side of the other of the close-time engaging portion and the close-time engaged portion.
[7-8] The formulation for injection according to any one of [7-1] to [7-5], wherein
   the syringe comprises partitioning means for partitioning an inside space of the barrel into a base end side space and a leading end side space,
   the partitioning means comprises a cylindrical housing disposed to liquid-tightly contact an inner surface of the barrel, and
   the filter is held in a leading end side portion of the housing.
[7-9] The formulation for injection according to any one of [7-6] to [7-8], wherein the partitioning means is disposed in such a manner that a volume of the leading end side space of the barrel is 150 mm³ or less.
[7-10] The formulation for injection according to any one of [7-1] to [7-9], wherein the particles have a particle size larger than 40 µm.
[7-11] The formulation for injection according to any one of [7-1] to [7-10], wherein the particles include a particle derived from a component of the solution, or a particle derived from a substance except for the component of the solution.
[7-12] The formulation for injection according to [7-11], wherein the particles are derived from a component of the solution.
[7-13] The formulation for injection according to [7-11], wherein the particles are derived from a substance except for the component of the solution.
[7-14] The formulation for injection according to any one of [7-1] to [7-13], wherein the protein is a monoclonal antibody.
[7-15] The formulation for injection according to any one of [7-1] to [7-14], wherein the solution is kept in a state not in contact with the filter before use.
[7-16] The formulation for injection according to any one of [7-1] to [7-14], wherein the solution is in a state in contact with the filter before use.
[7-17] The formulation for injection according to [7-15], wherein at the time of, or after production of the formulation for injection in which the solution is filled in the syringe, the solution is kept in a state not in contact with the filter.
[7-18] The formulation for injection according to any one of [7-1] to [7-17], wherein a concentration of the protein in the solution is 0.1 mg/mL or more.
[7-19] The formulation for injection according to any one of [7-1] to [7-18], wherein a concentration of the protein in the solution is in the range of 0.1 to 300 mg/mL.
[7-20] The formulation for injection according to any one of [7-1] to [7-19], wherein a concentration of the protein in the solution is in the range of 1 to 200 mg/mL, 50 to 200 mg/mL, or 80 to 200 mg/mL.
[7-21] The formulation for injection according to any one of [7-1] to [7-20], wherein the filter is not damaged, and an adherend between the filter and the housing is not peeled off even after discharging, through the filter, a solution having a viscosity of 95 cP or less at a discharge rate of 300 mm/min.
[7-22] The formulation for injection according to any one of [7-1] to [7-21], wherein when a solution having a viscosity of 95 cP or less is discharged at a discharge rate of 100 mm/min through the filter, an average sliding resistance value for 10 mm is within 1.3-fold, 1.4-fold, or 1.5-fold as compared with that of a syringe not including a filter.
[7-23] The formulation for injection according to any one of [7-1] to [7-22], wherein the solution comprises one or more pharmaceutically acceptable excipients selected from a sugar, a sugar alcohol, a buffer, a preservative, a carrier, an antioxidant, a chelating agent, a natural polymer, a synthetic polymer, a cryoprotective agent, an extending agent, and a stabilizing agent.
[7-24] The formulation for injection according to any one of [7-1] to [7-23], wherein the solution is an aqueous solution.
[7-25] The formulation for injection according to any one of [7-1] to [7-24], wherein the protein is emicizumab.
[8-1] A method for removing visually detectable particles contained in a formulation for injection, wherein
   the formulation for injection is a solution comprising a protein as an active ingredient, and is filled in a syringe,
   the syringe comprises a barrel formed in a cylindrical shape having a leading end portion and a base end portion, wherein the leading end portion is provided with a nozzle hole for externally ejecting the formulation for injection held therein; and a filter disposed within the barrel in a position on a base end side of the nozzle hole, the filter being provided in such a manner as to allow the solution to flow from the base end side to a leading end side through the filter, and
   the method comprises discharging the solution filled in the syringe through the filter, and the filter is a membrane, and a material of the membrane includes one or more selected from polyether sulfone (PES), polyvinylidene fluoride (PVDF), polysulfone (PS), and an acrylic copolymer.
[8-2] The method according to [8-1], wherein the material of the membrane is an acrylic copolymer.
[8-3] The method according to [8-2], wherein the acrylic copolymer is hydrophobic.
[8-4] The method according to any one of [8-1] to [8-3], wherein the filter has a pore size of 5 µm or less, and preferably 5 µm.
[8-5] The method according to any one of [8-1] to [8-4], wherein a leading end side subspace is provided on the leading end side of the filter, a base end side subspace is provided on the base end side of the filter, and when seen from an axial direction of the barrel, the nozzle hole has an area smaller than an area of the base end side subspace, and smaller than an area of the leading end side subspace.
[8-6] The method according to any one of [8-1] to [8-5], wherein
   the syringe comprises partitioning means for partitioning an inside space of the barrel into a base end side space and a leading end side space,
   the partitioning means comprises: a cylindrical housing disposed to liquid-tightly contact an inner surface of the barrel; and an inner plug portion disposed in such a manner as to be openable/closable to the inside of the housing for communicating or non-communicating the base end side space and the leading end side space with each other through the inside of the housing,
   the housing has a close-time engaging portion,
   the inner plug portion has a close-time engaged portion to be engaged with the close-time engaging portion from the base end side,
   the partitioning means is constituted in such a manner that one of the close-time engaging portion and the close-time engaged portion moves beyond the other to release engagement between the close-time engaging portion and the close-time engaged portion when a pressing force is applied from the base end side by a piston inserted into the barrel from the base end side, and that the inner plug portion is opened to the inside of the housing to communicate the base end side space and the leading end side space with each other, and
   the filter is held in a leading end side portion of the housing.
[8-7] The method according to [8-6], wherein one of the close-time engaging portion and the close-time engaged portion is disposed on the leading end side and the base end side of the other of the close-time engaging portion and the close-time engaged portion.
[8-8] The method according to any one of [8-1] to [8-5], wherein
   the syringe comprises partitioning means for partitioning an inside space of the barrel into a base end side space and a leading end side space,
   the partitioning means comprises a cylindrical housing disposed to liquid-tightly contact an inner surface of the barrel, and
   the filter is held in a leading end side portion of the housing.
[8-9] The method according to any one of [8-6] to [8-8], wherein the partitioning means is disposed in such a manner that a volume of the leading end side space of the barrel is 150 mm³ or less.
[8-10] The method according to any one of [8-1] to [8-9], wherein the particles have a particle size larger than 40 µm.
[8-11] The method according to any one of [8-1] to [8-10], wherein the particles include a particle derived from a component of the solution, or a particle derived from a substance except for the component of the solution.
[8-12] The method according to [8-11], wherein the particles are derived from a component of the solution.
[8-13] The method according to [8-11], wherein the particles are derived from a substance except for a component of the solution.
[8-14] The method according to any one of [8-1] to [8-13], wherein the protein is a monoclonal antibody.
[8-15] The method according to any one of [8-1] to [8-14], wherein the solution is kept in a state not in contact with the filter before use.
[8-16] The method according to any one of [8-1] to [8-14], wherein the solution is in a state in contact with the filter before use.
[8-17] The method according to [8-15], wherein at the time of, or after production of the formulation for injection in which the solution comprising a protein as an active ingredient is filled in the syringe, the solution is kept in a state not in contact with the filter before use.
[8-18] The method according to any one of [8-1] to [8-17], wherein a concentration of the protein in the solution is 0.1 mg/mL or more.
[8-19] The method according to any one of [8-1] to [8-18], wherein a concentration of the protein in the solution is in the range of 0.1 to 300 mg/mL.
[8-20] The method according to any one of [8-1] to [8-19], wherein a concentration of the protein in the solution is in the range of 1 to 200 mg/mL, 50 to 200 mg/mL, or 80 to 200 mg/mL.
[8-21] The method according to any one of [8-1] to [8-20], wherein the filter is not damaged, and an adherend between the filter and the housing is not peeled off even after discharging, through the filter, a solution having a viscosity of 95 cP or less at a discharge rate of 300 mm/min.
[8-22] The method according to any one of [8-1] to [8-21], wherein when a solution having a viscosity of 95 cP or less is discharged at a discharge rate of 100 mm/min through the filter, an average sliding resistance value for 10 mm is within 1.3-fold, 1.4-fold, or 1.5-fold as compared with that of a syringe not including a filter.
[8-23] The method according to any one of [8-1] to [8-22], wherein the solution comprises one or more pharmaceutically acceptable excipients selected from a sugar, a sugar alcohol, a buffer, a preservative, a carrier, an antioxidant, a chelating agent, a natural polymer, a synthetic polymer, a cryoprotective agent, an extending agent, and a stabilizing agent.
[8-24] The method according to any one of [8-1] to [8-23], wherein the solution is an aqueous solution.
[8-25] The method according to any one of [8-1] to [8-24], wherein the protein is emicizumab.
[9-1] A syringe filled with a solution comprising a protein as an active ingredient, the syringe comprising:
   a barrel formed in a cylindrical shape having a leading end portion and a base end portion, wherein the leading end portion is provided with a nozzle hole for externally ejecting a solution held therein; and a filter disposed within the barrel in a position on a base end side of the nozzle hole, the filter being provided in such a manner as to allow the solution to flow from the base end side to a leading end side through the filter,
   wherein the filter is a membrane, and a material of the membrane includes one or more selected from polyether sulfone (PES), polyvinylidene fluoride (PVDF), polysulfone (PS), and an acrylic copolymer, and the filter is not damaged, and an adherend between the filter and the housing is not peeled off even after discharging, through the filter, a solution having a viscosity of 95 cP or less at a discharge rate of 300 mm/min.
[9-2] A syringe filled with a solution comprising a protein as an active ingredient, the syringe comprising:
   a barrel formed in a cylindrical shape having a leading end portion and a base end portion, wherein the leading end portion is provided with a nozzle hole for externally ejecting a solution held therein; and a filter disposed within the barrel in a position on a base end side of the nozzle hole, the filter being provided in such a manner as to allow the solution to flow from the base end side to a leading end side through the filter,
   wherein the filter is a membrane, and a material of the membrane includes one or more selected from polyether sulfone (PES), polyvinylidene fluoride (PVDF), polysulfone (PS), and an acrylic copolymer, and when a solution having a viscosity of 95 cP or less is discharged at a discharge rate of 100 mm/min through the filter, an average sliding resistance value for 10 mm is within 1.3-fold, 1.4-fold, or 1.5-fold as compared with that of a syringe not including a filter.
[9-3] The syringe according to [9-1] or [9-2], wherein the material of the membrane is an acrylic copolymer.
[9-4] The syringe according to [9-3], wherein the acrylic copolymer is hydrophobic.
[9-5] The syringe according to any one of [9-1] to [9-4], wherein the filter has a pore size of 5 µm or less, and preferably 5 µm.
[9-6] The syringe according to any one of [9-1] to [9-5], wherein a leading end side subspace is provided on the leading end side of the filter, a base end side subspace is provided on the base end side of the filter, and when seen from an axial direction of the barrel, the nozzle hole has an area smaller than an area of the base end side subspace, and smaller than an area of the leading end side subspace.
[9-7] The syringe according to any one of [9-1] to [9-6], wherein
   the syringe comprises partitioning means for partitioning an inside space of the barrel into a base end side space and a leading end side space,
   the partitioning means comprises: a cylindrical housing disposed to liquid-tightly contact an inner surface of the barrel; and an inner plug portion disposed in such a manner as to be openable/closable to an inside of the housing for communicating or non-communicating the base end side space and the leading end side space with each other through the inside of the housing,
   the housing has a close-time engaging portion,
   the inner plug portion has a close-time engaged portion to be engaged with the close-time engaging portion from the base end side,
   the partitioning means is constituted in such a manner that one of the close-time engaging portion and the close-time engaged portion moves beyond the other to release engagement between the close-time engaging portion and the close-time engaged portion when a pressing force is applied from the base end side by a piston inserted into the barrel from the base end side, and that the inner plug portion is opened to the inside of the housing to communicate the base end side space and the leading end side space with each other, and
   the filter is held in a leading end side portion of the housing.
[9-8] The syringe according to [9-7], wherein one of the close-time engaging portion and the close-time engaged portion is disposed on the leading end side and the base end side of the other of the close-time engaging portion and the close-time engaged portion.
[9-9] The syringe according to [9-7] or [9-8], wherein a material of the inner plug portion is linear low density polyethylene (LLDPE), or low density polyethylene (LDPE), and is preferably low density polyethylene (LDPE).
[9-10] The syringe according to any one of [9-1] to [9-6], wherein
   the syringe comprises partitioning means for partitioning an inside space of the barrel into a base end side space and a leading end side space,
   the partitioning means comprises a cylindrical housing disposed to liquid-tightly contact an inner surface of the barrel, and
   the filter is held in a leading end side portion of the housing.
[9-11] The syringe according to any one of [9-7] to [9-10], wherein the partitioning means is disposed in such a manner that a volume of the leading end side space of the barrel is 150 mm³ or less.
[9-12] The syringe according to any one of [9-7] to [9-11], wherein a material of the housing is linear low density polyethylene (LLDPE), or low density polyethylene (LDPE), and is preferably low density polyethylene (LDPE).
[9-13] The syringe according to any one of [9-1] to [9-12], wherein the protein is a monoclonal antibody.
[9-14] The syringe according to any one of [9-1] to [9-13], wherein the solution is kept in a state not in contact with the filter before use.
[9-15] The syringe according to any one of [9-1] to [9-13], wherein the solution is in a state in contact with the filter before use.
[9-16] The syringe according to [9-14], wherein at the time of, or after production of the formulation for injection in which the solution comprising a protein as an active ingredient is filled in the syringe, the solution is kept in a state not in contact with the filter before use.
[9-17] The syringe according to any one of [9-1] to [9-16], wherein a concentration of the protein in the solution is 0.1 mg/mL or more.
[9-18] The syringe according to any one of [9-1] to [9-17], wherein a concentration of the protein in the solution is in the range of 0.1 to 300 mg/mL.
[9-19] The syringe according to any one of [9-1] to [9-18], wherein a concentration of the protein in the solution is in the range of 1 to 200 mg/mL, 50 to 200 mg/mL, or 80 to 200 mg/mL.
[9-20] The syringe according to any one of [9-1] to [9-19], wherein the solution comprises one or more pharmaceutically acceptable excipients selected from a sugar, a sugar alcohol, a buffer, a preservative, a carrier, an antioxidant, a chelating agent, a natural polymer, a synthetic polymer, a cryoprotective agent, an extending agent, and a stabilizing agent.
[9-21] The syringe according to any one of [9-1] to [9-20], wherein the solution is an aqueous solution.
[9-22] The syringe according to any one of [9-1] to [9-21], wherein the protein is emicizumab.
[10-1] A formulation for injection in which a solution is filled in a syringe, for use as a pharmaceutical storable for 10 days or more, wherein
   the syringe comprises: a barrel formed in a cylindrical shape having a leading end portion and a base end portion, and having, in the leading end portion, a nozzle hole for externally ejecting a solution held therein; and a filter disposed within the barrel in a position on a base end side of the nozzle hole, the filter being provided in such a manner as to allow the solution to flow from the base end side to a leading end side through the filter, and
   the filter is a membrane, and a material thereof includes one or more selected from polyether sulfone (PES), polyvinylidene fluoride (PVDF), polysulfone (PS), and an acrylic copolymer.
[10-2] The formulation for injection according to [10-1], wherein physical properties of the formulation are less changed even after storage at 5°C, 25°C, or 40°C as compared with a formulation filled in a pre-filled syringe not including a filter.
[10-3] The formulation for injection according to [10-1] or [10-2], wherein the material of the membrane is an acrylic copolymer.
[10-4] The formulation for injection according to [10-3], wherein the acrylic copolymer is hydrophobic.
[10-5] The formulation for injection according to any one of [10-1] to [10-4], wherein the filter has a pore size of 5 µm or less, and preferably 5 µm.
[10-6] The formulation for injection according to any one of [10-1] to [10-5], wherein a leading end side subspace is provided on the leading end side of the filter, a base end side subspace is provided on the base end side of the filter, and when seen from an axial direction of the barrel, the nozzle hole has an area smaller than an area of the base end side subspace, and smaller than an area of the leading end side subspace.
[10-7] The formulation for injection according to any one of [10-1] to [10-6], wherein
   the syringe comprises partitioning means for partitioning an inside space of the barrel into a base end side space and a leading end side space,
   the partitioning means comprises: a cylindrical housing disposed to liquid-tightly contact an inner surface of the barrel; and an inner plug portion disposed in such a manner as to be openable/closable to an inside of the housing for communicating or non-communicating the base end side space and the leading end side space with each other through the inside of the housing,
   the housing has a close-time engaging portion,
   the inner plug portion has a close-time engaged portion to be engaged with the close-time engaging portion from the base end side,
   the partitioning means is constituted in such a manner that one of the close-time engaging portion and the close-time engaged portion moves beyond the other to release engagement between the close-time engaging portion and the close-time engaged portion when a pressing force is applied from the base end side by a piston inserted into the barrel from the base end side, and that the inner plug portion is opened to the inside of the housing to communicate the base end side space and the leading end side space with each other, and
   the filter is held in a leading end side portion of the housing.
[10-8] The formulation for injection according to [10-7], wherein one of the close-time engaging portion and the close-time engaged portion is disposed on the leading end side and the base end side of the other of the close-time engaging portion and the close-time engaged portion.
[10-9] The formulation for injection according to any one of [10-1] to [10-6], wherein
   the syringe comprises partitioning means for partitioning an inside space of the barrel into a base end side space and a leading end side space,
   the partitioning means comprises a cylindrical housing disposed to liquid-tightly contact an inner surface of the barrel, and
   the filter is held in a leading end side portion of the housing.
[10-10] The formulation for injection according to any one of [10-7] to [10-9], wherein the partitioning means is disposed in such a manner that a volume of the leading end side space of the barrel is 150 mm³ or less.
[10-11] The formulation for injection according to any one of [10-1] to [10-10], wherein the protein is a monoclonal antibody.
[10-12] The formulation for injection according to any one of [10-1] to [10-11], wherein the formulation for injection is kept in a state not in contact with the filter before use.
[10-13] The formulation for injection according to any one of [10-1] to [10-11], wherein the formulation for injection is in a state in contact with the filter before use.
[10-14] The formulation for injection according [10-12], wherein at the time of, or after production of the formulation for injection in which the solution comprising a protein as an active ingredient is filled in the syringe, the solution is kept in a state not in contact with the filter even under stress application.
[10-15] The formulation for injection according to any one of [10-1] to [10-14], wherein a concentration of the protein in the solution is 0.1 mg/mL or more.
[10-16] The formulation for injection according to any one of [10-1] to [10-15], wherein a concentration of the protein in the solution is in the range of 0.1 to 300 mg/mL.
[10-17] The formulation for injection according to any one of [10-1] to [10-16], wherein a concentration of the protein in the solution is in the range of 1 to 200 mg/mL, 50 to 200 mg/mL, or 80 to 200 mg/mL.
[10-18] The formulation for injection according to any one of [10-1] to [10-17], wherein the filter is not damaged, and an adherend between the filter and the housing is not peeled off even after discharging, through the filter, a solution having a viscosity of 95 cP or less at a discharge rate of 300 mm/min.
[10-19] The formulation for injection according to any one of [10-1] to [10-18], wherein when a solution having a viscosity of 95 cP or less is discharged at a discharge rate of 100 mm/min through the filter, an average sliding resistance value for 10 mm is within 1.3-fold, 1.4-fold, or 1.5-fold as compared with that of a syringe not including a filter.
[10-20] The formulation for injection according to any one of [10-1] to [10-19], wherein the solution comprises one or more pharmaceutically acceptable excipients selected from a sugar, a sugar alcohol, a buffer, a preservative, a carrier, an antioxidant, a chelating agent, a natural polymer, a synthetic polymer, a cryoprotective agent, an extending agent, and a stabilizing agent.
[10-21] The formulation for injection according to any one of [10-1] to [10-20], wherein the solution is an aqueous solution.
[10-22] The formulation for injection according to any one of [10-1] to [10-21], wherein the solution comprises emicizumab as an active ingredient.

### ADVANTAGEOUS EFFECTS OF INVENTION

According to the present invention, even if visually detectable particles are present in a solution of a formulation for injection filled in a syringe, the whole amount of the solution filled in the syringe is filtered through a filter at the time of discharge, and therefore, a high quality formulation free from the particles can be supplied.

### BRIEF DESCRIPTION OF DRAWINGS

Fig. 1 is a cross-sectional view for describing a close state of an inner plug portion in a syringe of a pre-filled syringe according to an embodiment of the present invention.
Fig. 2 is a cross-sectional view for describing an open state of the inner plug portion in the pre-filled syringe.
Fig. 3 is an enlarged cross-sectional view of a region III of Fig. 1.
Fig. 4 is an enlarged cross-sectional view of a region IV of Fig. 2.
Fig. 5 is a perspective view of the inner plug portion in the syringe.
Fig. 6 is another perspective view of the inner plug portion.
Fig. 7 is a side view for describing a close state of an inner plug portion in a syringe of a pre-filled syringe according to a modification.
Fig. 8 is a cross-sectional view for describing an open state of the inner plug portion in the pre-filled syringe.
Fig. 9 is an enlarged cross-sectional view of a region IX of Fig. 7.
Fig. 10 is an enlarged cross-sectional view of a region X of Fig. 8.
Fig. 11 is a perspective view of the inner plug portion in the syringe.
Fig. 12 is another perspective view of the inner plug portion.
Fig. 13 illustrates schematic cross-sectional views of a pre-filled syringe according to a modification in use, wherein Fig. 13(a) illustrates a close state of an inner plug portion, Fig. 13(b) illustrates a state immediately after opening the inner plug portion, Fig. 13(c) illustrates an open state of the inner plug portion, and Fig. 13(d) illustrates a state after administering a drug.
Fig. 14 illustrates schematic cross-sectional views of a pre-filled syringe according to a modification in use, wherein Fig. 14(a) illustrates a close state of the inner plug portion, Fig. 14(b) illustrates a state immediately after opening the inner plug portion, Fig. 14(c) illustrates an open state of the inner plug portion, and Fig. 14(d) illustrates a state after administering a drug.

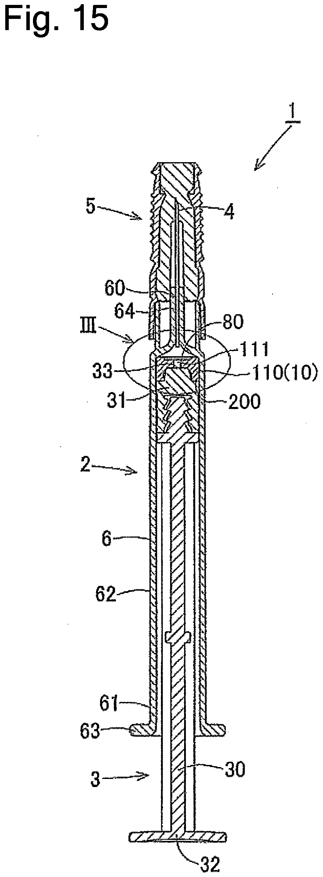

Fig. 15 is a cross-sectional view of a syringe of a pre-filled syringe including a holding member.
Fig. 16 is an enlarged cross-sectional view of a region III of Fig. 15.
Fig. 17 is a schematic diagram for describing attachment of a filter in the holding member.
Fig. 18 is a side view of a syringe of a pre-filled syringe according to a modification.
Fig. 19 is an enlarged cross-sectional view of a region VI of Fig. 18.
Fig. 20 is a side view of a syringe according to a modification.
Fig. 21 is an enlarged cross-sectional view of a region VIII of Fig. 20.
Fig. 22 illustrates photographs, taken after discharging a solution, of a normal filter not damaged (Fig. 22(a)), and an abnormal damaged filter (Fig. 22(b)).

### DESCRIPTION OF EMBODIMENTS

### (1) Pre-filled Syringe

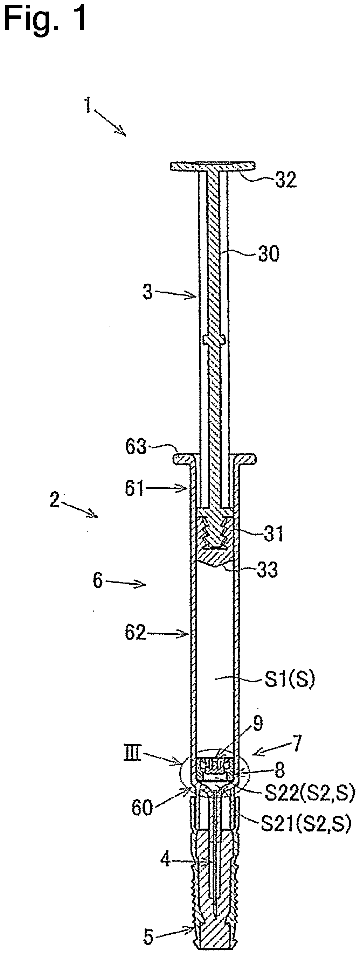

A pre-filled syringe including a syringe according to an embodiment of the present invention will now be described with reference to Figs. 1 to 6.

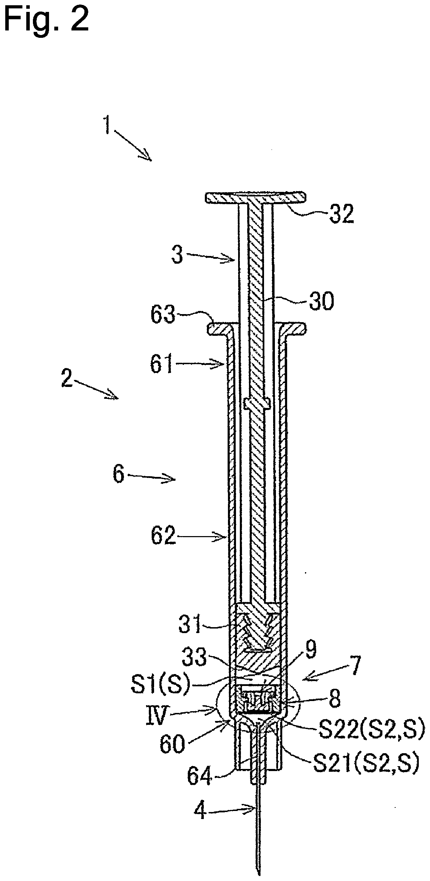

A pre-filled syringe 1 includes a syringe 2 for administering a drug as illustrated in Figs. 1 and 2. The pre-filled syringe 1 also includes a piston 3 to be inserted into the syringe 2. The pre-filled syringe 1 further includes a needle 4 to be connected to the syringe 2. The pre-filled syringe 1 includes a cap 5 for capping the needle 4. The drug is a liquid drug, such as a protein formulation.

The syringe 2 is formed in a cylindrical shape including a leading end portion 60 and a base end portion 61, and includes a barrel 6, and partitioning means 7 for partitioning a space S within the barrel 6 into a base end side space S1 and a leading end side space S2. Besides, the syringe 2 is in a substantially cylindrical shape.

Hereinafter, in the pre-filled syringe 1 and the syringe 2, a side in which the leading end portion 60 is disposed (lower sides in Figs. 1 to 4) is simply referred to as the "leading end side", and a side in which the base end portion 61 is disposed (upper sides in Figs. 1 to 4) is referred to as the "base end side". Besides, an axial direction of the syringe 2 is also simply referred to as the "axial direction".

The barrel 6 is a member for holding a drug therein. Besides, in the barrel 6, the leading end portion 60 is provided with a nozzle hole for externally ejecting the drug held therein. In the present embodiment, the barrel 6 includes, in addition to the leading end portion 60 and the base end portion 61, a cylindrical portion 62 for connecting the leading end portion 60 and the base end portion 61 to each other. Besides, the barrel 6 includes a flange portion 63 extending outward (radially outward direction of the cylindrical portion 62) from the entire outer circumference of the other end in the axial direction of the cylindrical portion 62.

The barrel 6 is formed, for example, from a material that is transparent and can withstand an internal pressure applied in administering the drug. Specifically, the material of the barrel 6 is a resin containing cyclic olefin such as norbornene in a repeating unit. More specifically, examples of the material of the barrel 6 include transparent resins such as COP (cycloolefin polymer) that is a homopolymer of cyclic olefin, and COC (cycloolefin copolymer) that is a copolymer of cyclic olefin and ethylene or the like. The material of the barrel 6 may be PP (polypropylene) or glass.

On the inner surface of the barrel 6 (for example, the inner surface of the cylindrical portion 62), silicone oil may be applied for reducing sliding resistance of the piston 3 against the inner surface of the barrel 6.

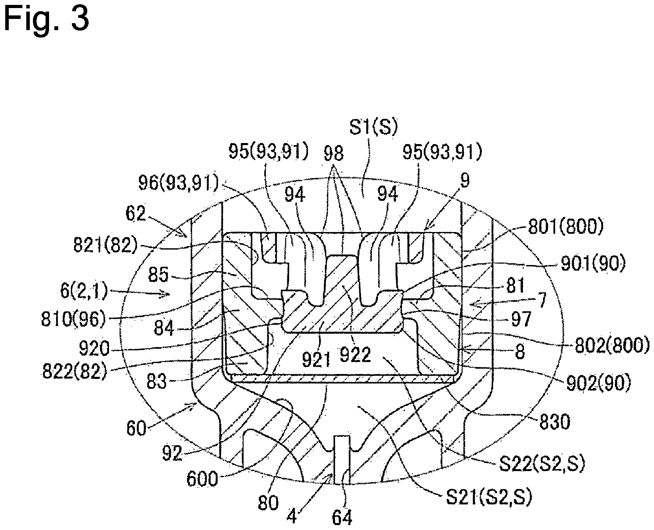

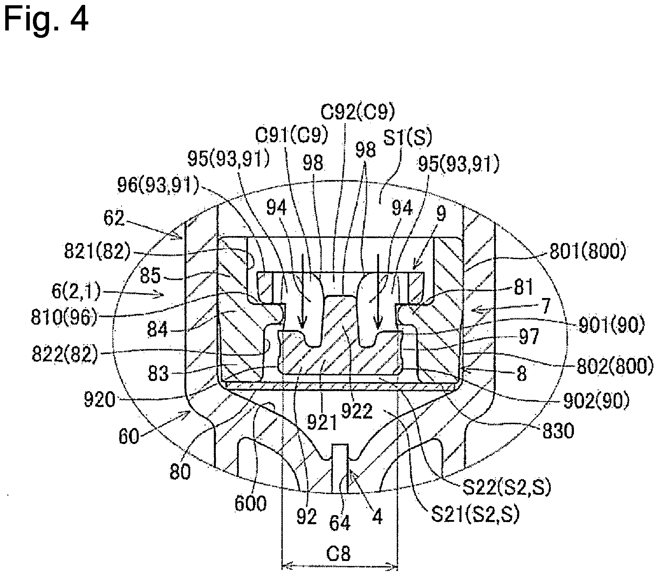

The leading end portion 60 of the barrel 6 is provided at one end (specifically, the end on the leading end side) in the axial direction of the cylindrical portion 62. The leading end portion 60 is provided, as illustrated in Figs. 3 and 4, with a nozzle hole 64 for externally ejecting the drug held therein. In the barrel 6 of the present embodiment, the nozzle hole 64 corresponds to a needle hole into which the needle 4 is to be inserted.

When the needle to be inserted into the nozzle hole 64 has a diameter of, for example, 27 gauge, the inner diameter of the nozzle hole 64 is 0.27 mm, and the area seen from the axial direction of the nozzle hole 64 is 0.057 mm². Alternatively, when the needle to be inserted into the nozzle hole 64 has a diameter of 29 gauge, the inner diameter of the nozzle hole 64 is 0.21 mm, and the area seen from the axial direction of the nozzle hole 64 is 0.035 mm².

A base side end surface 600 of the leading end portion 60 is formed in such a manner as to block a region, excluding the nozzle hole 64, of an end portion positioned on the leading end side of the cylindrical portion 62. The base side end surface 600 extends, for example, to be inclined to have a portion closer to the radially inward side positioned closer to the leading end side.

The area of the cylindrical portion 62 seen from the axial direction is, for example, substantially constant, and is specifically 12.56 mm² or more and 314 mm² or less. The inner diameter of the cylindrical portion 62 is, for example, substantially constant, and is specifically 4 mm or more and 20 mm or less.

The partitioning means 7 is housed in such a barrel 6, and the space S within the barrel 6 is partitioned by the partitioning means 7 into the base end side space S1 on the base end side in the axial direction, and the leading end side space S2 on the leading end side in the axial direction. In the barrel 6 of the present embodiment, as illustrated in Figs. 2 to 5, the base end side space S1 corresponding to the space on the base end side of the partitioning means 7 is in an empty state not holding a liquid drug therein, and the leading end side space S2 corresponding to the space on the leading end side of the partitioning means 7 is in an empty state not holding a drug therein. Specifically, the leading end side space S2 includes a first leading end side space S21 that is a space on the leading end side of a filter 80, and does not hold a drug therein, and a second leading end side space S22 that is a space on the base end side of the filter 80, and does not hold a drug therein.

It is noted that either one of the first leading end side space S21 and the second leading end side space S22 may hold a solid drug therein. Besides, the base end side space S1 and the leading end side space S2 may respectively hold liquid drugs therein.

The partitioning means 7 includes a cylindrical housing 8, and an inner plug portion 9 openable/closable to the inside of the housing 8. In the present embodiment, the partitioning means 7 includes one housing 8 and one inner plug portion 9. The partitioning means 7 is disposed in the leading end portion 60 of the barrel 6 (see Figs. 3 and 4). The partitioning means 7 further includes, within the barrel 6, the filter 80 attached to the housing 8. This partitioning means 7 includes one filter 80, but may include a plurality of stacked filters 80.

It is noted that the partitioning means 7 may not include the filter 80. In this case, the partitioning means 7 includes, for example, the cylindrical housing 8, and the inner plug portion 9 openable/closable to the inside of the housing 8.

The housing 8 is a member disposed to liquid-tightly contact with the inner surface of the barrel 6 (for example, the inner surface of the cylindrical portion 62). Besides, the housing 8 is housed in the barrel 6 in pressure contact with the barrel 6. Specifically, at least a part of an outer circumference 800 of the housing 8 is in close contact with the inner surface of the barrel 6.

In the present embodiment, the outer circumference 800 of the housing 8 includes a base end outer circumferential portion 801 disposed on the base end side, and a leading end outer circumferential portion 802 disposed on the leading end side, and the leading end outer circumferential portion 802 is formed to have a smaller diameter than the base end outer circumferential portion 801. The base end outer circumferential portion 801 liquid-tightly contacts with the inner circumference of the barrel 6. The leading end outer circumferential portion 802 opposes the inner circumference of the barrel 6 with a gap therebetween.

Besides, the housing 8 is formed from a flexible material. The housing 8 of the present embodiment is more flexible than the barrel 6. Specifically, the material of the housing 8 is, for example, a resin, a rubber, an elastomer, or the like softer than the material of the barrel 6. The flexibility of the barrel 6 and the housing 8 can be confirmed, for example, by measuring durometer hardness. Since the housing 8 is formed from such a material, the barrel 6 can be prevented from being cracked with the flexibility of the housing 8 against the barrel 6 ensured.

The housing 8 has a cylindrical shape. In the present embodiment, the housing 8 is disposed on the base end side of the filter 80. In other words, the housing 8 is constituted only by a base end side portion disposed on the base end side of the filter 80.

The housing 8 of the present embodiment is constituted only by the base end side portion, but may be integrally or separately constituted by a leading end side portion disposed on the leading end side of the filter 80.

The housing 8 has a close-time engaging portion 81. Specifically, the housing 8 has one close-time engaging portion 81. The close-time engaging portion 81 is a portion that engages with the inner plug portion 9 when the inner plug portion 9 is in a close state (see Fig. 3).

The close-time engaging portion 81 is provided to project radially inward from an inner circumference 82 of the housing 8, and is formed in a plural number adjacently and consecutively over the entire circumferential direction, or at intervals over the entire circumferential direction. In the present embodiment, the close-time engaging portion 81 is a rib projecting toward the cylindrical axis (radially inward) from the inner circumference 82 of the housing 8. Specifically, the close-time engaging portion 81 is a rib consecutive in the circumferential direction. Besides, the close-time engaging portion 81 is provided at the center in the axial direction of the inner circumference 82. It is noted that a base side end surface of the close-time engaging portion 81 is a flat surface extending in the radial direction.

In the present embodiment, the inner circumference 82 of the housing 8 includes a base end inner circumferential portion 821 disposed on the base end side, and a leading end inner circumferential portion 822 disposed on the leading end side, and the base end inner circumferential portion 821 is formed to have a larger diameter than the leading end inner circumferential portion 822.

The housing 8 described so far has a small-diameter inner circumference (specifically, the leading end inner circumferential portion 822) on the leading end side, a large-diameter inner circumference (specifically, the base end inner circumferential portion 821) on the base end side, and the close-time engaging portion 81 between the small diameter inner circumference and the large diameter inner circumference.

Besides, the housing 8 has a stepped surface 810 extending radially inward from the inner circumference 82, and facing the base end side. Although the base side end surface of the close-time engaging portion 81 is the stepped surface 810 in this housing 8, a surface of another portion except for the close-time engaging portion 81 may be the stepped surface 810. Besides, the stepped surface 810 may be positioned either on the leading end side or on the base end side of the close-time engaging portion 81.

In the present embodiment, the housing 8 includes an attachment portion 83 on which the filter 80 is attached, a body portion 84 in which the close-time engaging portion 81 is disposed, and an extension portion 85 extending from the body portion 84. In this housing 8, the attachment portion 83, the body portion 84, and the extension portion 85 are arranged in the stated order in a direction from the leading end side to the base end side. Besides, in this housing 8, the attachment portion 83, the body portion 84, and the extension portion 85 are all in a cylindrical shape. The thickness of the housing 8 (a distance between the inner circumference and the outer circumference of the housing 8 in the radial direction that is a direction vertical to the axial direction) is uneven. Specifically, the thickness of the attachment portion 83 is larger than the thickness of the extension portion 85.

The outer circumference of the attachment portion 83 is slightly spaced from the inner circumference of the barrel 6 (specifically, the inner circumference of the cylindrical portion 62 of the barrel 6). Besides, the attachment portion 83 is in a tapered shape having a smaller outer diameter toward the leading end side. The inner circumference of the attachment portion 83 is in a tapered shape having a larger inner diameter toward the leading end side.

The body portion 84 is a portion connecting the attachment portion 83 and the extension portion 85 to each other. The outer circumference of the body portion 84 is slightly spaced from the inner circumference of the barrel 6 (specifically, the inner circumference of the cylindrical portion 62 of the barrel 6). Besides, the body portion 84 is in a tapered shape having a smaller outer diameter toward the leading end side. The inner circumference of the body portion 84 is constituted by a projecting end surface of the close-time engaging portion 81. Besides, the inner circumference of the body portion 84 is positioned in the radially innermost position among the other inner circumferences of the housing 8.

The outer circumference of the extension portion 85 is in surface contact with the inner circumference of the barrel 6 (specifically, the inner circumference of the cylindrical portion 62 of the barrel 6). Since the outer circumference of the extension portion 85 is thus in close contact with the inner circumference of the barrel 6, a drug can be prevented from leaking to the leading end side through a gap between the outer circumference of the housing 8 and the inner circumference of the barrel 6. The inner circumference of the extension portion 85 is positioned radially outside the inner circumference of the attachment portion 83. In other words, the inner diameter of the extension portion 85 is larger than the inner diameter of the attachment portion 83.

In the housing 8 described so far, a liquid flow path C8 corresponding to a flow path of the drug solution from the base end side space S1 to the leading end side space S2 is formed radially inside the close-time engaging portion 81 (see Fig. 4). In other words, the liquid flow path C8 is formed inside the inner circumference of the body portion 84.

The inner plug portion 9 is a member disposed in such a manner as to be openable/closable to the inside of the housing 8 for communicating or non-communicating the base end side space S1 and the leading end side space S2 with each other through the inside of the housing 8. The inner plug portion 9 is a plug provided within the housing 8 in the present embodiment, but may be a cover covering the housing 8 from the leading end side.

The inner plug portion 9 further has a close-time engaged portion 90 to be engaged with the close-time engaging portion 81 when the inner plug portion 9 is in a close state (see Fig. 3). The close-time engaged portion 90 is disposed on the leading end side and the base end side of the close-time engaging portion 81. Specifically, the close-time engaged portion 90 includes a base end side close-time engaged portion 901 positioned on the base end side, and a leading end side close-time engaged portion 902 positioned on the leading end side. In other words, the inner plug portion 9 has a plurality of (for example, two) close-time engaged portions 90.

The inner plug portion 9 has regulating means 91 for regulating movement of the inner plug portion 9 toward the leading end side when the engagement between the close-time engaging portion 81 and the close-time engaged portion 90 is released for communicating the base end side space S1 and the leading end side space S2 with each other. Since the inner plug portion 9 has the regulating means 91, the inner plug portion 9 is prevented from blocking paths such as the nozzle hole 64 when the base end side space S1 and the leading end side space S2 are communicated with each other.

In the present embodiment, the regulating means 91 regulates the movement of the inner plug portion 9 toward the leading end side in a position where the inner plug portion 9 is spaced from the filter 80 toward the base end side. Thus, the inner plug portion 9 can be prevented from blocking pores of the filter 80.

Besides, the inner plug portion 9 has a cover portion 92 that has the close-time engaged portion 90 on the outer circumference, and is constituted to be capable of opening/closing the liquid flow path C8 of the housing 8. The inner plug portion 9 further has a step engaging portion 93 that is disposed on the base end side of the cover portion 92, and is provided to project radially outward beyond the cover portion 92, and a plug flow path C9 that is disposed radially inside the step engaging portion 93, and is formed in the axial direction between the cover portion 92 and the step engaging portion 93 (see Fig. 4). Thus, when the inner plug portion 9 is regulated in an open state, a drug solution held in the base end side space S1 flows through the plug flow path C9 disposed radially inside the step engaging portion 93 from the base end side to the leading end side through the liquid flow path C8 disposed radially inside the close-time engaging portion 81 (in a direction shown with an arrow in Fig. 4), and smoothly flows into the leading end side space S2 through a portion between the cover portion 92 and the close-time engaging portion 81. Besides, an outer circumference 920 of the cover portion 92 and the leading end inner circumferential portion 822 of the housing 8 are constituted not to contact each other over the entire circumference, and hence, the drug solution can uniformly flow over the entire circumference.

In the present embodiment, the inner plug portion 9 has, as the regulating means 91, the stepped surface 810, and the step engaging portion 93 projecting radially outward, and capable of engaging with the stepped surface 810 from the base end side. Since the regulating means 91 includes the stepped surface 810 and the step engaging portion 93, the step engaging portion 93 engages with the stepped surface 810 from the base end side, and hence the movement of the inner plug portion 9 can be definitely regulated.

The step engaging portion 93 is provided, for example, consecutively or intermittently over the entire circumferential direction, and is provided consecutively in the present embodiment. Owing to this constitution, when the engagement between the close-time engaging portion 81 and the close-time engaged portion 90 is released, and the inner plug portion 9 moves toward the leading end side for communicating the base end side space S1 and the leading end side space S2 completely with each other, the step engaging portion 93 provided consecutively or intermittently over the entire circumferential direction engages with the stepped surface 810 from the base end side, and thus the movement of the inner plug portion 9 toward the leading end side is regulated. Since both the step engaging portion 93 and the stepped surface 810 are formed radially outside the cover portion 92 over the entire circumference, the inner plug portion 9 is regulated in the movement in a stable posture.

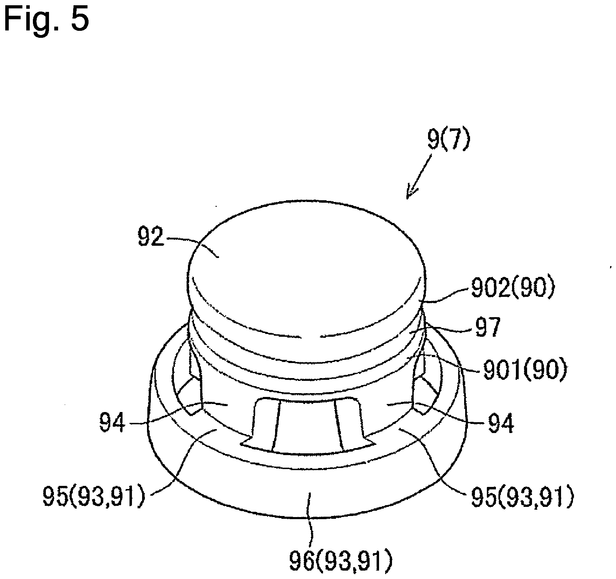

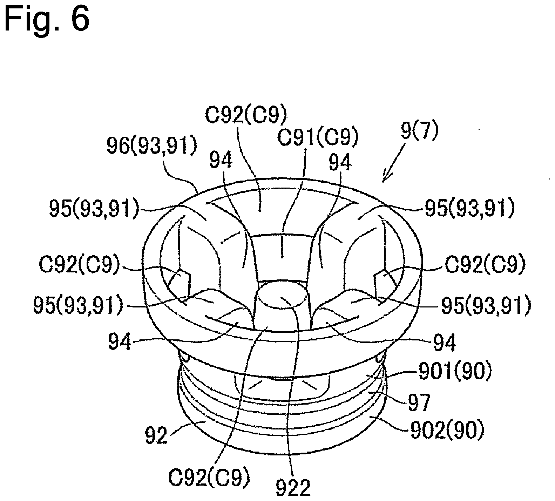

The inner plug portion 9 includes, in addition to the cover portion 92, a leg portion 94 extending from the cover portion 92. In the present embodiment, the inner plug portion 9 includes a plurality of leg portions 94 (specifically, four leg portions 94) as illustrated in Figs. 5 and 6. The inner plug portion 9 further includes a plurality of projecting portions 95 (specifically, four projecting portions 95) respectively projecting from the leg portions 94 toward the housing 8 (radially outward). The inner plug portion 9 further includes a circular portion 96 connecting the plurality of projecting portions 95 to one another.

It is noted that the inner plug portion 9 has the projecting portions 95 as the step engaging portion 93, and more specifically, also includes, as the step engaging portion 93, the circular portion 96 in addition to the projecting portions 95.

The cover portion 92 is a portion to be fit in the housing 8 (see Figs. 3 and 4). Besides, the cover portion 92 is a portion that has the close-time engaged portion 90 on the outer circumference, and is openable/closable to the inside of the housing 8. Further, the cover portion 92 is a portion partitioning the base end side space S1 and the leading end side space S2 from each other. The cover portion 92 is, for example, in substantially a disk shape. On the outer circumference 920 of the cover portion 92, the close-time engaged portion 90 is provided. An inner circumference 921 of the cover portion 92 (a portion positioned radially inside the outer circumference 920 in the cover portion 92) has a cover leg portion 922 extending toward the base end side of a portion on the disk. In the present embodiment, a base side end surface of the cover leg portion 922 is positioned on the leading end side of a base side end surface of the leg portion 94. Besides, the base side end surface of the cover leg portion 922 is positioned on the leading end side of a base side end surface of the projecting portion 95. Further, the base side end surface of the cover leg portion 922 is positioned on the leading end side of a base side end surface of the circular portion 96.

The cover leg portion 922 is provided to project from the center in the radial direction of the cover portion 92 toward the base end side. The outer diameter of the cover leg portion 922 is smaller than the outer diameter of the cover portion 92. The cover leg portion 922 is provided at the center in the radial direction of a region surrounded by the plurality of leg portions 94 (a plurality of projecting portions) arranged in the circumferential direction. Besides, the cover leg portion 922 is disposed at the center in the radial direction of a region surrounded by the circular portion 96.

The close-time engaged portion 90 is, for example, a projection projecting toward the housing 8. Specifically, the close-time engaged portion 90 is a rib projecting toward the housing 8. The close-time engaged portion 90 is a rib provided consecutively in the circumferential direction in the present embodiment, but may be a plurality of ribs provided at intervals in the entire circumferential direction.

The base end side close-time engaged portion 901 is provided in an end portion on the base end side of the cover portion 92. The leading side close-time engaged portion 902 is provided in an end portion on the leading end side of the cover portion 92.

The base end side close-time engaged portion 901 and the leading end side close-time engaged portion 902 are provided to be spaced from each other in the axial direction. A cover recess portion 97 is formed between the base end side close-time engaged portion 901 and the leading end side close-time engaged portion 902. With this cover recess portion 97, the close-time engaging portion 81 of the housing 8 engages from the radially outside direction. In this engagement state, the base end side close-time engaged portion 901 is engaged with the close-time engaging portion 81 from the base end side, and thus, the movement of the inner plug portion 9 toward the leading end side in the housing 8 is regulated. Besides, in the engagement state, the leading end side close-time engaged portion 902 is engaged with the close-time engaging portion 81 from the leading end side, and thus, the movement of the inner plug portion 9 toward the base end side in the housing 8 is regulated.

The leg portion 94 is a portion extending toward the base end side of the end portion on the base end side of the outer circumference 920 of the cover portion 92. The plurality of leg portions 94 (specifically, four leg portions 94) are disposed at equal intervals in the circumferential direction.

Radially inside the leg portions 94, the plug flow path C9 is formed. The plug flow path C9 includes an axial flow path C91 formed in the axial direction radially inside the leg portions 94, and a communication flow path C92 for communicating the axial flow path C91 with a space radially outside the leg portions 94 (see Fig. 4). The axial flow path C91 is disposed radially inside the projecting portions 95, and is formed along the axial direction between the projection portions 95 and the cover portion 92. The communication flow path C92 is formed between the plural leg portions 94 to be opened radially outside, and communicates with the axial flow path C91 radially outside the axial flow path C91.

The projecting portion 95 is a portion extending toward the housing 8 from the end portion on the base end side of the leg portion 94. The plural projecting portions 95 (specifically, four projecting portions 95) are disposed at equal intervals in the circumferential direction.

The circular portion 96 is a portion connecting the ends on the side of the housing 8 (radially outside ends) of the projection portions 95. Besides, the circular portion 96 is, for example, in a ring shape. It is noted that the circular portion 96 may be a circular shape other than the ring shape, such as a rectangular ring shape.

In the inner plug portion 9 described so far, in the state where the inner plug portion 9 is opened, the step engaging portion 93 (specifically, the projecting portions 95, and in the present embodiment, the projecting portions 95 and the circular portion 96) of the inner plug portion 9 engages with the stepped surface 810 (the base side end surface of the close-time engaging portion 81) of the housing 8, and therefore, the movement of the inner plug portion 9 further toward the leading end side (a fall of the inner plug portion 9 off from the close-time engaging portion 81) is regulated.

Besides, the piston 3 to be inserted into the barrel 6 from the base end side can come into contact with the inner plug portion 9. In other words, the inner plug portion 9 includes a contact surface 98 which the inserted piston 3 can come into contact with.

The contact surface 98 is an end surface on the base end side of the inner plug portion 9. Specifically, the contact surface 98 is constituted by an end surface on the base end side of the cover portion 92. More specifically, the contact surface 98 is constituted by the base side end surface of the cover leg portion 922, and the base side end surfaces of the leg portions 94.

The inner plug portion 9 is formed from a flexible material. The inner plug portion 9 of the present embodiment is more flexible than the barrel 6. Specifically, the material of the inner plug portion 9 is, for example, a resin, a rubber, an elastomer, or the like softer than the material of the barrel 6. The flexibility of the inner plug portion 9 can be confirmed by, for example, measuring the durometer hardness. The material of the inner plug portion 9 is, for example, the same as the material of the housing 8, or may be different therefrom.

The filter 80 is a filter for filtering a drug. Besides, the filter 80 is disposed in a leading end side portion of the housing 8 (on a leading end surface 830 of the attachment portion 83 of the housing 8 in the present embodiment), and is disposed, within the barrel 6, on the base end side of the nozzle hole 64.

In the present embodiment, the filter 80 is attached to the leading end side portion of the housing 8, and specifically is adhered to the leading end surface 830. This adhesion is ultrasonic welding, and alternatively, an adhesion method including another welding method such as heat welding may be employed. It is noted that the filter 80 may be adhered to the leading end surface 830.

In the syringe 2 described so far, the partitioning means 7 is constituted as follows: When a pressing force is applied from the base end side by the piston 3 inserted into the barrel 6 from the base end side, one of the close-time engaging portion 81 and the close-time engaged portion 90 moves beyond the other to release the engagement between the close-time engaging portion 81 and the close-time engaged portion 90. Thus, the inner plug portion 9 is opened to the inside of the housing 8 to communicate the base end side space S1 and the leading end side space S2 with each other. In other words, the partitioning means 7 is constituted so that the state of the inner plug portion 9 is changed from a close state (see Fig. 3) to an open state (see Fig. 4) by this pressing force. Specifically, the partitioning means 7 is constituted as follows: When the pressing force is applied by the piston 3 from the base end side, at least one of the close-time engaging portion 81 and the close-time engaged portion 90 is elastically deformed, and the close-time engaged portion 90 moves beyond the close-time engaging portion 81, and hence the inner plug portion 9 moves toward the leading end side. As a result, the engagement between the close-time engaging portion 81 and the close-time engaged portion 90 is released, the inner plug portion 9 is opened to the inside of the housing 8, and thus, the base end side space S1 and the leading end side space S2 communicate with each other.

Besides, in the syringe 2, the barrel 6 and the attachment portion 83 of the housing 8 together clamp the filter 80. Specifically, the base side end surface 600 of the leading end portion 60 of the barrel 6 and the leading end surface 830 of the attachment portion 83 together clamp the filter 80 in the axial direction. Therefore, even when a pressure is applied to the filter 80 in administering a drug solution, the filter 80 is difficult to come off from the attachment portion 83.

Besides, in the syringe 2, the base end side space S1, the leading end side space S2, and the nozzle hole 64 are arranged in the stated order in a direction from the base end side to the leading end side. In the syringe 2 of the present embodiment, the central axis of the base end side space S1 and the central axis of the leading end side space S2 both accord with the central axis of the nozzle hole 64. Therefore, in administering a drug, the flow of the drug from the base end side space S1 to the nozzle hole 64 is favorable.

It is noted that the syringe 2 of the present embodiment may be sterilized with radiation of y rays or the like. This sterilization may be performed with gas such as ethylene oxide gas, or with an autoclave.

The piston 3 is a member operated in ejecting the drug held in the barrel 6. The piston 3 of the present embodiment has a shaft rod portion 30, a gasket 31 attached to one end in the longitudinal direction of the rod portion 30, and in close contact with the entire inner circumference of the cylindrical portion 62 of the barrel 6, and an operation portion 32 attached to the other end in the longitudinal direction of the rod portion 30.

In the piston 3 of the present embodiment, a leading end surface 33 is constituted by a leading end surface of the gasket 31. Specifically, when the administration of the drug is completed, the leading end surface 33 comes into contact with the end surface on the base end side of the inner plug portion 9.

It is noted that the piston 3 is operated by a doctor, a nurse, or the like, and may be operated by a patient. In this case, the pre-filled syringe 1 may be attached to an autoinjector.

The needle 4 is a member for administering the drug held in the barrel 6 to a patient. The leading end of the needle 4 is covered with the cap 5.

According to the syringe 2 described so far, when one of the close-time engaging portion 81 and the close-time engaged portion 90 moves, from a state where these members are engaged with each other, beyond the other due to a pressing force applied by the piston 3 (in the present embodiment, from the state where the close-time engaging portion 81 and the base end side close-time engaged portion 901 are engaged with each other, the base end side close-time engaged portion 901 moves beyond the close-time engaging portion 81 due to the pressing force), this engagement is released all at once. Therefore, the partitioning means 7 partitioning the barrel 6 is definitely opened to communicate the base end side space S1 and the leading end side space S2 with each other, and thus, the drug solution can be smoothly ejected.

In the syringe 2 of the present embodiment, one of the close-time engaging portion 81 and the close-time engaged portion 90 disposed on the leading end side and the base end side is positioned to be sandwiched between the other (in the present embodiment, the close-time engaging portion 81 is positioned between the base end side close-time engaged portion 901 and the leading end side close-time engaged portion 902), and therefore, the movement, in the housing 8, toward the leading end side and the base end side in the axial direction of the inner plug portion 9 in a close state can be regulated, and thus, unintended movement otherwise caused during transportation or the like can be regulated.

In the syringe 2 of the present embodiment, the filter 80 is disposed in the leading end side portion of the housing 8, and the barrel 6 is partitioned by the partitioning means 7 until the drug is ejected, and therefore, the filter 80 can be prevented from contacting the drug before ejection if the drug is held in the base end side space S1.

Besides, in the syringe 2 of the present embodiment, since the partitioning means 7 is disposed in the leading end portion of the barrel 6, the filter 80 can be prevented from contacting the drug held in the base end side space S1 until the ejection of the drug, and in addition, the filter 80 can filter the drug after the partitioning means 7 partitioning the barrel 6 is opened.

The structure of the partitioning means 7 may be different from the structure described above. For example, the inner plug portion 9 may have another structure different from that described above as long as it includes the close-time engaged portion 90.

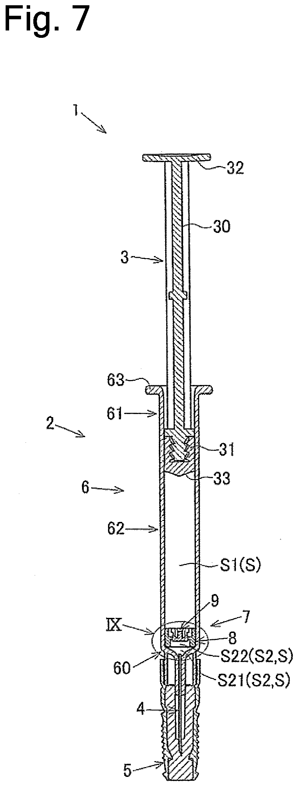

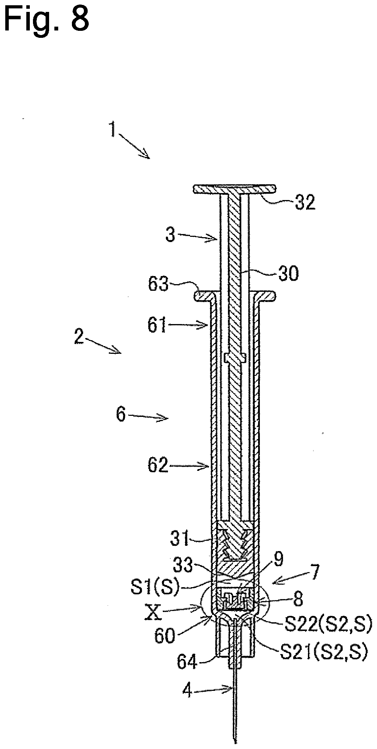

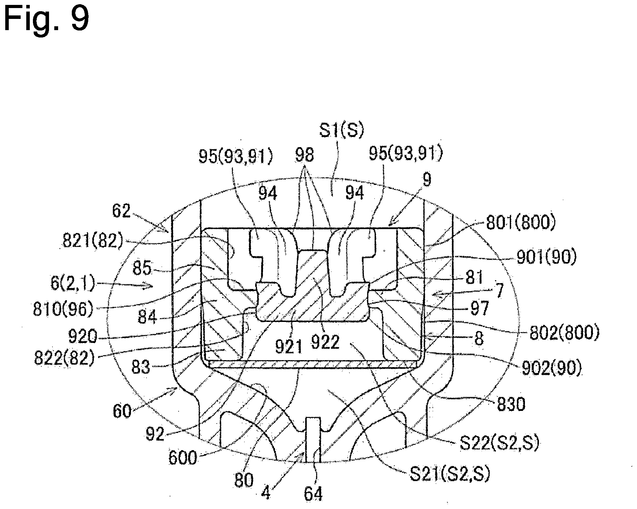

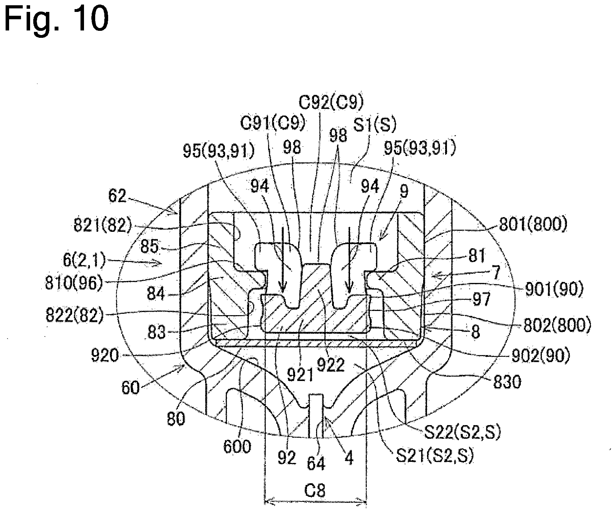

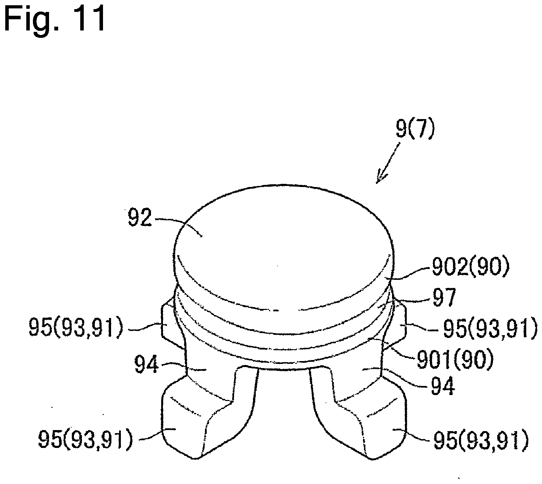

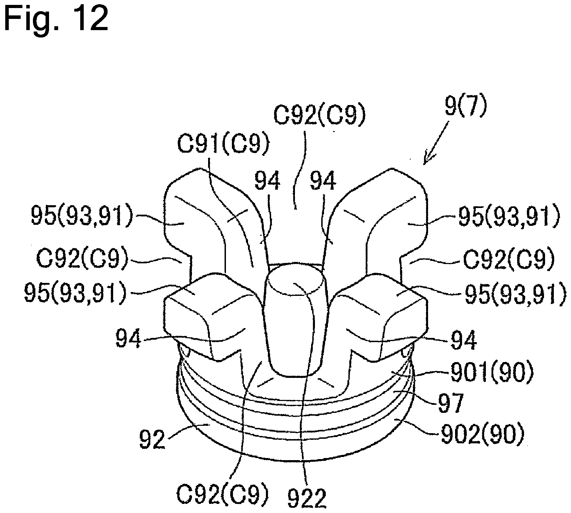

Specifically, the inner plug portion 9 may not be provided with the circular portion 96 as illustrated in Figs. 7 to 12. In other words, it can be deemed that the inner plug portion 9 includes the close-time engaged portion 90, the cover portion 92 covering the base end side space S1, the leg portions 94 (specifically, four leg portions 94) extending from the cover portion 92, and a plurality of projecting portions 95 (specifically, four projecting portions 95) respectively projecting from the leg portions 94 toward the housing 8 (radially outward).

The inner plug portion 9 may include a connecting portion for connecting the cover portion 92 (specifically, the cover leg portion 922 of the cover portion 92) and the leg portions 94. In this case, the connecting portion can regulate deflection in the radial direction of the leg portions 94 otherwise caused when the inner plug portion 9 is pressed by the piston 3 with the step engaging portion 93 (the projection portions 95) engaged with the stepped surface 810.

Besides, the inner plug portion 9 may include, instead of the circular portion 96, a plug extension portion extending from the end portions of the projection portions 95 on the side of the housing 8 (radially outside end portions) in the circumferential direction. The plug extension portion may, for example, extend from the respective projecting portions 95, and may be provided in a plural number. The plurality of plug extension portions may be disposed at intervals in the circumferential direction.

Besides, the inner plug portion 9 may not include the leg portions 94 and the projecting portions 95, and may be constituted by, for example, the close-time engaged portion 90 and the cover portion 92.

In the above-described embodiment, when the pressing force is applied by the piston 3 from the base end side, at least one of the close-time engaging portion 81 and the close-time engaged portion 90 is elastically deformed, and the close-time engaged portion 90 moves beyond the close-time engaging portion 81, and hence the inner plug portion 9 moves toward the leading end side, thereby releasing the engagement between the close-time engaging portion 81 and the close-time engaged portion 90. Instead, when this pressing force is applied, at least one of the close-time engaging portion 81 and the close-time engaged portion 90 may be elastically deformed, and the close-time engaging portion 81 may move beyond the close-time engaged portion 90, and the housing 8 may move toward the leading end side, thereby releasing the engagement between the close-time engaging portion 81 and the close-time engaged portion 90.

Even in such a structure, when one of the close-time engaging portion 81 and the close-time engaged portion 90 moves, from a state where these members are engaged with each other, beyond the other due to a pressing force applied by the piston 3, this engagement is released all at once. Therefore, the partitioning means 7 partitioning the barrel 6 is definitely opened to communicate the base end side space S1 and the leading end side space S2 with each other, and thus, the drug solution can be smoothly ejected.

Although the partitioning means 7 has one close-time engaging portion 81 and a plurality of (specifically, two) close-time engaged portions 90 in the above-described embodiment, the partitioning means 7 may have a plurality of close-time engaging portions 81 and one close-time engaged portion 90. Specifically, the close-time engaging portions 81 may be disposed on the leading end side and the base end side of the close-time engaged portion 90. In this case, a recess may be formed between the close-time engaging portions 81 disposed on the leading end side and the base end side in the housing 8. In this recess, the close-time engaged portion 90 of the inner plug portion 9 is fit in the radially inside direction. In this fitting state, the close-time engaging portion 81 disposed on the base end side engages with the close-time engaged portion 90 from the base end side, and thus, the movement of the inner plug portion 9 toward the base end side in the housing 8 is regulated. Besides, in the fitting state, the close-time engaging portion 81 disposed on the leading end side engages with the close-time engaged portion 90 from the leading end side, and thus, the movement of the inner plug portion 9 toward the leading end side in the housing 8 is regulated. It is noted that the partitioning means 7 may have one close-time engaging portion 81 and one close-time engaged portion 90.

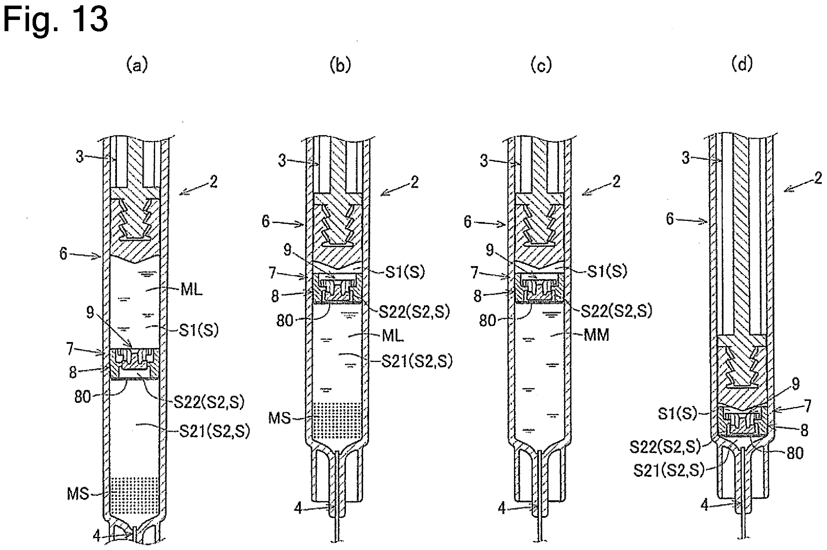

Although the partitioning means 7 is disposed in the leading end portion 60 of the barrel 6 in the above-described embodiment, the partitioning means 7 may be disposed in a middle position in the axial direction of the barrel 6. For example, in a state where the partitioning means 7 is disposed in a middle position in the axial direction of the barrel 6 for partitioning the base end side space S1 and the leading end side space S2 from each other, a liquid drug ML may be held in the base end side space S1, and a solid drug MS may be held in the leading end side space S2 (specifically, the first leading end side space S21) (see Fig. 13(a)). From this state, when a pressing force is applied from the base end side by the piston 3, the inner plug portion 9 is opened to the inside of the housing 8 for communicating the base end side space S1 and the leading end side space S2 with each other as illustrated in Fig. 13(b), the liquid drug ML flows from the base end side space S1 into the leading end side space S2, and thus, a mixed drug MM corresponding to a mixture of the solid drug MS and the liquid drug ML is obtained as illustrated in Fig. 13(c). When the pressing force is further applied from the base end side by the piston 3, the mixed drug MM can be administered as illustrated in Fig. 13(d).

In this manner, in the present embodiment, the partitioning means 7 is disposed in a middle position in the axial direction of the barrel 6 for partitioning the space within the barrel 6 into the base end side space S1 and the leading end side space S2, and is opened when the pressure applied by the piston 3 from the side of the base end side space S1 is a prescribed pressure, and moves up to the leading end portion 60 of the barrel 6 in the open state in accordance with the pressing operation of the piston 3 toward the leading end side of the barrel 6.

Besides, since the partitioning means 7 for partitioning the base end side space S1 and the leading end side space S2 from each other includes the filter 80, when the partitioning means 7 is opened, and the liquid drug ML flows from the base end side space S1 into the leading end side space S2, unwanted solid matters are captured by the filter 80.

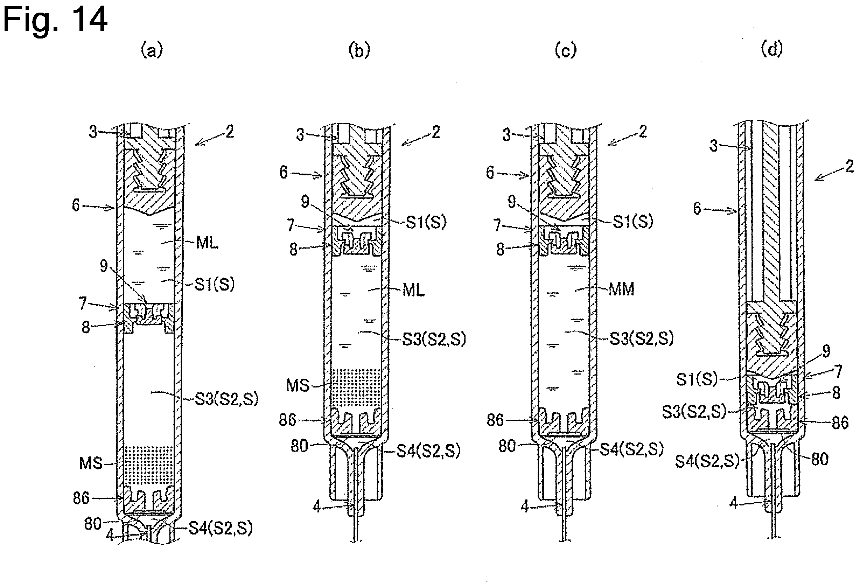

Alternatively, the partitioning means 7 may have a structure not including the filter 80. For example, it can be deemed, as illustrated in Fig. 14(a), that the partitioning means 7 includes only the housing 8 and the inner plug portion 9, and is disposed in a middle position of the barrel 6, and a filter member 86 including the filter 80 is disposed on the leading end side of and spaced from the partitioning means 7.

Also in this case, it can be deemed that the space S within the barrel 6 is partitioned by the partitioning means 7 into the base end side space S1, that is, the space on the base end side of the housing 8 and the inner plug portion 9, and the leading end side space S2, that is, the space on the leading end side of the housing 8 and the inner plug portion 9. In the present embodiment, the leading end side space S2 includes a third leading end side space S3, that is, a space between the housing 8 and the inner plug portion 9, and the filter member 86, and a fourth leading end side space S4, that is, a space on the leading end side of the filter member 86. It can be deemed, in this structure, that the liquid drug ML is held in the base end side space S1, the liquid drug ML or the solid drug MS is held in the third leading end side space S3, and the solid drug MS is held in the fourth leading end side space S4, or this space is empty. In the present embodiment, the liquid drug ML is held in the base end side space S1, the solid drug MS is held in the third leading end side space S3, and the fourth leading end side space S4 is empty.

It is noted that in this partitioning means 7, the housing 8 does not include the filter 80 but the filter member includes the filter 80. Besides, the housing 8 and the filter member 86 are both in a substantially cylindrical shape. Although an inner circumference of the filter member 86 is formed to have a smaller diameter than the inner circumference of the housing 8 in the present embodiment, the inner circumference of the filter member 86 may be formed to have substantially the same diameter as the inner circumference of the housing 8.

In this structure, in a state where the partitioning means 7 partitions the base end side space S1 and the third leading end side space S3 from each other, the liquid drug ML held in the base end side space S1 and the solid drug MS held in the third leading end side space S3 are partitioned from each other. From this state, when the pressing force is applied from the base end side by the piston 3, and the inner plug portion 9 is opened to the inside of the housing 8 for communicating the base end side space S1 and the third leading end side space S3 with each other as illustrated in Fig. 14(b), the liquid drug ML flows from the base end side space S1 into the third leading end side space S3, and the mixed drug MM corresponding to a mixture of the solid drug MS and the liquid drug ML is obtained as illustrated in Fig. 14(c). When the pressing force is further applied from the base end side by the piston 3, the mixed drug MM can be administered as illustrated in Fig. 14(d).

In this manner, the partitioning means 7 partitions, by the housing 8 and the inner plug portion 9 disposed in a middle position in the axial direction of the barrel 6, the space S within the barrel 6 into the base end side space S1 and the leading end side space S2, and is opened when the pressure applied by the piston 3 from the side of the base end side space S1 becomes a prescribed pressure, and move up to the leading end portion 60 of the barrel 6 in the open state in accordance with the pressing operation of the piston 3 toward the leading end side of the barrel 6. Further, this structure includes the filter member 86 disposed on the leading end side of the housing 8, and having a flow hole formed for communication from the base end side to the leading end side, and the filter 80 attached to the filter member 86 for filtering a liquid flowing through the flow hole. Therefore, when the inner plug portion 9 is opened to the housing 8, and the liquid drug ML flows from the base end side space S1 into the leading end side space S2 to be mixed with the solid drug MS, and the mixture is ejected from the leading end portion 60 of the barrel 6, unwanted solid matters are captured by the filter 80. Besides, the filter member 86 is disposed so as not to move toward the leading end side in the leading end portion 60 of the barrel 6, and hence, the pressure resistance of the piston 3 caused when the housing 8 moves toward the leading end side can be equalized.

Besides, it can be deemed that, for example, the partitioning means 7 includes two sets of the housing 8 and the inner plug portion 9, with one of the sets disposed on the base end side and the other set disposed on the leading end side to be spaced from each other. In this case, it can be deemed that the liquid drug ML is held in a space on the base end side of the housing 8 and the inner plug portion 9 positioned on the base end side, the liquid drug ML or the solid drug MS is held in a space between the housing 8 and the inner plug portion 9 positioned on the base end side and the housing 8 and the inner plug portion 9 positioned on the leading end side, and the solid drug MS is held in a space on the leading end side of the housing 8 and the inner plug portion 9 positioned on the leading end side, or this space is empty.

It is noted that when the close-time engaging portion 81 is provided in a plural number at intervals in the entire circumferential direction, the plural close-time engaging portions 81 may be disposed in opposite positions with the axis disposed therebetween. Alternatively, the plural close-time engaging portions 81 may be disposed at equal intervals in the circumferential direction. When the close-time engaged portion 90 is also similarly provided in a plural number at intervals in the entire circumferential direction, the plural close-time engaged portions 90 may be disposed in opposite positions with the axis disposed therebetween, or may be disposed at equal intervals in the circumferential direction. In such a structure, the close-time engaging portions 81 and the close-time engaged portions 90 stably engage with each other, and therefore, the inner plug portion 9 is difficult to come off from the housing 8 during transportation or the like of the syringe 2.

In one aspect of the present invention, a holding member 10 may be disposed in the barrel instead of the partitioning means. Herein, the holding member 10 refers to partitioning means not having an inner plug but including only a housing. The holding member 10 is a member in which the filter 80 is attached. Besides, the holding member 10 is housed in the barrel 6 in a state where it is in pressure contact with the barrel 6. Specifically, at least a part of an outer circumference of the holding member 10 is in close contact with the inner circumference of the barrel 6.

Besides, the holding member 10 is formed from a flexible material. The holding member 10 of the present embodiment is more flexible than the barrel 6. Specifically, the material of the holding member 10 is, for example, a resin, a rubber, an elastomer, or the like softer than the material of the barrel 6. The flexibility of the barrel 6 and the holding member 10 can be confirmed, for example, by measuring durometer hardness. Since the holding member 10 is formed from such a material, the barrel 6 can be prevented from being cracked with the flexibility of the holding member 10 against the barrel 6 ensured.

The holding member 10 includes a base end side portion 110 disposed on the base end side of the filter 80. The holding member 10 of the present embodiment includes only the base end side portion 110. The base end side portion 110 of the holding member 10 is provided with a flow path 111 for causing a drug to flow from the base end side to the leading end side.

The base end side portion 110 of the holding member 10 includes, as a base side end surface 200, a contact surface 200 which the piston 3 to be inserted into the barrel 6 from the base end side can come into contact with. The base end side portion 110 is in a cylindrical shape having a leading end side covered by a disk having a through hole formed at the center.

The contact surface 200 of the base end side portion 110 has a shape corresponding to the leading end surface 33 of the piston 3. Besides, the contact surface 200 is constituted in such a manner that the leading end surface 33 of the piston 3 is in surface contact over the entire circumferential direction of the barrel 6.

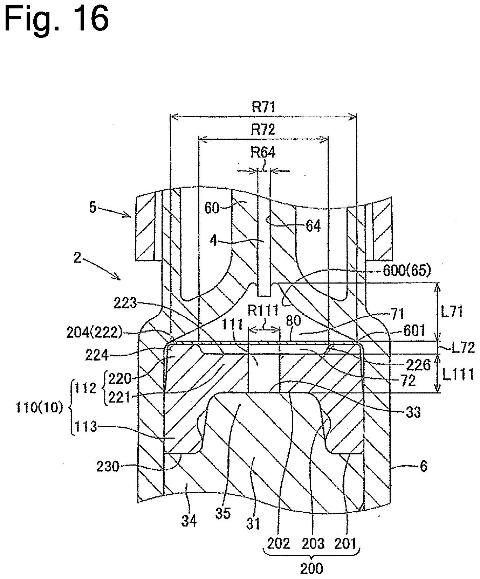

The contact surface 200 of the present embodiment includes, for example, as illustrated in Fig. 16, a base end side contact surface 201 positioned on the base end side, a leading end side contact surface 202 positioned on the leading end side, and a connection contact surface 203 connecting the base end side contact surface 201 and the leading end side contact surface 202 to each other.

The base end side contact surface 201 is, for example, a surface substantially vertical to the axial direction. The leading end side contact surface 202 is, for example, a surface substantially vertical to the axial direction.

The connection contact surface 203 is a surface extending along the axial direction. For example, a center portion in the axial direction of the connection contact surface 203 is recessed in the radially outward direction.

The base end side portion 110 of the present embodiment includes an attachment portion 112 to which the filter 80 is attached, and a leg portion 113 extending from the attachment portion 112. In this base end side portion 110, the attachment portion 112 is provided on the leading end side, and the leg portion 113 is disposed on the base end side.

The flow path 111 is provided in the attachment portion 112 of the base end side portion 80. The area of the flow path 111 of the present embodiment seen from the axial direction (the cross-sectional area of the flow path 111 in a direction perpendicular to the axis) is constant. Besides, the area of the flow path 111 seen from the axial direction is 0.64 mm² or more and 1.33 mm² or less. The inner diameter of the flow path 111 of the present embodiment is constant. Besides, the inner diameter of the flow path 111 is, for example, 0.3 mm or more and 0.9 mm or less. It is noted that the flow path 111 is a through hole, and specifically, is in a cylindrical shape, and may be in a rectangular tube shape.

The attachment portion 112 has, in the outer circumferential portion thereof, a shape projecting toward the leading end side. The outer circumference of the attachment portion 112 of the present embodiment is slightly spaced from the inner circumference of the barrel 6 (specifically, the inner circumference of the cylindrical portion 62 of the barrel 6). The attachment portion 112 is in a tapered shape having a smaller outer diameter toward the leading end side. Besides, the attachment portion 112 has an attachment outer circumferential portion 220 constituting the outer circumference, and an attachment inner circumferential portion 221 positioned radially inside the attachment outer circumferential portion 220, and defining the flow path 111.

Besides, in the attachment portion 112, an outer circumferential end surface 222 corresponding to the leading end surface of the attachment outer circumferential portion 220 is positioned on the leading end side of an inner circumferential end surface 223 corresponding to the leading end surface of the attachment inner circumferential portion 221. In other words, the leading end surface of the attachment portion 112 is recessed in a center portion thereof in the circumferential direction. The attachment outer circumferential portion 220 has a shoulder portion 224 as the outer circumferential portion on the leading end side.

The leg portion 113 has, in the outer circumferential portion thereof, a shape projecting toward the base end side. A base end surface 230 of the leg portion 113 constitutes the contact surface 200 of the base end side portion 110. The outer circumference of the leg portion 113 is in surface contact with the inner circumference of the barrel 6 (specifically, the inner circumference of the cylindrical portion 62 of the barrel 6). Since the outer circumference of the leg portion 113 is in close contact with the inner circumference of the barrel 6 in this manner, the drug definitely passes through the flow path 111 of the holding member 10 to be filtered by the filter 80.

The filter 80 of the present embodiment is attached to a leading end surface 804 of the base end side portion 110 of the holding member 10. Specifically, the filter 80 is adhered to a leading end surface 204. More specifically, the filter 80 is adhered to the outer circumferential end surface 222 of the attachment outer circumferential portion 220. This adhesion is ultrasonic welding, and alternatively, an adhesion method including another welding method such as heat welding may be employed. Besides, the filter 80 may be adhered to the leading end surface 204.

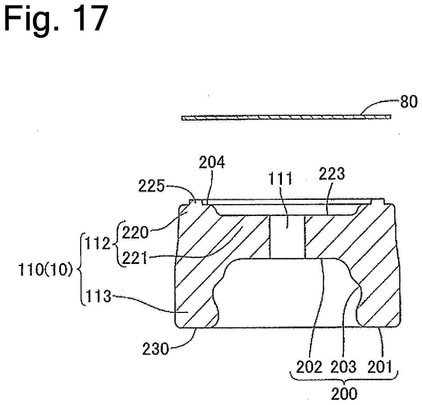

It is noted that a projection 225 projecting toward the leading end side is provided in the base end side portion 110 (for example, the attachment outer circumferential portion 220) in a state before the adhesion of the filter 80 as illustrated in Fig. 17. Since the projection 225 melts and spreads during the adhesion of the filter 80, the leading end surface 204 of the base end side portion 110 is a substantially flat surface after the adhesion of the filter 80.

A leading end side subspace 71 is defined by an inner circumference 65 of the barrel 6 (specifically, the base side end surface 600 of the leading end portion 60) and the filter 80 (specifically, the leading end surface of the filter 80). Besides, the leading end side subspace 71 is continuous to the nozzle hole 64. Herein, the leading end side subspace 71 corresponds to the first leading end side space S21.

Besides, the leading end side subspace 71 is in a shape having an area seen from a cylindrical direction (a cross-sectional area in the direction perpendicular to the axis) reduced in a direction from the base end side to the leading end side, namely, in a tapered shape having a smaller area seen from the cylindrical direction (cross-sectional area in the direction perpendicular to the axis) toward the leading end side. The leading end side subspace 71 of the present embodiment is in a substantially cylindrical shape. This leading end side subspace 71 is, for example, in a shape having an inner diameter reduced in the direction from the base end side to the leading end side, namely, a tapered shape having a smaller inner diameter toward the leading end side. Besides, the leading end side subspace 71 is in a shape having a distance between the filter 80 and the base side end surface 600 of the leading end portion 60 of the barrel 6 reduced toward an outer circumferential portion thereof. Thus, in administration of the drug, the drug solution is pushed out to the leading end side subspace 71 in a state where it easily moves from an outer circumferential side to an axial side in the circumferential direction, and hence, ejection resistance can be suppressed. It is noted that the leading end side subspace 71 may be in a rectangular tube shape.

A base end side portion of the leading end side subspace 71, namely, a portion having the largest area (for example, inner diameter) in the leading end side subspace 71, aligns with the shoulder portion 224 in the axial direction of the barrel 6, and has an inner diameter substantially the same as the outer diameter of the shoulder portion of the holding member 10.

A base end side subspace 72 is defined by the filter 80 (specifically, the base end side surface of the filter 80) and the leading end surface 204 of the base end side portion 110 (specifically, the inner circumferential end surface 223 of the attachment inner circumferential portion 221, and the attachment inner circumference 226 corresponding to the inner circumference of the attachment outer circumferential portion 220). Besides, the base end side subspace 72 is formed by denting the inner circumferential end surface 223 of the attachment inner circumferential portion 221 toward the base end side beyond the outer circumferential end surface 222 of the attachment outer circumferential portion 220, namely, by denting the leading end surface of the attachment portion 112 toward the base end side. Herein, the base end side subspace 72 corresponds to the second leading end side space S22. Besides, a space formed by the leading end side subspace 71 and the base end side subspace 72 together corresponds to the leading end side space S2.

Besides, the base end side subspace 72 is continuous to the flow path 111. Further, the base end side subspace 72 is, for example, in a shape having an area seen from the axial direction increased in the direction from the base end side to the leading end side, namely, in a tapered shape having a larger area seen from the axial direction toward the leading end side. The base end side subspace 72 of the present embodiment is in a substantially cylindrical shape. This base end side subspace 72 is, for example, in a shape having an inner diameter increased in the direction from the base end side to the leading end side, namely, in a tapered shape having a larger inner diameter toward the leading end side. An inclination angle to the axial direction of the attachment inner circumference 226 of the attachment outer circumferential portion 220 defining the base end side subspace 72 is smaller than an inclination angle to the axial direction of the base side end surface 600 of the leading end portion 60 defining the leading end side subspace 71. It is noted that the base end side subspace 72 may be in a rectangular tube shape.

In the syringe 2 described so far, the barrel 6 and the base end side portion 110 of the holding member 10 together clamp the filter 80. Specifically, the base side end surface 600 of the leading end portion 60 of the barrel 6 (specifically, a base end side edge 601 of the base side end surface 600) and the shoulder portion 224 of the base end side portion 110 together clamp the filter 80 in the axial direction. Thus, even when a pressure is applied to the filter 80 in the administration of the drug solution, the filter 80 is difficult to come off from the base end side portion 110.

The flow path 111, the base end side subspace 72, the leading end side subspace 71, and the nozzle hole 64 are arranged in the stated order in the direction from the base end side to the leading end side. In the syringe 2 of the present embodiment, the central axis of the flow path 111, the central axis of the base end side subspace 72, and the central axis of the leading end side subspace 71 all accord with the central axis of the nozzle hole 64. Therefore, in administering the drug, the flow of the drug from the flow path 111 to the nozzle hole 64 is favorable.

A dimension L111 in the axial direction of the flow path 111 is larger than a dimension L72 in the axial direction of the base end side subspace 72. Besides, a dimension L71 in the axial direction of the leading end side subspace 71 (specifically, a dimension on the central axis of the leading end side subspace 71) is larger than the dimension L72 in the axial direction of the base end side subspace 72.

When seen from the axial direction, the area of the nozzle hole 64 is smaller than the area of the base end side subspace 72, smaller than the area of the leading end side subspace 71, and smaller than the area of the flow path 111. This means that the area of the nozzle hole 64 is smaller than those of any portions in the axial direction of the base end side subspace 72, smaller than those of any portions in the axial direction of the leading end side subspace 71, and smaller than those of any portions in the axial direction of the flow path 111. Specifically, the area of the nozzle hole 64 is smaller than the area of the base end side subspace 72, smaller than the maximum area of the leading end side subspace 71 (the area at a base end side edge of the leading end side subspace 71), and smaller than the area of the flow path 111.

Besides, when seen from the axial direction, the area of the flow path 111 is smaller than the area of the base end side subspace 72. This means that the area of the flow path 111 is smaller than the areas of any portions in the axial direction of the base end side subspace 72.

Specifically, an inner diameter R64 of the nozzle hole 64 is smaller than an inner diameter R72 of the base end side subspace 72, smaller than the maximum inner diameter R71 of the leading end side subspace 71 (inner diameter at the base end side edge of the leading end side subspace 71), and smaller than an inner diameter R111 of the flow path 111. Besides, the inner diameter R81 of the flow path 111 is smaller than the inner diameter R72 of the base end side subspace 72.

Regarding this dimensional relationship, an experiment was performed using a combination of a barrel 6 having an inner diameter R64 of a nozzle hole 64 of 0.27 mm or 0.21 mm, and a holding member 10 having an inner diameter R111 of a flow path 111 of 0.3 mm, 0.6 mm, or 0.9 mm, and an inner diameter R72 of a base end side subspace 72 of 0.6 mm, 2.1 mm, or 3.6 mm for examining influence on ejection resistance (specifically, sliding load).

Results of this experiment was as follows. When the inner diameter R72 of the base end side subspace 72 was 0.6 mm, the sliding load was confirmed to increase. When the inner diameter R72 of the base end side subspace 72 was 2.1 mm or 3.6 mm, the sliding load was not largely changed. Besides, when the inner diameter R111 of the flow path 111 was changed within the above-described range, the sliding load was not largely changed.

For example, the inner diameter R111 of the flow path 111 is preferably more than 1-fold and 5-fold or less of the inner diameter R64 of the nozzle hole 64, and is desirably 1.1-fold or more and 4.3-fold or less of the inner diameter R64 of the nozzle hole 64. When the inner diameter R64 of the nozzle hole 64 is 0.21 mm or 0.27 mm, the inner diameter R111 of the flow path 111 can be selected from any one of 0.3 mm, 0.6 mm, and 0.9 mm. Besides, for example, the inner diameter R72 (the filtration area of the filter 80) of the base end side subspace 72 is preferably 5-fold or more and 20-fold or less of the inner diameter R64 of the nozzle hole 64, and is desirably 7-fold or more and 18-fold or less of the inner diameter R64 of the nozzle hole 64. When the inner diameter R64 of the nozzle hole 64 is 0.21 mm or 0.27 mm, the inner diameter R72 of the base end side subspace 72 can be selected from 2.1 mm and 3.6 mm. When the inner diameters R72 and R71 of the base end side subspace 72 and the leading end side subspace 71 respectively fall in these ranges, the ejection resistance can be suppressed with the filtration area ensured in the administration of the drug.

It is noted that the syringe 2 of the present embodiment may be sterilized with radiation of y rays or the like. This sterilization may be performed with gas such as ethylene oxide gas, or with an autoclave.

The piston 3 is a member operated in ejecting the drug held in the barrel 6. The piston 3 of the present embodiment has the shaft rod portion 30, the gasket 31 attached to one end in the longitudinal direction of the rod portion 30, and in close contact with the entire inner circumference of the cylindrical portion 62 of the barrel 6, and the operation portion 32 attached to the other end in the longitudinal direction of the rod portion 30.

In the piston 3 of the present embodiment, a leading end surface 33 is constituted by a leading end surface of the gasket 31. Specifically, in the administration of the drug, the leading end surface 33 comes into contact with at least the base end side contact surface 201 of the base end side portion 110 of the holding member 10, and more specifically, comes into contact also with the connection contact surface 203 in addition to the base end side contact surface 201.

Besides, the piston 3 has a close contact portion 34 to be brought into close contact with the inner circumference of the barrel 6, and a projection 35 provided to project from a leading end surface of the close contact portion 34. The close end surface of the close contact portion 34 comes into contact with the base end side contact surface 201, and the leading end surface of the projection 35 comes into contact with the leading end side contact surface 802. Besides, the outer circumference of the projection 35 comes into close contact with the connection contact surface 203.

The needle 4 is a member for administering the drug held in the barrel 6 to a patient. The leading end of the needle 4 is covered with the cap 5.

According to the syringe 2 described so far, even when particles are present in the drug held in the barrel 6, the particles can be captured by the filter 80 at the time of the administration of the drug. Since the drug passes through the flow path 111, the base end side subspace 72, and the leading end side subspace 71 having a larger area (for example, inner diameter) than the nozzle hole 64 to be ejected from the nozzle hole 64, the resistance in passing through the flow path 111, the base end side subspace 72, and the leading end side subspace 71 can be suppressed, resulting in suppressing the ejection resistance of the drug.

In the syringe 2 of the present embodiment, the area (for example, the inner diameter) of the flow path 111 is small and the area (for example, the inner diameter) of the base end side subspace 72 is large, and therefore, with the area of the filter 80 contacting the drug (the filtration area of the filter 80) ensured, the amount of the drug remaining in the flow path 111 can be reduced, and thus, a residual liquid amount can be suppressed with the ejection resistance suppressed.

Besides, in the syringe 2 of the present embodiment, at the time of the administration of the drug, the leading end surface 33 of the piston 3 moves until it comes into surface contact with the contact surface 200 of the holding member 10 in the entire circumferential direction. Thus, a residual liquid between the piston 3 and the holding member 10 can be suppressed, and hence the residual liquid amount of the drug in the barrel 6 can be suppressed.

The syringe of the present invention is not limited to those of the above-described embodiments, and can be, needless to say, variously changed or modified without departing from the scope of the present invention. For example, a structure of a given embodiment can be added to a structure of another embodiment, or a part of a structure of a given embodiment can be replaced with a structure of another embodiment. Besides, a part of a structure of a given embodiment can be eliminated.

The structure of the holding member 10 may be different from the structure described above. For example, the contact surface 200 of the base end side portion 80 may be in a different shape as long as it accords with the contact surface 33 of the piston 3.

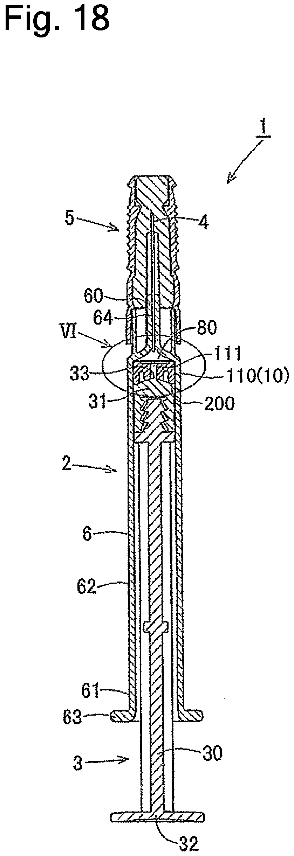

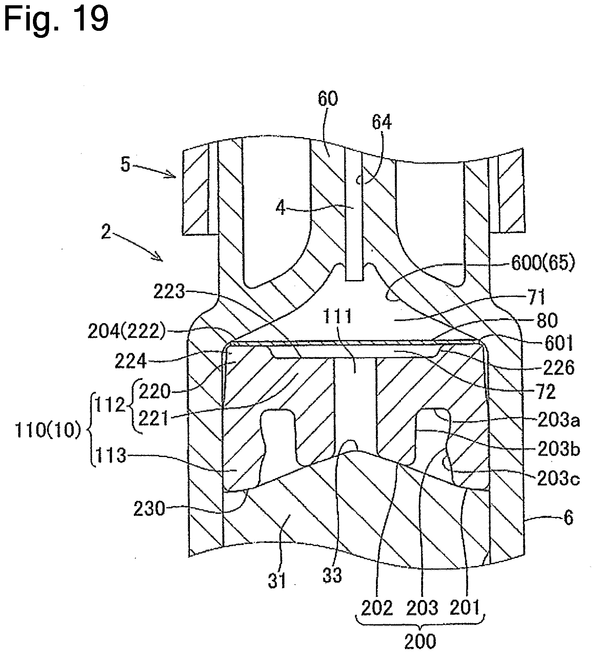

Specifically, although the connection contact surface 203 of the above-described embodiment extends along the axial direction, it may include a portion extending in a direction inclined against the axial direction. More specifically, as illustrated in Figs. 18 and 19, the connection contact surface 203 may include a first connection contact surface 203a that is a surface substantially vertical to the axial direction, and second connection contact surfaces 203b and 203c that are a pair of surfaces extending from the both edges of the first connection contact surface 203a along the axial direction.

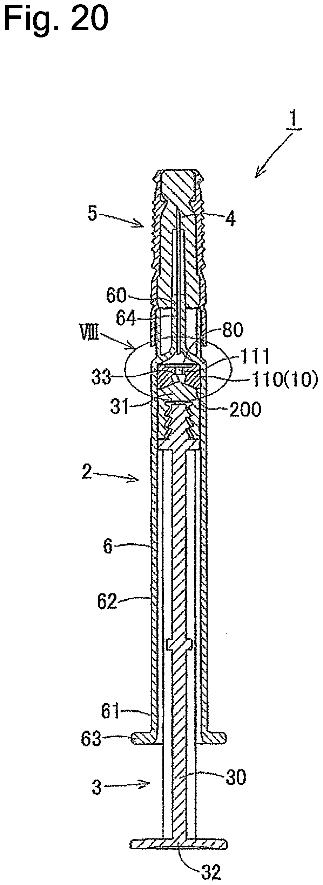

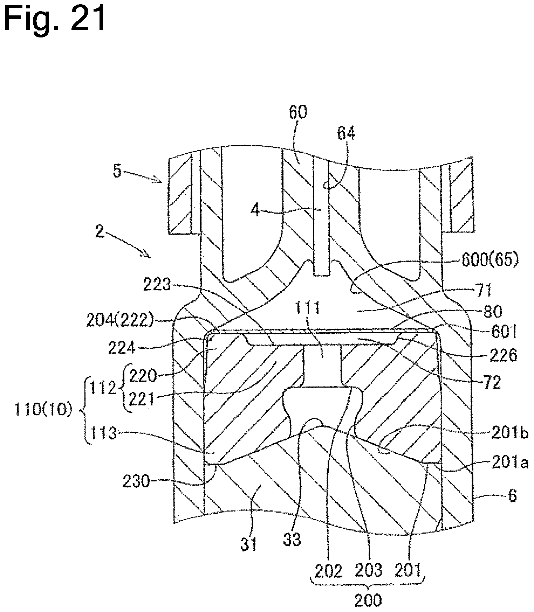

Specifically, although the base end side contact surface 201 of the above-described embodiment is a surface substantially vertical to the axial direction, it may include a portion inclined at an angle other than vertical against the axial direction. More specifically, as illustrated in Figs. 20 and 21, the base end side contact surface 201 may include a first base end side contact surface 201a that is a surface substantially vertical to the axial direction, and a second base end side contact surface 201b that is a surface extending further to the radially inward direction from the edge in the radially inside direction of the first base end side contact surface 201a, and inclined to have a radially inner portion positioned closer to the leading end side.

Even if such a structure is employed, when the leading end surface 33 of the piston 3 moves until it comes into surface contact with the contact surface 200 of the holding member 10 in the entire circumferential direction, a residual liquid between the piston 3 and the holding member 10 can be suppressed, and hence the residual liquid amount of the drug in the barrel 6 can be suppressed.

Although the holding member 10 of the above-described embodiment includes the base end side portion 110, it may be constituted in such a manner as to include the leading end side portion disposed on the leading end side of the filter 80 integrally with or separately from the base end side portion 110.

Besides, although the inner diameter of the flow path 111 is smaller than the inner diameter of the base end side subspace 72 in the holding member 10 of the above-described embodiment, it may substantially the same as the inner diameter of the base end side subspace 72.

Although the holding member 10 of the above-described embodiment is constituted in such a manner that the leading end surface 33 of the piston 3 comes into surface contact with the contact surface 200 of the base end side portion 110 over the entire circumferential direction of the barrel 6, it may be constituted in such a manner that the leading end surface 33 of the piston 3 comes into surface contact therewith partially in the circumferential direction. For example, it can be deemed that recesses or projections may be provided intermittently along the circumferential direction on the base end side contact surface 201 of the contact surface 200 of the base end side portion 110.

Although the leading end side subspace 71 is in a tapered shape having a smaller inner diameter toward the leading end side in the syringe 2 of the above-described embodiment, it may be in another shape, such as a shape having an even inner diameter. In this case, it can be deemed that the base side end surface 600 extends, for example, in a direction vertical to the axial direction.

Although the base end side subspace 72 is in a tapered shape having a smaller inner diameter toward the leading end side, it may be in another shape, such as a shape having an even inner diameter.

Besides, the dimension in the axial direction of the flow path 111 may be equal to or smaller than the dimension in the axial direction of the base end side subspace 72.

Besides, the dimension in the axial direction of the leading end side subspace 71 may be equal to or smaller than the dimension in the axial direction of the base end side subspace 72. In this case, it can be deemed, for example, that the base end side portion 110 of the holding member 10 is disposed in a position contacting the base side end surface 600 with the inclination angle of the base side end surface 600 of the leading end portion 60 of the barrel 6 against the axis reduced, or that the dimension in the axial direction of the base end side portion 110 of the holding member 10 is increased.

The partitioning means 7 or the holding member 10 is disposed in such a manner that the volume of the leading end side space of the barrel 6 is 150 mm³ or less, 110 mm³ or less, 40 mm³ or less, or 20 mm³ or less in some cases, and preferably 18 mm³ or less. The volume of the leading end side space (S2) of the barrel 6 is 15% by volume or less, 7% by volume or less, 5% by volume or less, or 2% by volume or less, and preferably 1.8% by volume or less of the entire barrel 6.

Herein, the sliding resistance of the syringe refers to resistance caused in sliding the piston within the barrel, and is expressed as sliding load. Herein, the term "ejection resistance" is also used in the same meaning as the sliding resistance.

### (2) Filter

The filter 80 may be, for example, a membrane, a mesh, a sintered body, or a foam body. The material of the filter 80 is a resin, a ceramic, a metal, paper, or the like. In the present embodiment, the filter 80 is, for example, a membrane filter. It is noted that the filter 80 may be a pre-filter.

In one aspect, the filtration area of the filter 80 is the area of the leading end side edge of the base end side subspace (72, or S22), and the area of the base end side edge of the leading end side subspace (71, or S21). The area of the leading end side edge of the base end side subspace (72, or S22) is, for example, equal to the area of the base end side edge of the leading end side subspace (71, or S21).

In one aspect, the filter has a thickness of 100 µm to 400 µm, 125 µm to 350 µm, or 150 µm to 310 µm.

In one aspect, the filter has, when dried or wet, tensile strength of 1000 Psi or more, 2000 Psi or more, or 3000 Psi or more, and preferably 3500 Psi or more. More preferably, the filter has tensile strength of 3700 Psi or more when dried, and tensile strength of 3900 Psi or more when wet.

In one aspect, the filter has, when dried or wet, elongation at break of 10% or more, 20% or more, 30% or more, or 40% or more, and has elongation at break of preferably 45% or more. More preferably, the filter has elongation at break of 50% or more when dried, and 60% or more when wet.

In one aspect, the material of the filter contains one or more selected from polyether sulfone (PES), polyvinylidene fluoride (PVDF), polysulfone (PS), and an acrylic copolymer.

Polyether sulfone (PES) has a structure including a repeating unit of the following formula:

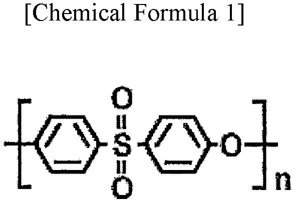

In one aspect, polyether sulfone is preferably Supor(R) (Nihon Pall Ltd.), Millipore Express (Merck), or Sartopore(R) (Sartorius), and particularly preferably Supor.

Polyvinylidene fluoride (PVDF) contains a repeating unit of the following formula:

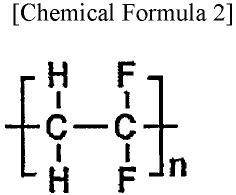

In one aspect, polyvinylidene fluoride is preferably Durapore(R) (Merck), or Fluorodyne(R) II (Nihon Pall Ltd.), and particularly preferably Durapore.

Polysulfone (PS) has a structure including a repeating unit of the following formula:

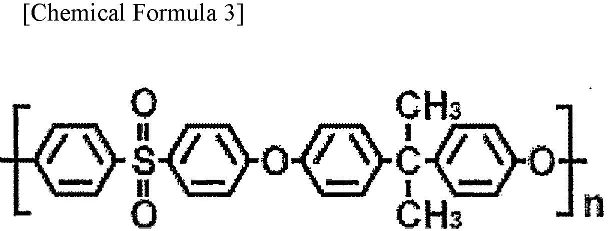

In one aspect, polysulfone is preferably Asymmetric Super Micron Polysulfone (Nihon Pall Ltd.).

In one aspect, an acrylic copolymer may be formed on a non-woven nylon support. Alternatively, an acrylic copolymer may be coated with a hydrophilic or hydrophobic coating. In one aspect, an acrylic copolymer filter is preferably a hydrophobic acrylic copolymer filter, and particularly preferably VERSAPOR(R) (Nihon Pall Ltd.).

When air enters a hydrophilic filter after it is wet with a solution, an air lock phenomenon that an air reservoir is formed within the thickness of the membrane may occur in some cases. A pre-filled syringe has a prescribed amount of voids therein, and it cannot be denied that there is a possibility of formation of an air lock because the air contained in the voids pass therethrough after the solution passes depending on the direction of the usage. When an air lock is formed, the filtration performance is seriously degraded, but it is known that such a phenomenon does not generally occur in a hydrophobic filter. Accordingly, when the filter is a hydrophobic filter, the above-described risk can be avoided.

In one aspect, the filter has a pore size of 5 µm or less, and preferably of 5 µm.

Herein, the term "pore size of a filter" refers to a property of the filter rated in accordance with the diameter of a particle definitely expected to be highly efficiently captured. As filter rating, "nominal filter rating" and "absolute filter rating" are used. The nominal filter rating refers to a range of sizes of microparticles for which the filter maker can guarantee against a prescribed foreign matter removal rate. The absolute filter rating refers to a value corresponding to the size of the minimum microparticle that is completely removable by the filter. A method for measuring the nominal filter rating can be ACFTD (AC Fine Test Dust), and in this method, a particle size satisfying a set particle removal rate is determined as the filter rating. A method for measuring the absolute filter rating can be a method in which ACFTD or glass beads are caused to pass through the filter once, and the maximum diameter of ACFTD or beads flowed out downstream is measured. Herein, the pore size is expressed by the absolute filter rating.

Herein, the term "filter integrity" refers to a standard for the quality of a filter, and indicates whether or not the filter exhibits expected filtration performance in a significant step requiring sterility assurance. Examples of a filter integrity test include a forward flow test and a bubble point test. The forward flow test is a quantitative test in which a given test pressure is applied to a completely wet membrane on the primary side of the filter when the secondary side of the membrane is in an atmospheric pressure state, and the amount of diffusional flow passing through the liquid membrane is measured based on a difference in pressure between the primary side and the secondary side of the filter. The bubble point test is a quantitative test in which a given test pressure is applied to a completely wet membrane on the primary side of the filter when the secondary side of the membrane is in an atmospheric pressure state, and the amount of diffusional flow passing through the liquid membrane is measured based on a difference in pressure between the primary side and the secondary side of the filter.

Herein, the phrase "the solution is kept in a state not in contact with the filter before use" refers to that the solution is filled in the barrel in such a manner as not to contact the filter provided in the barrel, and is kept in a state not in contact therewith before use even if stress at usually expected level is applied thereto. In this case, the term "before use of the formulation for injection" includes during and after the production of the formulation for injection. The term "stress at usually expected level" refers to, but is not limited to, mechanical stress including drop stress, vibration stress, and rotation stress applied during the production and operations such as transportation after the production. In one aspect, the formulation for injection is subjected to, as the stress at usually expected level, drop stress once, twice, three times, four times, five times, six times, seven times, eight times, nine times, ten times, eleven times, twelve times, thirteen times, fourteen times, fifteen times, sixteen times, seventeen times, eighteen times, nineteen times, twenty times, twenty-one times, twenty-two times, twenty-three times, twenty-four times, twenty-five times, twenty-five times or more, thirty times or more, or forty times or more. The stress applied to the pre-filled syringe at the time of drop varies depending on not only the number of times of the drop but also the height, the direction, and the like of the drop. The drop height is, but is not limited to, for example, 38.1 cm, which is described in American Society for Testing and Materials (ASTM) D4169. In one aspect, the non-contact state between the solution and the filter is realized by partitioning the barrel with the partitioning means, holding the pharmaceutical formulation in the base end side space, and disposing the filter on the leading end side of the partitioning means. The phrase "the solution is kept in a state in contact with the filter before use" refers to that the solution is kept in a state in contact with the filter during a prescribed period or whole period during the production of the formulation for injection, or storage, transportation and the like thereof after the production.

Herein, the phrase "the solution flows through the filter from the base end side to the leading end side of the filter" refers to that the solution filled on the base end side of the filter in the barrel of the syringe moves to the leading end side of the filter in the barrel owing to the piston, or pressure application including the weight. The solution having moved to the leading end side of the filter in the barrel is ejected externally through the nozzle hole.

### (3) Pharmaceutical Formulation

In one aspect of the present invention, the pharmaceutical formulation is a solution containing, as an active ingredient, a low molecular weight compound, a middle molecular weight compound including a natural product, a polynucleotide, and a peptide, or a protein. Herein, the low molecular weight compound refers to a compound having a molecular weight lower than 500, and the middle molecular weight compound refers to a compound having a molecular weight of 500 to 1500. In one aspect of the present invention, the middle molecular weight compound encompasses a natural product having a molecular weight of 500 to 2000, a polynucleotide having a molecular weight of 3000 to 15000, and a peptide having a molecular weight of 600 to 6000. The peptide encompasses a cyclic peptide. Besides, the polynucleotide encompasses a ribozyme, an antisense molecule, an inhibitor oligonucleotide, an aptamer, a micro-RNA, and a small interfering RNA (siRNA). The pharmaceutical formulation may be a formulation for injection.

In one aspect of the present invention, the formulation for injection is a pharmaceutical formulation that is filled in a container for injection to be administered by injection, and contains a protein as an active ingredient in a solution. In one aspect of the present invention, the formulation for injection includes a container for injection such as a syringe as a constituent element.

In one aspect of the present invention, the solution of the pharmaceutical formulation has a viscosity of 95 cP or less, 90 cP or less, 85 cP or less, 80 cP or less, 75 cP or less, 70 cP or less, 65 cP or less, 60 cP or less, 55 cP or less, 50 cP or less, 45 cP or less, 40 cP or less, 35 cP or less, 30 cP or less, 25 cP or less, 20 cP or less, 15 cP or less, 10 cP or less, or 1 cP to 95 cP. The viscosity can be measured by employing, for example, electromagnetically spinning (EMS) method. In this measurement method, a torque is applied by remote control to a probe that is a conductor, and the resultant rotation is optically read to measure the viscosity.

In one aspect of the present invention, the pharmaceutical formulation is stored without freezing the solution in the container at -30°C to 40°C, or -30°C to 25°C, preferably from the freezing point of the solution to 25°C, more preferably at 1°C to 10°C, more preferably at 2°C to 8°C, and even more preferably at 5°C. The storage is carried out for 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 48 hours, 60 hours, 72 hours, 84 hours, or 96 hours. The storage is carried out for at least 24 hours, at least 2 days, at least 3 days, at least 4 days, at least 10 days, at least 20 days, at least 30 days, at least 40 days, at least 50 days, at least 60 days, at least 1 month, at least 2 months, at least 3 months, at least 4 months, at least 5 months, at least 6 months, at least 7 months, at least 8 months, at least 9 months, at least 10 months, at least 11 months, or at least 12 months.

In one aspect of the present invention, the protein used in a liquid formulation encompasses, but is not limited to, an antibody, a fusion protein, an enzyme, a hormone, a cytokine, and a vaccine. More specifically, the protein encompasses a monoclonal antibody, granulocyte colony-stimulating factor (G-CSF), granulocyte macrophage colony-stimulating factor (GM-CSF), erythropoietin (EPO), interferon, interleukins such as IL-1 or IL-6, tissue plasminogen activator (TPA), thrombopoietin, urokinase, serum albumin, blood coagulation factor VIII, leptin, stem cell factor (SCF), and the like.

In one aspect of the present invention, the protein used in the pharmaceutical formulation has substantially the same biological activity as a bioactive protein of a mammal, in particular of a human, and encompasses proteins derived from nature and those obtained by genetic engineering. Proteins obtained by genetic engineering include those having the same amino acid sequence as a native protein, or those obtained by deleting, substituting, or adding one or more amino acid sequences and having the above-described biological activity.

In one aspect of the present invention, a concentration of the protein in the solution may be 0.1 mg/mL or more, within a range of 0.1 to 300 mg/mL, within a range of 1 to 200 mg/mL, 50 to 200 mg/mL, or 80 to 200 mg/mL.

In one aspect of the present invention, an antibody to be used is not particularly limited as long as it binds to a desired antigen, may be a polyclonal antibody or a monoclonal antibody, and is preferably a monoclonal antibody because a homogeneous antibody can be thus stably produced. In one aspect of the present invention, the antibody to be used may be a monospecific antibody or a bispecific antibody, or may be a multispecific antibody having three or more antigen-recognizing sites in a molecule.

In one aspect of the present invention, the monoclonal antibody to be used encompasses not only a monoclonal antibody derived from an animal, such as a human, a mouse, a rat, a hamster, a rabbit, a sheep, a camel, or a monkey, but also a recombinant antibody obtained by artificial modification, such as a chimeric antibody, a humanized antibody, or a bispecific antibody. Furthermore, a recombinant antibody obtained by artificial modification of a constant region or the like of an antibody for modifying physical properties of an antibody molecule (specifically, modification of an isoelectric point (p1), modification of affinity of Fc receptor, and the like) for purposes of improving retention in blood and pharmacokinetics is also encompassed.

In one aspect of the present invention, the immunoglobulin class of the antibody to be used is not particularly limited, but may be any of classes including IgG such as IgG1, IgG2, IgG3, and IgG4, and IgA, IgD, IgE, and IgM, among which IgG is preferred, and IgG1, IgG2 and IgG4 are particularly preferred.

Further, in one aspect of the present invention, the antibody to be used encompasses not only an antibody including a constant region and a variable region (full-length antibody) but also a bound fragment of an antibody, such as Fv, Fab, and F(ab)₂, and a low-molecular-weight antibody like a bispecific antibody such as one- or two-associated single chain Fv (scF, sc(Fv)₂) having a variable region of the antibody bound with a linker such as a peptide linker, or a scFV dimer, and a full-length antibody is preferred.

In one aspect of the present invention, the antibody to be used can be produced by a known method. A hybridoma used for producing a monoclonal antibody can be produced as follows basically by known techniques. Specifically, a desired antigen or a cell expressing a desired antigen used as a sensitizing antigen is immunized by a usual immunization method, and the resultant immune cell is fused with a known parent cell by a usual cell fusion method, the resultant is subjected to a usual screening method for screening a monoclonal antibody producing cell (hybridoma), and thus, the hybridoma can be produced. The production of a hybridoma can be carried out in accordance with, for example, the method of Milstein et al., (Kohler, G. and Milstein, C., Methods Enzymol.

(1981) 73: 3-46) or the like. If immunogenicity of the antigen is low, the antigen may be bound to a macromolecule having immunogenicity, such as albumin, before the immunization.

Alternatively, a recombinant antibody obtained by cloning an antibody gene from a hybridoma, and incorporating the gene into an appropriate vector to be introduced into a host by genetic engineering techniques can be used (see, for example, Carl, A. K. Borrebaeck, James, W. Larrick, THERAPEUTIC MONOCLONAL ANTIBODIES, Published in the United Kingdom by MACMILLAN PUBLISHERS LTD., 1990). Specifically, cDNA of a variable region (V region) of an antibody is synthesized from mRNA of the hybridoma with reverse transcriptase. DNA encoding the V region of the target antibody thus obtained is linked to DNA encoding a desired antibody constant region (C region), and the resultant is incorporated into an expression vector. Alternatively, DNA encoding the V region of the antibody may be incorporated into an expression vector containing DNA of an antibody C region. The resultant is incorporated into the expression vector so as to express under control of an expression control region, such as an enhancer or a promoter. Next, a host cell is transformed with the resultant expression vector, and thus, the antibody can be expressed.

In one aspect of the present invention, a recombinant antibody obtained by artificial modification for purpose of reducing heteroantigenicity against human, such as a chimeric antibody or a humanized antibody, can be used. Such a modified antibody can be produced by a known method. A chimeric antibody is an antibody containing heavy-chain and light-chain variable regions of an antibody of a mammal other than a human, for example, a mouse antibody, and heavy-chain and light-chain constant regions of a human antibody, and can be obtained by linking DNA encoding a variable region of a mouse antibody to DNA encoding a constant region of a human antibody, incorporating the resultant into an expression vector, and introducing the vector into a host to produce the antibody.

A humanized antibody is designated also as a reshaped human antibody, and is obtained by transplanting a complementary determining region (CDR) of an antibody of a mammal other than a human, for example, a mouse antibody, into a complementary determining region of a human antibody, and a general genetic engineering method for such an antibody is also known. Specifically, a DNA sequence designed to link CDR of a mouse antibody to a framework region (FR) of a human antibody is synthesized by PCR method from several oligonucleotides produced to have overlapping portions at the ends. The thus obtained DNA is linked to DNA encoding a human antibody constant region, the resultant is subsequently incorporated into an expression vector, and the resultant vector is introduced into a host to produce the antibody (see European Patent Application No. 239400, and WO 96/02576). As the FR of a human antibody to be linked via CDR, one having a complementary determining region forming a good antigen-binding site is selected. If necessary, an amino acid in a framework region of a variable region of an antibody may be substituted so as to cause the complementary determining region of the reshaped human antibody to form a suitable antigen-binding site (Sato, K. et al., Cancer Res. (1993) 53, 851-856).

As techniques for substituting an amino acid of an antibody for improving activity, physical properties, pharmacokinetics, safety and the like of the antibody, for example, the following techniques are known, and in one aspect of the present invention, the antibody to be used encompasses such an antibody having substitution (including deletion and addition) of an amino acid.

As techniques for amino acid substitution in a variable region of an IgG antibody, not only humanization (Tsurushita N., Hinton P.R., Kumar S., Design of humanized antibodies: from anti-Tac to Zenapax., Methods, 2005 May; 36 (1): 69-83) but also affinity maturation by amino acid substitution in a complementary determining region (CDR) for enhancing binding activity (Rajpal A., Beyaz N., Haber L., Cappuccilli G., Yee H., Bhatt R.R., Takeuchi T., Lerner R.A., Crea R., A general method for greatly improving the affinity of antibodies by using combinatorial libraries, Proc Natl Acad Sci USA, 2005 Jun 14; 102 (24): 8466-71), and improvement of physicochemical stability by amino acid substitution in framework (FR) (Ewert S., Honegger A., Pluckthun A., Stability improvement of antibodies for extracellular and intracellular applications: CDR grafting to stable frameworks and structure-based framework engineering, Methods, 2004 Oct; 34(2): 184-99. Review) have been reported. As techniques for amino acid substitution in Fc region of an IgG antibody, techniques for enhancing antibody dependent cellular cytotoxicity (ADCC) or complement dependent cellular cytotoxicity (CDC) are known (Kim S.J., Park Y., Hong H.J., Antibody engineering for the development of therapeutic antibodies, Mol Cells, 2005 Aug 31; 20(1): 17-29 Review.). In addition, techniques for amino acid substitution in Fc by not only enhancing such effector function but also improving half-life in blood of an antibody have been reported (Hinton P.R., Xiong J.M., Johlfs M.G., Tang M.T., Keller S., Tsurushita N., An engineered human IgG1 antibody with longer serum half-life, J Immunol. 2006 Jan 1; 176(1): 346-56, Ghetie V., Popov S., Borvak J., Radu C., Matesoi D., Medesan C., Ober R.J., Ward E.S., Increasing the serum persistence of an IgG fragment by random mutagenesis, Nat Biotechnol. 1997 Jul; 15(7): 637-40.). Furthermore, various techniques for amino acid substitution in a constant region for purposes of improving physical properties of an antibody are known (WO 09/41613).

Besides, there are known methods for obtaining a human antibody. For example, human lymphocyte is sensitized with a desired antigen or a cell expressing a desired antigen *in vitro,* and the thus sensitized lymphocyte is fused to a human myeloma cell, for example, U266, and thus, a desired human antibody having a binding activity to an antigen can be obtained (see Japanese Patent Publication No. 1-59878). Furthermore, when a transgenic animal having all repertoires of human antibody genes is immunized with an antigen, a desired human antibody can be obtained (see WO 93/12227, WO 92/03918, WO 94/02602, WO 94/25585, WO 96/34096, and WO 96/33735). Besides, a technique for obtaining a human antibody by panning using a human antibody library is also known. For example, a variable region of a human antibody is expressed as a single chain antibody (scFv) on the surface of a phage by a phage display method, and thus, a phage binding to an antigen can be selected. When the gene of the selected phage is analyzed, a DNA sequence encoding the variable region of a human antibody binding to the antigen can be determined. When the DNA sequence of scFv binding to the antigen is clarified, an appropriate expression vector containing the sequence can be produced to obtain a human antibody. These methods are already known, and can be executed with the reference to WO 92/01047, WO 92/20791, WO 93/06213, WO 93/11236, WO 93/19172, WO 95/01438, and WO 95/15388. In one aspect of the present invention, the antibody to be used encompasses such human antibodies.

When an antibody is produced by isolating an antibody gene once, and introducing the gene into an appropriate host, an appropriate combination of a host and an expression vector can be used. When a eukaryotic cell is used as the host, an animal cell, a plant cell, or a fungal cell can be used. As the animal cell, (1) mammal cells such as CHO, COS, myeloma, BHK (baby hamster kidney), HeLa, and Vero, (2) amphibian cells such as Xenopus oocyte, and (3) insect cells such as sf9, sf21, and Tn5 are known. As the plant cell, a cell derived from the genus *Nicotiana,* such as a cell derived from *Nicotiana tabacum,* is known, and this cell may be callus cultured. As the fungal cell, yeast, for example, the genus *Saccharomyces,* such as *Saccharomyces serevisiae,* and filamentous fungus, for example, the genus *Aspergillus* such as *Aspergillus niger* are known. When a prokaryotic cell is used, there is a production system using a bacterial cell. As the bacterial cell, *Escherichia coli* (*E. coli*) and *Bacillus subtilis* are known. When a target antibody gene is introduced into such a cell by transformation, and the thus transformed cell is cultured *in vitro,* the antibody can be obtained.

Besides, the antibody to be used in the pharmaceutical formulation encompasses a modified antibody. For example, antibodies binding to various molecules of polyethylene glycol (PEG), cytotoxic drugs and the like can be used (Farmaco. 1999 Aug 30; 54(8): 497-516, Cancer J. 2008 May-June; 14(3): 154-69). Such a modified antibody can be obtained by chemically modifying an antibody. Such a method has been already established in this field.

In one aspect of the present invention, the antibody of the present disclosure may be a chimeric antibody. A chimeric antibody is described in, for example, US Patent No. 4,816,567, and Morrison et al., Proc. Natl. Acad. Sci. USA, 81: 6851-6855 (1984). A chimeric antibody may contain a non-human variable region (variable region derived from, for example, a non-human primate such as a monkey, or a mouse, a rat, a hamster, a rabbit or the like) and a human constant region.

In one aspect of the present invention, the antibody of the present disclosure may be a humanized antibody. Representatively, a humanized antibody is humanized for reducing immunogenicity in a human with specificity and affinity of a parent non-human antibody retained. A humanized antibody representatively contains one or more variable regions, and an HVR, for example, CDR derived from a non-human antibody (or a part thereof) and FR derived from a human antibody sequence (or a part thereof) are present therein. A humanized antibody can optionally contain at least a part of a human constant region. In one embodiment, amino acid residues of FR in a humanized antibody may be substituted with corresponding amino acid residues of a non-human antibody (for example, an antibody from which HVR residues are derived) for, for example, retaining or improving specificity and affinity of the antibody.

A humanized antibody and a method for producing the same are reviewed in, for example, the following (Almagro and Fransson, Front. Biosci. 13: 1619-1633 (2008)), and are described in, for example, the following: Riechmann et al., Nature 332: 323-329 (1988); Queen et al., Proc. Nat'l Acad. Sci. USA 86: 10029-10033 (1989); US Patent Nos. 5,821,337, 7,527,791, 6,982,321, and 7,087,409; Kashmiri et al., Methods 36: 25-34 (2005) (describing specificity determining region (SDR) grafting); Padlan, Mol. Immunol. 28: 489-498 (1991) (describing "resurfacing"); Dall'Acqua et al., Methods 36: 43-60 (2005) (describing "FR shuffling"); and Osbourn et al., Methods 36: 61-68 (2005) and Klimka et al., Br. J. Cancer, 83: 252-260 (2000) (describing "guided selection" approach to FR shuffling).

In one aspect of the present invention, a human framework that may be used in humanization may contain, for example, a framework selected by a "best fit" method (Sims et al., J. Immunol. 151: 2296 (1993)), a framework derived from a consensus sequence of a human antibody belonging to a specific subgroup of a heavy chain or light chain variable region (Carter et al., Proc. Natl. Acad. Sci. USA, 89: 4285 (1992), and Prest et al., J. Immunol., 151: 2623 (1993)), or a framework region derived from screening of FR library (Baca et al., J. Biol. Chem. 272: 10678-10684 (1997) and Rosok et al., J. Biol. Chem. 271: 22611-22618 (1996)).

In one aspect of the present invention, the antibody of the present disclosure may be a human antibody. A human antibody can be produced by various techniques. A human antibody is outlined in, for example, van Dijk and van de Winkel, Curr. Opin. Pharmacol. 5: 368-374 (2001) and Lonberg, Curr. Opin. Immunol. 20: 450-459 (2008). A human antibody may be prepared by administering an immunogen to a transgenic animal having been modified to produce a complete human antibody or a complete antibody containing a human variable region in response to an antigen. Such an animal representatively contains the entire or a part of human immunoglobulin locus, and the entire or a part of the human immunoglobulin locus is present in a state where it is substituted with an endogenous immunoglobulin locus, or it is randomly incorporated outside the chromosome or inside the chromosome of the animal. In such a transgenic mouse, endogenous immunoglobulin locus is usually inactivated. A method for obtaining a human antibody from a transgenic animal is reviewed in Lonberg, Nat. Biotech. 23: 1117-1125 (2005). Besides, make reference to, for example, US Patent Nos. 6,075,181 and 6,150,584 describing XENOMOUSE(TM) technology; US Patent No. 5,770,429 describing HUMAB(R) technology; US Patent No. 7,041,870 describing K-M MOUSE(R); and US2007/0061900 describing VELOCIMOUSE(R) technology. A human variable region from a complete antibody generated from such an animal may be further modified by, for example, combining with a different human constant region.

In another aspect of the present invention, a human antibody can be produced by a method based on a hybridoma. A human myeloma cell and a mouse-human heteromyeloma cell line for producing a human monoclonal antibody is described in the following (for example, Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991)). A human antibody generated through human B cell hybridoma technology is described in Li et al., Proc. Natl. Acad. Sci. USA, 103: 3557-3562 (2006). Other examples of the method include US Patent No. 7,189,826 (describing production of a monoclonal human IgM antibody from a hybridoma cell line), and Ni, Xiandai Mianyixue, 26 (4): 265-268 (2006) (describing a human-human hybridoma). Human hybridoma technology (trioma technology) is described in Vollmers and Brandlein, Histology and Histopathology, 20(3): 927-937 (2005), and Vollmers and Brandlein, Methods and Findings in Experimental and Clinical Pharmacology, 27(3): 185-91 (2005).

In another aspect of the present invention, a human antibody may also be generated by isolating an Fv clone variable domain sequence selected from human-derived phage display libraries. Such a variable region sequence can then be combined with a desired human constant region. Techniques for selecting a human antibody from antibody libraries will be described below.

In one aspect of the present invention, the antibody of the present disclosure may be isolated by screening a combinatorial library for an antibody having one or more desired activities. For example, a method for creating a phage display library, a method for screening such a library for an antibody having a desired binding characteristic, and the like are known in this technical field. Such methods are reviewed in Hoogenboom et al. in Methods in Molecular Biology 178:1-37 (O'Brien et al., ed., Human Press, Totowa, NJ, 2001), and are described in, for example, McCafferty et al., Nature 348: 552-554; Clackson et al., Nature 352: 624-628 (1991); Marks et al., J. Mol. Biol. 222: 581-597 (1992); Marks and Bradbury, Molecular Biology 248: 161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In a specific phage display method employed in one aspect of the present invention, repertoires of VH and VL can be separately cloned by polymerase chain reaction (PCR), and recombined randomly in phage libraries, and the phage libraries can then be screened for antigen-binding phage as described in Winter et al., Ann. Rev. Immunol., 12: 433-455 (1994). A phage displays a bound fragment of an antibody, such as scFv or Fab. Libraries from immunized sources can provide high-affinity antibodies to the immunogen without the requirement of constructing hybridomas. In another embodiment, a naive repertoire can be cloned (for example, from a human) to provide a single source of antibodies to a wide range of non-self or self-antigens without any immunization as described by Griffiths et al., EMBO J, 12: 725-734 (1993). In a still another embodiment, naive libraries can also be created synthetically by cloning unrearranged V-gene segments from stem cells, and using PCR primers containing a random sequence encoding the highly variable region CDR3 and to accomplish rearrangement *in vitro,* as described in Hoogenboom and Winter, J. Mol. Biol., 227: 381-388 (1992). Examples of patent publications describing human antibody phage libraries include US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

An antibody isolated from a human antibody library, or a bound fragment thereof is herein regarded as a human antibody or a bound fragment of a human antibody.

In one aspect of the present invention, the antibody of the present disclosure is a multispecific antibody (such as a bispecific antibody). A multispecific antibody is an antibody having binding specificities in at least two different sites (for example, a monoclonal antibody). In one embodiment, one of the binding specificities is specificity to an antigen, and the other is specificity to another antigen. In another embodiment, a bispecific antibody may bind to two epitopes of different antigens. The bispecific antibody may be used for localizing a cytotoxic agent in a cell expressing the antigen. The bispecific antibody may be prepared as a full-length antibody or a bound fragment of an antibody.

A method for producing a multispecific antibody is not limited, and examples include recombinant co-expression of two immunoglobulin heavy chain-light chain pairs having different specificities (for example, Milstein and Cuello, Nature 305: 537 (1983), WO93/08829, and Traunecker et al., EMBO J. 10: 3655 (1991)), and knob-in-hole technology (for example, US Patent No. 5,731,168). A multispecific antibody may be produced by controlling electrostatic steering effects for producing an Fc heterodimer molecule (for example, WO2009/089004 A1); cross-linking two or more antibodies or bound fragments thereof (for example, US Patent No. 4,676,980 and Brennan et al., Science, 229: 81 (1985)); producing an antibody having two specificities with leucine zipper (for example, Kostelny et al., J. Immunol., 148 (5): 1547-1553 (1992)); producing a bispecific bound fragment by "diabody" technology (for example, Hollinger et al., Proc. Natl. Acad. Sci. USA, 90: 6444-6448 (1993)); using a scFv dimer (for example, Gruber et al., J. Immunol., 152: 5368 (1994)); or preparing a trispecific antibody (for example, Tutt et al., J. Immunol. 147: 60 (1991)). Alternatively, the multispecific antibody may be an antibody engineered to have three or more functional antigen-binding sites, including an "octopus antibody" (for example, US2006/0025576).

In one aspect of the present invention, the antibody or the bound fragment thereof of the present disclosure may be a "dual acting Fab" or "DAF" containing one antigen-binding site binding to the antigen and another different antigen (for example, US2008/0069820).

In one aspect, a variant (mutant) of an amino acid sequence of the antibody of the present disclosure can be prepared by introducing an appropriate modification to a nucleic acid encoding a molecule of the antibody, or by synthesizing a peptide. Such a modification may be performed through one of or an appropriate combination of a plurality of deletion, insertion, and substitution of an arbitrary amino acid (residue) in the amino acid sequence. An arbitrary combination of deletion, insertion, and substitution can be employed as long as a final construct possesses a desired characteristic (for example, antigen-binding property).

In one aspect of the present invention, when an antigen variant (mutant) resulting from one of or a plurality of amino acid substitutions is provided, a target site for introducing substitution mutation can contain HVR and FR.

Examples of the antibody used in the pharmaceutical formulation include, but are not limited to, an anti-tissue factor antibody, anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, an anti-VLA4 antibody, an anti-HM1.24 antibody, an anti-parathyroid gland hormone-related peptide antibody (anti-PTHrP antibody), an anti-ganglioside GM3 antibody, an anti-TPO receptor agonist antibody, a coagulation factor VIII substitution antibody, an anti-IL31 receptor antibody, an anti-HLA antibody, an anti-AXL antibody, an anti-CXCR4 antibody, an anti-NR10 antibody, and a bispecific antibody of factor IX and factor X.

Examples of a preferable reshaped humanized antibody to be used in the pharmaceutical formulation include a humanized anti-interleukin 6 (IL-6) receptor antibody (tocilizumab, hPM-1, or MRA, see WO92/19759), a humanized anti-HM1.24 antigen monoclonal antibody (see WO98/14580), a humanized anti-parathyroid gland hormone-related peptide antibody (anti-PTHrP antibody) (see WO98/13388), a humanized anti-tissue factor antibody (see WO99/51743), a humanized anti-glypican-3 IgG1κ antibody (codrituzumab, GC33, see WO2006/006693), a humanized anti-NR10 antibody (see WO2009/072604), and a bispecific humanized antibody of factor IX and factor X (ACE910, see WO2012/067176).

In one aspect of the present invention, the active ingredient contained in the solution filled in the syringe is an antibody selected from emicizumab, tocilizumab, and satralizumab. Emicizumab, tocilizumab, and satralizumab are antibodies respectively contained, as an active ingredient, for example, in HEMLIBRA(R), ACTEMRA(R), and ENSPRYNG(R), and their amino acid sequences are known. Herein, antibodies specified by the terms emicizumab, tocilizumab, and satralizumab encompass antibodies that are substantially the same in amino acid sequences as these antibodies, and have equivalent medical effects.

In one aspect of the present invention, the pharmaceutical formulation can be prepared, if necessary, by mixing with an appropriate pharmaceutically acceptable carrier, medium, or the like into a liquid formulation. The solvent of a liquid formulation is water or a pharmaceutically acceptable organic solvent. Examples of such an organic solvent include propylene glycol (1,2-propanediol), polyethylene glycol 300, polyethylene glycol 400, ethanol, glycerol, and acetic acid. Examples of appropriate pharmaceutically acceptable carrier and medium include sterilized water and physiological saline, a stabilizing agent, an antioxidant (such as ascorbic acid), a buffer (such as phosphoric acid, citric acid, histidine, or another organic acid), an antiseptic agent, a surfactant (such as PEG, or Tween), a chelating agent (such as EDTA), and a binding agent. Other low molecular weight polypeptides, proteins such as serum albumin, gelatin, and immunoglobulin, amino acids such as glycine, glutamine, asparagine, glutamic acid, aspartic acid, methionine, arginine, and lysine, sugars and carbohydrates such as polysaccharides and monosaccharides, and sugar alcohols such as mannitol and sorbitol may be contained. In the case of obtaining a solution for injection, examples include physiological saline, an isotonic solution containing glucose or another auxiliary drug, such as D-sorbitol, D-mannose, D-mannitol, or sodium chloride, and it may be used together with an appropriate solubilizing agent such as alcohol (such as ethanol), polyalcohol (such as propylene glycol, or PEG), and a nonionic surfactant (such as polysorbate 80, polysorbate 20, poloxamer 188, or HCO-50).

In one aspect of the present invention, the buffer to be used in a liquid formulation is prepared using a substance for retaining the pH of the solution. In one aspect of the present invention, a liquid formulation containing high concentration of an antibody has a pH of the solution of preferably 4.5 to 7.5, more preferably 5.0 to 7.0, and even more preferably 5.5 to 6.5. In one aspect of the present invention, a usable buffer can adjust the pH within this range, and is pharmaceutically acceptable. Such a buffer is known to those skilled in the art in the field of liquid formulation, and inorganic salts such as phosphates (sodium or potassium) and sodium bicarbonate; organic salts such as citrates (sodium or potassium), sodium acetate, and sodium succinate; and acids such as phosphoric acid, carbonic acid, citric acid, succinic acid, malic acid, and gluconic acid can be used. Alternatively, Tris buffers, and Good's buffers such as MES, MOPS, and HEPES, histidine (such as histidine hydrochloride), glycine, and such can be used.

Generally, the concentration of the buffer is 1 to 500 mmol/L, preferably 5 to 100 mmol/L and more preferably 10 to 20 mmol/L. In using a histidine buffer, the buffer preferably contains 5 to 25 mmol/L histidine, and more preferably contains 10 to 20 mmol/L histidine.

In one aspect of the present invention, a liquid formulation containing a high concentration of an antibody is preferably stabilized by addition of a stabilizing agent suitable for the antibody corresponding to the active ingredient. In one aspect of the present invention, no significant change is observed in a "stable" liquid formulation containing a high concentration of an antibody for at least 12 months, preferably for 2 years, and more preferably for 3 years at refrigeration temperature (2 to 8°C); or for at least 3 months, preferably for 6 months, and more preferably for 1 year at room temperature (22 to 28°C). For example, a total amount of dimers and decomposed matters after storage at 5°C for 2 years is 5.0% or less, preferably 2% or less, and more preferably 1.5% or less, or the total amount of dimers and decomposed matters after storage at 25°C for 6 months is 5.0% or less, preferably 2% or less, and more preferably 1.5% or less.

In one aspect of the present invention, representative examples of the surfactant include nonionic surfactants, for example, sorbitan fatty acid esters such as sorbitan monocaprylate, sorbitan monolaurate, and sorbitan monopalmitate; glycerin fatty acid esters such as glycerin monocaprylate, glycerin monomyristate, and glycerin monostearate; polyglycerin fatty acid esters such as decaglyceryl monostearate, decaglyceryl distearate, and decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, and polyoxyethylene sorbitan tristearate; polyoxyethylene sorbit fatty acid esters such as polyoxyethylene sorbit tetrastearate and polyoxyethylene sorbit tetraoleate; polyoxyethylene glycerin fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol ether, polyoxyethylene polyoxypropylene propyl ether, and polyoxyethylene polyoxypropylene cetyl ether; polyoxyethylene alkyl phenyl ethers such as polyoxyethylene nonylphenyl ether; polyoxyethylene hydrogenated castor oils such as polyoxyethylene castor oil, and polyoxyethylene hydrogenated castor oil (polyoxyethylene hydrogen castor oil); polyoxyethylene beeswax derivatives such as polyoxyethylene sorbit beeswax; polyoxyethylene lanolin derivatives such as polyoxyethylene lanolin; polyoxyethylene fatty acid amides such as polyoxyethylene stearic acid amide that have HLB 6 to 18; anionic surfactants, for example, alkyl sulfates having an alkyl group having 10 to 18 carbon atoms such as sodium cetyl sulfate, sodium lauryl sulfate, and sodium oleyl sulfate; polyoxyethylene alkyl ether sulfates such as sodium polyoxyethylene lauryl sulfate having an average added molar number of ethylene oxide of 2 to 4 and an alkyl group having 10 to 18 carbon atoms; alkylsulfosuccinic acid esters having an alkyl group having 8 to 18 carbon atoms such as sodium lauryl sulfosuccinate ester; and natural surfactants, for example, lecithin and glycerophospholipids; sphingo-phospholipids such as sphingomyelin; and sucrose fatty acid esters of fatty acids having 12 to 18 carbon atoms. In one aspect of the present invention, one or more of these surfactants can be added in combination to the formulation.

Preferred surfactants include polyoxyethylene sorbitan fatty acid esters and polyoxyethylene polyoxypropylene alkyl ethers, polysorbates 20, 21, 40, 60, 65, 80, 81, 85, and Pluronic(R) surfactants are particularly preferred, and polysorbates 20 and 80 and Pluronic F-68 (poloxamer 188) are most preferred.

In one aspect of the present invention, an amount of the surfactant to be added to the antibody formulation is generally 0.0001 to 10% (mg/mL), preferably 0.001 to 5%, and more preferably 0.005 to 3%.

To the formulation of the present invention, a cryoprotectant, a suspending agent, a dissolution assisting agent, a tonicity agent, a preservative, an adsorption inhibitor, a diluent, an excipient, a pH adjustor, a soothing agent, a sulfur-containing reducing agent, an antioxidant and the like can be appropriately added.

Examples of the cryoprotectant include sugars such as trehalose, sucrose, and sorbitol.

Examples of the dissolution assisting agent include polyoxyethylene hydrogenated castor oil, polysorbate 80, nicotinamide, polyoxyethylene sorbitan monolaurate, macrogol, and castor oil fatty acid ethyl ester.

Examples of the tonicity agent include sodium chloride, potassium chloride, and calcium chloride.

Examples of the preservative include methyl parahydroxybenzoate, ethyl parahydroxybenzoate, sorbic acid, phenol, cresol, and chlorocresol.

Examples of the adsorption inhibitor include human serum albumin, lecithin, dextran, an ethylene oxide/propylene oxide copolymer, hydroxypropyl cellulose, methyl cellulose, polyoxyethylene hydrogenated castor oil, and polyethylene glycol.

Examples of the sulfur-containing reducing agent include those containing sulfhydryl groups such as N-acetylcysteine, N-acetylhomocysteine, thioctic acid, thiodiglycol, thioethanol amine, thioglycerol, thiosorbitol, thioglycolic acid and salts thereof, sodium thiosulfate, glutathione, and thioalkanoic acids having 1 to 7 carbon atoms.

Examples of the antioxidant include erythorbic acid, dibutylhydroxytoluene, butylhydroxyanisole, α-tocopherol, tocopherol acetate, L-ascorbic acid and salts thereof, L-ascorbic acid palmitate, L-ascorbic acid stearate, sodium hydrogen sulfite, sodium sulfite, triamyl gallate, propyl gallate, and chelating agents such as disodium ethylenediamine tetraacetate (EDTA), sodium pyrophosphate, and sodium metaphosphate.

In one aspect of the present invention, the pharmaceutical formulation is used for treating an autoimmune disease, an immune disease, an infectious disease, an inflammatory disease, a nervous system disease, and a tumor disease and a neoplasm disease including cancer. In a specific embodiment, the pharmaceutical is used for treating congestive heart failure (CHF), ischemia-induced severe arrhythmia, hypercholesteremia, vasculitis, rosacea, acne, eczema, myocarditis, and other states of cardiac muscle, Kawasaki Disease, systemic lupus erythematosus, diabetes, spondylosis, synovial fibroblast, and bone marrow stroma; bone loss; Paget's disease, giant cell tumor of bone; breast cancer; disuse bone loss; malnutrition, periodontal disease, Gaucher's disease, Langerhans cell histiocytosis, spinal cord injury, acute purulent arthritis, osteomalacia, Cushing's syndrome, monostotic fibrous dysplasia, polyostotic fibrous dysplasia, periodontal membrane reconstruction, and fracture; sarcoidosis; melanoma, prostate cancer, pancreas cancer, osteolytic bone cancer, breast cancer, lung cancer, stomach cancer, renal cancer, and rectal cancer; bone metastasis, bone pain management, humoral hypercalcemia of malignancy, ankylosing spondylitis, and other spondylarthrosis; transplant rejection, viral infection, hematological neoplasm, and neoplasm-like state such as Hodgkin's lymphoma; non-Hodgkin's lymphoma (Burkitt's lymphoma, small lymphocytic lymphoma/chronic lymphatic leukemia, mycosis fungoides, mantle cell lymphoma, follicularlymphoma, diffuse large B-cell lymphoma, marginal zone lymphoma, hairy cell leukemia, and lymphoplasmacytic leukemia), tumors of lymphocyte progenitor cells including B-cell acute lymphoblastic leukemia/lymphoma and T-cell acute lymphoblastic leukemia/lymphoma, thymoma, peripheral T-cell leukemia, adult T-cell leukemia/T-cell lymphoma, and tumors of mature T-cell and nature NK cell including large granular lymphocyte leukemia, Langerhans cell histiocytosis, AML with maturation, AML without differentiation, acute myelocytic leukemias including acute promyelocytic leukemia, acute myelomonocytic leukemia, and acute monocytic leukemia, myelodysplastic syndromes, and bone marrow neoplasms such as chronic myeloproliferative disease including chronic myelocytic leukemia, tumors of central nervous system, for example, brain tumor (glioma, neuroblastoma, astrocytoma, medulloblastoma, ependymoma, and retinoblastoma), solid tumor (nasopharyngeal cancer, basal cell carcinoma, pancreatic cancer, cholangiocarcinoma, Kaposi's sarcoma, testicular cancer, uterine cancer, vaginal cancer or cervical cancer, ovarian cancer, primary hepatic cancer, or endometrial cancer, and tumors of vascular system (angiosarcoma and hemangiopericytoma), osteoporosis, hepatitis, HIV, AIDS, spondyloarthritis, rheumatoid arthritis, inflammatory bowel disease (IBD), sepsis and septic shock, Crohn's disease, psoriasis, scleroderma, graft versus host disease (GVHD), allogeneic islet graft rejection, multiple myeloma (MM), myelodysplastic syndromes (MDS), and hematologic malignancy such as acute myelogenous leukemia (AML), inflammation related to tumor, peripheral nerve injury, or demyelinating disease. In a specific embodiment, the pharmaceutical is used for treating psoriasis vulgaris, pancreatitis, ulcerative colitis, non-Hodgkin's lymphoma, breast cancer, colorectal cancer, mesothelioma, soft tissue sarcoma, juvenile idiopathic arthritis, macular degeneration, respiratory syncytial virus, Crohn's disease, rheumatoid arthritis, psoriatic arthritis, Castleman's disease, ankylosing spondylitis, osteoporosis, treatment-induced bone loss, bone metastasis, multiple myeloma, Alzheimer's disease, glaucoma, Sjogren's disease, Still's disease, multiple sclerosis, hyperimmunoglobulinemia, anemia, mesangial proliferative nephritis, and asthma.

In one aspect of the present invention, an antigen to which the antibody has a specific binding property can be a transmembrane molecule (such as a receptor) or a ligand such as a growth factor. Representative examples of the antigen include a molecule such as renin; growth hormones including human growth hormone and bovine growth hormone; a growth hormone-releasing factor; parathyroid gland hormone; thyroid stimulating hormone; lipoprotein; α-1 anti-trypsin; insulin A chain; insulin B chain; proinsulin; follicle-stimulating hormone; calcitonin; luteinizing hormone; glucagon; coagulation factors such as factor VIIIC, factor IX, tissue factor (TF), and von Willebrand factor; anticoagulation factors such as protein C; atrial natriuretic factor; a pulmonary surfactant; plasminogen activators such as urokinase, and human urinary or tissue plasminogen activator (t-PA); bombesin; thrombin; hematopoietic cell growth factor; tumor necrosis factor-α and -β; enkephalinase; RANTES (regulated on activation normally T-cell expressed and secreted); human macrophage inflammatory protein (MIP-1-α); serum albumin such as human serum albumin; Mullerian-inhibiting substance; relaxin A chain; relaxin B chain; prorelaxin; mouse gonadotropin releasing hormone-related peptide; microbial proteins such as β-lactamase; DNAse; IgE; cytotoxic T-lymphocyte related antigens (CTLA) such as CTLA-4; inhibin; activin; vascular endothelial growth factor (VEGF); hormone or growth factor receptors; protein A or D; rheumatoid factor; neurotrophic factor, such as bone-derived neurotrophic factor (BDNF), or neurotrophin-3, -4, -5, or -6 (NT-3, NT-4, NT-5, or NT-6), and nerve growth factor such as NGF-b; platelet-derived growth factor (PDGF); fibroblast growth factors such as aFGF and bFGF; epidermal growth factor (EGF); transforming growth factors (TGF) such as TGF-α, and TGF-β including TGF-b1, TGF-b2, TGF-b3, TGF-b4, and TGF-b5; tumor necrosis factors (TNF) such as TNF-α and TNF-β; insulin-like growth factor-I and -II (IGF-I and IGF-II); des(1-3)-1GF-I (brain IGF-I), and insulin-like growth factor binding protein; CD proteins such as CD3, CD4, CD8, CD19, CD20, CD22, and CD40; erythropoietin; bone morphogenetic protein; an antitoxin; bone morphogenetic protein (BMP); interferons such as interferon-α, -β, and -γ; colony stimulating factors (CSF) such as M-CSF, GM-CSF, and G-CSF; interleukins (IL) such as IL-1, IL-2, IL-3, IL-4, IL-5, IL-6, IL-7, IL-8, IL-9, and IL-10; superoxide dismutase; a T-cell receptor; a surface membrane protein; a decay acceleration factor; viral antigens such as a part of AIDS envelope; a transport protein; a homing receptor; addressin; regulatory proteins; integrins such as CD11a, CD11b, CD11c, CD18, ICAM, VLA-4, and VCAM; tumor-related antigens such as HER2, HER3, and HER4 receptors; and a fragment of any of the above-described polypeptides.

In one aspect of the present invention, examples of a molecular target of the antibody comprised therein include CD proteins such as CD3, CD4, CD8, CD19, CD20, CD22, CD34, and CD40; members of the ErbB receptor family such as EGF receptor, and HER2, HER3, or HER4 receptor; B-cell surface antigens such as CD20 and BR3; members of the tumor necrosis receptor superfamily including DR5; prostate stem cell antigen (PSCA); cell adhesion molecules such as LFA-1, Mac1, p150.95, VLA-4, ICAM-1, VCAM, and αv/β3 integrins including any one of α4/β7 integrin, and α or β subunit thereof (such as an anti-CD11a, anti-CD18, or anti-CD1 1b antibody); growth factors such as VEGF and receptors thereof; tissue factors (TF); tumor necrosis factors (TNF), such as TNF-α or TNF-β, and α-interferon (α-IFN); interleukins such as IL-8; IgE; blood group antigens; flk2/flk3 receptor; obesity (OB) receptor; mp1 receptor; CTLA-4; and protein C.

### (4) Particles

In one aspect of the present invention, the phrase "to remove visually detectable particles" refers to that in a solution of a pharmaceutical formulation in which visually detectable particles are formed under prescribed conditions, when the solution is ejected from the syringe, the solution is ejected through the filter provided in the barrel, thereby removing the visually detectable particles from the ejected solution. In one aspect of the present invention, the number of the visually detectable particles in the ejected solution becomes zero. The particle size and number can be determined by a light obscuration particle count method, a microscopic particle count method, a flow cytometric particle image analysis method, visual inspection, and infrared microspectroscopy (infrared spectroscopy ;IR) or Raman microspectroscopic measurement performed after isolation of particles, and are measured preferably by a combination of visual inspection and infrared microspectroscopy or Raman microspectroscopic measurement.

### (5) Visually Detectable Particles

Herein, a visually detectable particle refers to a particle that is visually detectable under high illuminance and has a particle size of 40 µm or more. Among them, particles that are visually detectable under standard illuminance as prescribed in the Japanese pharmacopoeia (about 2,000 to 3,000 lx) are designated as "visible particles" or "insoluble visible particles". Generally, visible particles have a particle size greater than 100 µm (Non Patent Literature 1). Particles that are smaller in size than visible particles, and cannot be seen with eyes with standard illuminance as prescribed in the Japanese pharmacopoeia (about 2,000 to 3,000 lx) but can be visually detected by increasing illuminance or by increasing an observation time period are "particles visually detectable only under high illuminance", and have a particle size of 40 µm to 100 µm. Visible particles can be confirmed by visual inspection with naked eyes for 5 seconds or more under illumination at standard illuminance (about 2,000 to 3,000 lx), by slowly rotating or inverting the container in front of a black background or a white background. Particles visually detectable only under high illuminance can be confirmed by visual inspection with naked eyes for 30 seconds or more under illumination at high illuminance (6,000 lx or more) by slowly rotating or inverting the container in front of a black background. Visible particles can also be confirmed with inspection under high illuminance.

Herein, the term "protein-derived particle" or "particle derived from a component of the solution" refers to a visually detectable particle generated from a protein, including a particle containing only a protein, and a particle containing a complex of a protein and polydimethylsiloxane (PDMS). It can be confirmed by Raman microspectroscopic measurement that the particle contains a protein molecule. It is only an active pharmaceutical ingredient (API) that is contained in the solution as a protein, and a visually detectable particle is generated from the API. The number of visually detectable particles can be determined by a light obscuration particle count method, a microscopic particle count method, a flow cytometric particle image analysis method, visual inspection, and infrared microspectroscopy (infrared spectroscopy; IR) or Raman microspectroscopic measurement performed after isolation of particles, and is measured preferably by a combination of visual inspection and infrared microspectroscopy or Raman microspectroscopic measurement.

Herein, the term "external particle" or "particle derived from a substance except for the component of the solution" refers to a visually detectable particle excluding those described above.

Herein, the term "aggregate" is a protein species having a comparatively high molecular weight generated by aggregation of a large number of modified proteins, and is used interchangeably with the term "high molecular weight species" and "HMWS". Protein aggregates can be generally different in the following points: The sizes (aggregates of a small size (dimer) to a large size (microscopically visible particles, or more visible particles), having a diameter in the range of nanometer to micrometer), the forms (substantially spherical to fibrous forms), the protein structure (native or non-native/modified), the type of intermolecular bond (covalent or non-covalent), the reversibility, and the solubility. Soluble aggregates cover the size range of about 1 to 100 nm, and protein particles cover a microscopically visible range (about 0.1 to 100 µm), and a visible range (> 100 µm). Protein aggregates of all of the above-described types are generally encompassed in this term. Accordingly, the term "(protein) aggregate" refers to all types of non-native species in which two or more protein monomers are physically associated with or chemically bound to each other.

In one aspect of the present invention, the term "pre-filled syringe" means a syringe used as a container and filled with a liquid composition. In one embodiment, a pre-filled syringe contains a pharmaceutical composition for administration to a patient filled in the syringe. Here, the syringe may be covered with a syringe closure, such as, but not limited to, a stopper. In one embodiment, the composition is put into the syringe in a filling facility for production. In one embodiment, the syringe is sterilized before putting the composition therein. In one embodiment, the pre-filled syringe may be stored, before administering the composition to a patient, for a period of one day, or at least 7 days, or at least 14 days, or at least 1 month, or at least 6 months, or at least 1 year, or at least 2 years. In one aspect, in the pre-filled syringe, the solution held in the syringe is kept in a state not in contact with the filter even if stress at usually expected level is applied during the production or transportation.

### EXAMPLES

### Example 1 Selection of Filter Material having High Microparticle Removal Ability

Each of a general COP syringe with a 27G needle (1 mL standard), and six types of COP syringes with a 27G needle (1 mL standard) respectively including filters of a pore size of 5 µm of different materials (polyether sulfone (PES) (Supor(R); Nihon Pall Ltd.), polyvinylidene fluoride (PVDF) (Durapore(R); Merck), polyethylene terephthalate (PET), nylon, polysulfone (Asymmetric Super Micron Polysulfone; Nihon Pall Ltd.), and a hydrophobic acrylic copolymer (VERSAPOR(R); Nihon Pall Ltd.) was filled with 1.0 mL of an antibody solution (satralizumab (mAb1): 120 mg/mL, buffer: 20 mmol/L histidine, stabilizer: 150 mmol/L arginine and 162 mmol/L aspartic acid, surfactant: 0.50 mg/mL poloxamer 188, pH 6.0), and the resultant syringes were plugged with stoppers. The antibody solution was supplied to this test after storage at 25°C or 40°C for intentionally increasing the amount of microparticles in the formulation. The antibody solution was in contact with the filter. In each of the thus filled and plugged samples, the antibody solution held in the syringe was discharged with an autograph (Model No. AG-1, manufactured by Shimadzu Corporation) at a rate of 210 mm/min or 100 m/min into a clean glass vial having been washed and sterilized. Phosphate buffered saline was added in an appropriate ratio to the thus discharged solution or the original solution, and thereafter, the resultant was mixed by inverting twenty times, and then was allowed to stand still for 2 hours. Immediately before measurement, the resultant was mixed by slowly inverting three times. The number of microparticles contained in the solution was measured with a micro-flow imaging device (Model No. DPA4100, manufactured by Brightwell) for trials 1 and 2, and with a micro-flow imaging device (Model No. MFI5200, manufactured by Protein Simple) for a trial 3.

### [Evaluation Results]

The numbers of microparticles of 10 µm or more, and 25 µm or more in the solutions discharged from the general COP syringe with a 27G needle and the COP syringes with a 27G needle respectively including the various filters are shown in Table 1.

As a result, the following was found. When the antibody solution was discharged from the syringes including the filters of PES, PVDF, polysulfone, and the hydrophobic acrylic copolymer, the number of microparticles in the solution can be largely reduced, but when the solution was discharged from the syringe including the filter of PET or nylon, the reduction of the amount of microparticles in the solution is insufficient. Such a difference is caused probably because the filters of PES, PVDF, polysulfone, and the hydrophobic acrylic copolymer are all membrane filters, but the filters of PET and nylon are net filters, that is, a type of a depth filter.

**[Table 1]**

| Table 1: Number of microparticles in solution after discharge from general syringe and syringe including filter | | | | | |
|---|---|---|---|---|---|
| Solution | Experiment | Filger | Discharge rate (mm/min) | Number of microparticles (/ 1.0 mL) | |
| | | | | ≥ 10 µm | ≥ 25 µm |
| mAb1 120 mg/mL | Trial 1 | - (General syringe) | 210 | 1542 | 124 |
| | | PES 5 µm | | 14 | 0 |
| | | PVDF 5 µm | | 41 | 0 |
| | | PET 5 µm | | 972 | 41 |
| | | Nylon 5 µm | | 1229 | 55 |
| mAb1 120 mg/mL | Trial 2 | - (Original solution) | - | 1367 | 139 |
| | | Polysulfone 5 µm | 210 | 21 | 0 |
| mAb1 120 mg/mL | Trial 3 | - (Original solution) | - | 601 | 54 |
| | | Acrylic copolymer 5 µm | 100 | 10 | 0 |

### Example 2 Selection of Optimum Filter Pore Size

An antibody solution (satralizumab (mAb1): 120 mg/mL, buffer: 20 mmol/L histidine, stabilizer: 150 mmol/L arginine and 162 mmol/L aspartic acid, surfactant: 0.50 mg/mL poloxamer 188, pH 6.0) was filtered through a filter unit of stainless steel in which a filter of PES of a pore size of 1.2 µm or 5 µm punched out into a shape of a circle having a diameter of 47 mm to be collected in a clean plastic container. Phosphate buffered saline was added in an appropriate ratio to the thus collected solution or the original solution, and thereafter, the resultant was mixed by inverting twenty times, and then was allowed to stand still for 2 hours. Immediately before measurement, the resultant was mixed by slowly inverting three times, and the number of microparticles contained in the solution was measured with a micro-flow imaging device (Model No. MFI5200, manufactured by Protein Simple). In the same manner as in Example 1, the antibody solution was supplied to this test after storage at 25°C or 40°C for intentionally increasing the amount of microparticles in the formulation.

### [Evaluation Results]

The numbers of particles of 25 µm or more, and 70 µm or more in the original solution and the sample solutions filtered through the filters of a pore size of 1.2 µm and a pore size of 5 µm are shown in Table 2.

As a result, it was revealed that when the solution is filtered through the filters of a pore size of 1.2 µm and a pore size of 5 µm, the ability of reducing the amount of particles in the solution is not largely different therebetween, and that the pore size of about 5 µm may be sufficient. Besides, the amount of flow under a pressure of 70 kPa is 315 mL/min/cm² when the pore size is 1.2 µm, and 778 mL/min/cm² when the pore size is 5 µm. Therefore, the ejection resistance caused by the introduction of the filter is about 2.5-fold when the pore size is 1.2 µm as compared with that when the pore size is 5 µm. In consideration of the balance between the particle removal ability to be attained and the increase of the ejection resistance, it was determined that the pore size of 5 µm is optimum.

Besides, a COP syringe with a 27G needle (1 mL standard) including a hydrophobic acrylic copolymer filter (pore size: 5 µm) was filled with 1.0 mL of a sucrose solution (viscosity: 7 cP), and the resultant was plugged with a stopper. The sucrose solution was kept in a state not in contact with the filter. In the thus filled and plugged sample, the sucrose solution held in the syringe was discharged with an autograph (Model No.: AG-1, manufactured by Shimadzu Corporation) at a rate of 100 mm/min or 210 m/min into an appropriate container. Average sliding resistance values for 10 mm to 25 mm are shown in Table 3. The average sliding resistance value was obtained by calculation performed based on data obtained by the autograph.

As a result, the average sliding resistance value of the syringe including the filter was within 1.3-fold of that of a general syringe, and thus, the sliding resistance was found to be suppressed to a sufficiently small value although the filter is included.

**[Table 2]**

| Table 2: Number of microparticles in solution after discharge from syringe including filter of 1.2 µm and 5 µm | | | |
|---|---|---|---|
| Solution | Filter | Number of microparticles (/ 1.0 mL) | |
| | | ≥ 25 µm | ≥ 70 µm |
| mAb1 120 mg/mL | - (Original solution) | 56 | 3 |
| | PES 1.2 µm | 2 | 0 |
| | PES 5 µm | 13 | 0 |

**[Table 3]**

| Table 3: Average sliding resistance value for 10 mm to 25 mm of general syringe and syringe including filter | | | | |
|---|---|---|---|---|
| Type/discharge rate | 100 mm/min | | 210 mm/min | |
| | Resistance value (N) | Ratio to general syringe | Resistance value (N) | Ratio to general syringe |
| General syringe | 2.60 | 1 | 5.07 | 1 |
| Syringe including filter | 3.30 | 1.27 | 6.18 | 1.22 |

### Example 3 Evaluation of Filter Durability

Each of three types of sucrose solutions (viscosity: 10 cP, 15 cP, and 20 cP) was filled in an amount of 1.0 mL in a COP syringe with a 27G needle (1 mL standard) including a hydrophobic acrylic copolymer filter (pore size: 5 µm), and the resultant was plugged with a stopper. Besides, a drug solution was discharged from Cimzia(R): 200mg AutoClicks for S.C. Injection, and filled in an amount of 1.0 mL in a COP syringe with a 27G needle (1 mL standard) including a hydrophobic acrylic copolymer filter (pore size: 5 µm), and the resultant was plugged with a stopper. The viscosity of the Cimzia solution measured with an EMS viscometer (Model No. EMS-1000, manufactured by Kyoto Electronics Manufacturing Co., Ltd.) was 96.4 cP at 25°C. The sucrose solution and the Cimzia solution were kept in a state not in contact with the filter. In the thus filled and plugged sample, the sucrose solution held in the syringe was discharged with an autograph (Model No. AG-1, manufactured by Shimadzu Corporation) at a rate of 1000 mm/min into an appropriate container. Besides, the Cimzia solution held in the syringe was manually discharged (N = 10). The discharging time was measured with a stopwatch to calculate a discharge rate. After the discharge, the filter unit was taken out of the syringe to confirm whether or not the filter was damaged.

### [Evaluation Results]

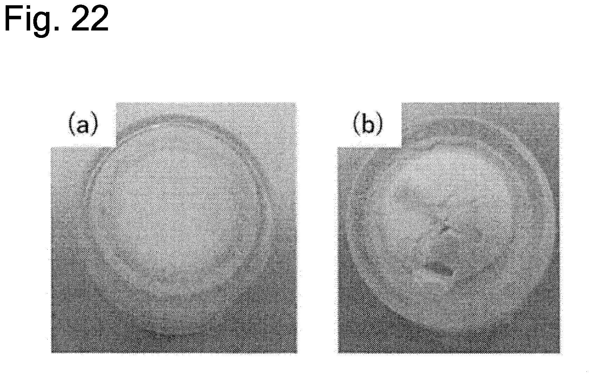

The maximum load in discharging the sucrose solutions with a viscosity of 10 to 20 cP at a rate of 1000 mm/min, and whether or not the filter was damaged after the discharge are shown in Table 4. The discharge rate in manually discharging the Cimzia solution with a viscosity of 96.4 cP, and whether or not the filter was damaged after the discharge are shown in Table 5. Besides, examples of a normal filter and a damaged filter are illustrated in Fig. 22. As a result of the evaluation, it was understood that the filter was damaged in none of the samples, and thus had sufficient durability, and can be used for a variety of drugs.

**[Table 4]**

| Table 4: Maximum load and filter damage after discharge of sucrose solution at 1000 mm/min | | |
|---|---|---|
| Solution | Maximum load | Filter damage rate (%) |
| Sucrose solution 10cP | about 30 N | 0/16 (0%) |
| Sucrose solution 15cP | about 45 N | 0/16 (0%) |
| Sucrose solution 20cP | about 60 N | 0/16 (0%) |

**[Table 5]**

| Table 5: Calculated discharge rate and filter damage after manual discharge of Cimzia solution | | |
|---|---|---|
| Solution | Discharge rate (mm/min) | Filter damage rate (%) |
| Cimzia solution 96.4cP | 152 to 316 | 0/10 (0%) |

Example 4 Evaluation of Proteinaceous Foreign Matter Removal Ability The following three pharmaceutical formulations were used for evaluation:
[Formulation 1] ACTEMRA(R) (active ingredient: tocilizumab): 162 mg for subcutaneous injection
[Formulation 2] HEMLIBRA(R) (active ingredient: emicizumab): 150 mg for subcutaneous injection
[Formulation 3] ENSPRYNG(R) (active ingredient: satralizumab): 120 gm for subcutaneous injection

Each formulation was discharged into a plastic container, and then was filled in an amount of 1.0 mL in a COP syringe with a 27G needle (1 mL standard) including a hydrophobic acrylic copolymer filter (pore size: 5 µm), and the resultant was plugged with a stopper. It is noted that a sample obtained by replacement with buffer components the same as those for the formulations 2 and 3 (buffer: 20 mmol/L histidine, stabilizer: 150 mmol/L arginine and 162 mmol/L aspartic acid, surfactant: 0.50 mg/mL poloxamer 188, pH 6.0) was used as the formulation 1. Samples were kept at 40°C for intentionally generating proteinaceous foreign matters in the syringes. Samples containing proteinaceous foreign matters were created (N = 5) for each formulation, and before discharging the solution, the syringe was slowly rotated or inverted at a position directly below a white light source, at a brightness of about 10,000 lx, and in front of a black background, and visual inspection was performed for about 30 seconds with naked eyes to confirm whether or not foreign matters were present in the solution filled in the syringe, and if present, the number was confirmed. Besides, the syringe was slowly rotated or inverted also at a brightness of about 3,000 lx, and in front of black and white backgrounds, and visual inspection was performed for about 30 seconds with naked eyes to confirm whether or not foreign matters were present in the solution filled in the syringe, and if present, the number was confirmed. The number of proteinaceous foreign matters was found, at a brightness of about 10,000 lx, to be 1 in a sample including a few foreign matters, and 10 or more in a sample including many foreign matters. Besides, the number was found to be 0 to 4 at a brightness of about 3,000 lx. Each of the filled and plugged samples was manually discharged into a clean glass vial having been washed and sterilized. After the discharge, it was confirmed whether or not foreign matters were present in the vial at a brightness of about 10,000 lx, and a brightness of about 3,000 lx, and if present, the number was confirmed.

### [Evaluation Results]

The presence rate of the proteinaceous foreign matters and the number of proteinaceous foreign matters obtained after the discharge from the COP syringe with a 27G needle including the hydrophobic acrylic copolymer filter (pore size: 5 µm) are shown in Tables 6 and 7. As a result, it was confirmed that all the ten types of proteinaceous foreign matters can be appropriately removed by the discharge from the syringe including the hydrophobic acrylic copolymer filter having a pore size of 5 µm.

**[Table 6]**

| Table 6: Presence rate of proteinaceous foreign matters before and after discharge from syringe including filter | | | | |
|---|---|---|---|---|
| Type of foreign matters | Presence rate of proteinaceous foreign matters (%) (observation brightness: 10,000 Ix) | | Presence rate of proteinaceous foreign matters (%) (observation brightness: 3,000 Ix) | |
| | before discharge | after discharge | before discharge | after discharge |
| Formulation 1 | 5/5 (100%) | 0/5 (0%) | 4/5 (80%) | 0/5 (0%) |
| Formulation 2 | 5/5 (100%) | 0/5 (0%) | 5/5 (100%) | 0/5 (0%) |
| Formulation 3 | 5/5 (100%) | 0/5 (0%) | 5/5 (100%) | 0/5 (0%) |

**[Table 7]**

| Table 7: Number of proteinaceous foreign matters before and after discharge from syringe including filter | | | | |
|---|---|---|---|---|
| Type of foreign matters | Number of proteinaceous foreign matters | | Number of proteinaceous foreign matters | |
| | (observation brightness: 10,000 Ix) | | (observation brightness: 3,000 Ix) | |
| | before discharge | after discharge | before discharge | after discharge |
| Formulation 1 | 10 | 0 | 5 | 0 |
| Formulation 2 | 50 | 0 | 20 | 0 |
| Formulation 3 | 50 | 0 | 24 | 0 |

| | | | | |
|---|---|---|---|---|
| *1 A sample found to have 10 or more foreign matters was determined to have 10 foreign matters for convenience. | | | | |

In addition to the above-described three types, five types of prefilled syringe formulations or vial formulations already on the market were subjected to a similar test. Among these, three types were formulations for subcutaneous injection, and two were those for intravenous drip infusion. These samples were stored, after the discharge from the containers, in a plastic tube at 40°C for intentionally generating proteinaceous foreign matters. Each of the solutions was filled in an amount of 1.0 mL in a COP syringe with a 27G needle (1 mL standard) including the hydrophobic acrylic copolymer filter (pore size: 5 µm), and the resultant was plugged with a stopper. As a result of the test on these five formulations, it was confirmed that 13 to 31 proteinaceous foreign matters can be removed at an observation brightness of 10,000 lx, and that 4 to 7 proteinaceous foreign matters can be removed at an observation brightness of 3,000 lx.

### Example 5 Evaluation of External Foreign Matter Removal Ability

A sucrose solution having a viscosity of 7 cP was filled in an amount of 1.0 mL in a COP syringe with a 27G needle (1 mL standard) including a hydrophobic acrylic copolymer filter (pore size: 5 µm). Five each of five types of external foreign matters (size: about 700 µm to 6000 µm, material: glass, SUS, a rubber plug, a plastic, and fiber) were added respectively to different syringes, and the resultants were plugged with stoppers. N = 3 samples containing each of the external foreign matters were created, and before discharging the solution from the syringe, it was confirmed at a brightness of about 3,000 lx that there were foreign matters in the syringe. A Rigid Needle Shield (RNS) was removed by assembling a plunger rod thereon, and then, the solution held in the syringe was manually discharged into a clean glass vial having been washed and sterilized. It was confirmed at a brightness of about 3,000 lx whether or not the foreign matters were present in the solution discharged to the vial.

### [Evaluation Results]

Table 8 shows whether or not the external foreign matters were present in the solution discharged from the COP syringe with a 27G needle including the hydrophobic acrylic copolymer filter (pore size: 5 µm). As a result of this test, it was confirmed that all of these five types of external foreign matters can be appropriately removed by discharging the solutions containing the external foreign matters from the syringes including the hydrophobic acrylic copolymer filters with a pore size of 5 µm.

**[Table 8]**

| Table 8: Presence rate (%) of external foreign matters before and after discharge from syringe including filter | | |
|---|---|---|
| Type of foreign matters | Presence rate (%) of external foreign matters (observation brightness: 3,000 Ix) | |
| | before discharge | after discharge |
| Glass | 3/3 (100%) | 0/3 (0%) |
| SUS | 3/3 (100%) | 0/3 (0%) |
| Rubber plug | 3/3 (100%) | 0/3 (0%) |
| Plastic | 3/3 (100%) | 0/3 (0%) |
| Fiber | 3/3 (100%) | 0/3 (0%) |

### Example 6 Drug Solution and Stability

The formulation 2 was filled in an amount of 1.0 mL in a general COP syringe with a 27G needle (1 mL standard) and in a COP syringe with a 27G needle (1 mL standard) including a hydrophobic acrylic copolymer filter (pore size: 5 µm), and the resultants were plugged with stoppers. The thus filled and plugged samples were stored at 5°C for 12 months, or at 25°C for 6 months, and the resultants were subjected to size exclusion chromatography (SE-HPLC) measurement, UV measurement (Model No. UV-2700i, manufactured by Shimadzu Corporation), and PX188 concentration measurement under the following conditions. In the size exclusion chromatography measurement, a detected main peak was defined as Main Peak, a sum of peaks detected before Main Peak was defined as High Molecular Weight (HMW), and a sum of peaks detected after Main Peak was defined as Low Molecular Weight (LMW). Results thus obtained are shown in Table 9. After the storage at 5°C for 12 months, or at 25°C for 6 months in the general COP syringe and the COP syringe including the filter, the physical properties were little changed in all the samples. Thus, it was confirmed that the COP syringe including the filter is highly compatible with drug solutions.

**[Table 9]**

| Table 9: Physical pro syringe including filter perty data after long-term storage in general syringe and | | | | | | | |
|---|---|---|---|---|---|---|---|
| Test item | | General syringe | | | Syringe including filter | | |
| | | Initial | after 12 months at 5°C | after 6 months at 25°C | Initial | after 12 months at 5°C | after 6 months at 25°C |
| SE-HPLC (area%) | Main | 99.9 | 99.7 | 99.2 | 99.9 | 99.8 | 99.5 |
| | HMW | 0.1 | 0.3 | 0.7 | 0.1 | 0.2 | 0.5 |
| | LMW | N.D.* | N.D.* | 0.1 | N.D.* | N.D.* | N.D.* |
| Protein concentration (mg/mL) | | 154 | 153 | 152 | 154 | 154 | 152 |
| PX188 concentration (mg/mL) | | 0.51 | 0.52 | 0.49 | 0.51 | 0.53 | 0.50 |

| | | | | | | | |
|---|---|---|---|---|---|---|---|
| *N.D. Not detected | | | | | | | |

- Size Exclusion Chromatography
- Size exclusion chromatograph: "2695 or e2695" manufactured by Waters
- Detector: UV absorptiometer
- Measurement wavelength: 280 nm
- Column: TSKgel G3000SWXL (manufactured by Tosoh Corporation)
- Column temperature: 25°C
- Mobile phase: phosphate buffer (pH 7.0)
- Flow rate: 0.5 mL/min
- Injection amount: 60 µL
- PX188 Concentration Measurement
- PX188 Concentration measuring device: "2690, 2695, e2795, or e2695" manufactured by Waters
- Detector: Differential refractometer (2414 Refractive Index Detector)
- Column: TSKgel SuperSW2000 (manufactured by Tosoh Corporation)
- Column temperature: 25°C
- Mobile phase: 40% acetonitrile solution containing 30 mmol/L sodium chloride
- Flow rate: 0.3 mL/min
- Injection amount: 20 µL.

### REFERENCE SIGNS LIST

1 ... pre-filled syringe, 2 ... syringe, 3 ... piston, 4 ... needle, 5 ... cap, 6 ... barrel, 7 ... partitioning means, 8 ... housing, 9 ... inner plug portion, 30 ... rod portion, 31 ... gasket, 32 ... operation portion, 33 ... leading end surface, 60 ... leading end portion, 61 ... base end portion, 62 ... cylindrical portion, 63 ... flange portion, 64 ... nozzle hole, 80 ... filter, 81 ... close-time engaging portion, 82 ... inner circumference, 83 ... attachment portion, 84 ... body portion, 85 ... extension portion, 86 ... filter member, 90 ... close-time engaged portion, 91 ... regulating means, 92 ... cover portion, 93 ... step engaging portion, 94 ... leg portion, 95 ... projecting portion, 96 ... circular portion, 97 ... cover recess portion, 98 ... contact surface, 600 ... base side end surface, 800 ... outer circumference, 801 ... base end outer circumferential portion, 802 ... leading end outer circumferential portion, 810 ... stepped surface, 821 ... base end inner circumferential portion, 822 ... leading end inner circumferential portion, 830 ... leading end surface, 901 ... base end side close-time engaged portion, 902 ... leading end side close-time engaged portion, 920 ... outer circumference, 921 ... inner circumference, 922 ... cover leg portion, S ... space, S1 ... base end side space, S2 ... leading end side space, S21 ... leading end side space, S22 ... second leading end side space, S3 ... third leading end side space, S4 ... fourth leading end side space, C8 ... liquid flow path, C9 ... plug flow path, C91 ... axial flow path, C92 ... communication flow path, ML ... liquid drug, MS ... solid drug, MM ... mixed drug, 80 ... filter, 10 ... holding member, 34 ... close contact portion, 35 ... projection, 65 ... inner circumference, 71 ... leading end side subspace, 72 ... base end side subspace, 110 ... base end side portion, 111 ... flow path, 112 ... attachment portion, 113 ... leg portion, 600 ... base side end surface, 601 ... base end side edge, 200 ... contact surface (base side end surface), 201 ... base end side contact surface, 201a ... first base end side contact surface, 201b ... second base end side contact surface, 202 ... leading end side contact surface, 203 ... connection contact surface, 203a ... first connection contact surface, 203b ... second connection contact surface, 203c ... second connection contact surface, 204 ... leading end surface, 220 ... attachment outer circumferential portion, 221 ... attachment inner circumferential portion, 222 ... outer circumferential end surface, 223 ... inner circumferential end surface, 224 ... shoulder portion, 225 ... projection, 226 ... attachment inner circumference, 230 ... base end surface

## Claims

1. A formulation for injection in which a solution is filled in a syringe, wherein
the syringe comprises:
a barrel formed in a cylindrical shape having a leading end portion and a base end portion, wherein the leading end portion is provided with a nozzle hole for externally ejecting a solution held therein; and
a filter disposed within the barrel in a position on a base end side of the nozzle hole, wherein
the filter is provided in such a manner as to allow the solution to flow from the base end side to a leading end side through the filter, and
the filter is a membrane, and a material of the membrane includes one or more selected from polyether sulfone (PES), polyvinylidene fluoride (PVDF), polysulfone (PS), and an acrylic copolymer.

2. A formulation for injection in which a solution is filled in a syringe, wherein
the syringe comprises:
a barrel formed in a cylindrical shape having a leading end portion and a base end portion, wherein the leading end portion is provided with a nozzle hole for externally ejecting a solution held therein; and
a filter disposed within the barrel in a position on a base end side of the nozzle hole, wherein
the filter is provided in such a manner as to allow the solution to flow from the base end side to a leading end side through the filter,
the filter is a membrane, and a material of the membrane includes one or more selected from polyether sulfone (PES), polyvinylidene fluoride (PVDF), polysulfone (PS), and an acrylic copolymer, and
in discharging the solution, visually detectable particles are removable from the discharged solution.

3. The formulation for injection according to claim 1 or 2, wherein the material of the membrane is an acrylic copolymer.

4. The formulation for injection according to claim 3, wherein the acrylic copolymer is hydrophobic.

5. The formulation for injection according to any one of claims 1 to 4, wherein the filter has a pore size of 5 µm or less, and preferably 5 µm.

6. The formulation for injection according to any one of claims 1 to 5, wherein the leading end side of the filter is provided with a leading end side subspace, the base end side of the filter is provided with a base end side subspace, and when seen from an axial direction of the barrel, the nozzle hole has an area smaller than an area of the base end side subspace, and smaller than an area of the leading end side subspace.

7. The formulation for injection according to any one of claims 1 to 6, wherein
the syringe comprises partitioning means for partitioning an inside space of the barrel into a base end side space and a leading end side space,
the partitioning means comprises: a cylindrical housing disposed to liquid-tightly contact an inner surface of the barrel; and an inner plug portion disposed in such a manner as to be openable/closable to an inside of the housing for communicating or non-communicating the base end side space and the leading end side space with each other through the inside of the housing,
the housing has a close-time engaging portion,
the inner plug portion has a close-time engaged portion to be engaged with the close-time engaging portion from the base end side,
the partitioning means is constituted in such a manner that one of the close-time engaging portion and the close-time engaged portion moves beyond the other to release engagement between the close-time engaging portion and the close-time engaged portion when a pressing force is applied from the base end side by a piston inserted into the barrel from the base end side, and that the inner plug portion is opened to the inside of the housing to communicate the base end side space and the leading end side space with each other, and
the filter is held in a leading end side portion of the housing.

8. The formulation for injection according to claim 7, wherein one of the close-time engaging portion and the close-time engaged portion is disposed on the leading end side and the base end side of the other of the close-time engaging portion and the close-time engaged portion.

9. The formulation for injection according to claim 7 or 8, wherein a material of the inner plug portion is linear low density polyethylene (LLDPE), or low density polyethylene (LDPE), and is preferably low density polyethylene (LDPE).

10. The formulation for injection according to any one of claims 1 to 6, wherein
the syringe comprises partitioning means for partitioning an inside space of the barrel into a base end side space and a leading end side space,
the partitioning means comprises a cylindrical housing disposed to liquid-tightly contact an inner surface of the barrel, and
the filter is held in a leading end side portion of the housing.

11. The formulation for injection according to any one of claims 7 to 10, wherein the partitioning means is disposed in such a manner that a volume of the leading end side space of the barrel is 150 mm³ or less.

12. The formulation for injection according to any one of claims 1 to 11, wherein a compound contained in the solution as an active ingredient is a low molecular weight compound, a middle molecular weight compound, or a protein.

13. The formulation for injection according to any one of claims 1 to 12, wherein the solution comprises emicizumab as an active ingredient.

14. A method for removing visually detectable particles contained in a formulation for injection, wherein
the formulation for injection is a solution, and is filled in a syringe,
the syringe comprises: a barrel formed in a cylindrical shape having a leading end portion and a base end portion, wherein the leading end portion is provided with a nozzle hole for externally ejecting the formulation for injection held therein; and a filter disposed within the barrel in a position on a base end side of the nozzle hole, the filter being provided in such a manner as to allow the solution to flow from the base end side to a leading end side through the filter,
the method comprises discharging the solution filled in the syringe through the filter, and the filter is a membrane, and a material of the membrane includes one or more selected from polyether sulfone (PES), polyvinylidene fluoride (PVDF), polysulfone (PS), and an acrylic copolymer.

15. The method according to claim 14, wherein a compound contained in the solution as an active ingredient is a low molecular weight compound, a middle molecular weight compound, or a protein.

16. The method according to any one of claim 14 or 15, wherein the solution comprises emicizumab as an active ingredient.
